# EUROPEAN PATENT APPLICATION

(11) **EP 4 455 166 A2**
(43) Date of publication of application: **30.10.2024**
(21) Application number: 24196402.2
(22) Date of filing: 08.08.2019
(51) Int. Cl.: C07K 17/00

(54) **METHOD FOR PRODUCING CD3-POSITIVE CELL**

(30) Priority: 10.08.2018 JP 2018151580; 08.03.2019 JP 2019042666; 25.06.2019 JP 2019117878
(62) Divisional of application: 19847695.4
(71) Applicant: Kyoto University, Kyoto-shi, Kyoto 606-8501 (JP); Takeda Pharmaceutical Company Limited, Osaka-shi, Osaka 541-0045 (JP)
(72) Inventor: Kaneko, Shin, Kyoto-shi, Kyoto, 606-8501 (JP); Kawai, Yohei, Kyoto-shi, Kyoto, 606-8501 (JP); Arima, Suguru, Fujisawa-shi, Kanagawa, 251-0012 (JP); Takiguchi, Maiko, Fujisawa-shi, Kanagawa, 251-0012 (JP); Nakayama, Kazuhide, Fujisawa-shi, Kanagawa, 251-0012 (JP); Kassai, Yoshiaki, Fujisawa-shi, Kanagawa, 251-0012 (JP); Hayashi, Akira, Fujisawa-shi, Kanagawa, 251-0012 (JP)
(74) Representative: Hoffmann Eitle

(57) **Abstract**

To enable stable supply of differentiated T cells, the present invention provides a method for producing, or a method for expansion culture, or a kit for expansion culture of a CD3-positive cell in which T cell marker CD3 is expressed on the cell membrane.

## Description

### [Technical Field]

The present invention relates to a production method or an expansion culture method of CD3-positive cells (or CD3-positive CD197-positive cells), including a step of culturing CD3-positive cells in the presence of a CD3/TCR complex agonist, fibronectin or a variant thereof, and a CD30 agonist, and a kit for expansion culture of CD3-positive cells. The present invention also relates to a method for maintaining CD3-positive cells as CD3-positive CD197-positive cells, including a step of stimulating a CD30 signal in the CD3-positive cells, and a kit for maintaining CD3-positive cells as CD3-positive CD197-positive cells.

### (Background of the Invention)

Immunotherapy has been clarified to show a strong life-prolonging effect even on cancers for which conventional treatments are not effective and is becoming a leading therapy in cancer treatment. CART therapy including transfer of autologous T cells transfected with anti-CD19 chimeric antigen receptor (CAR) gene shows a high complete remission rate for B cell malignancies, and the FDA approved two CART therapies for B-cell acute lymphoblastic leukemia and diffuse large B-cell lymphoma in 2017. In addition, many clinical trials are underway for TCR-T cell therapy in which a T cell receptor (TCR) gene that recognizes cancer cell antigen is introduced. While these genetically-modified T cell therapies using patients' own (self) T cells show extremely superior clinical results, they involve transport, gene modification, cell culture, and the like of T cells. Such complicated processes requiring several weeks or more make suppression of costs and quality control difficult. Moreover, since they lead to the loss of treatment opportunities for patients with fast-growing cancer, the development of an off-the-shelf allogenic T cell therapy is strongly demanded. As a method for expansion culture of T cells collected from patients, a method for producing CD8-positive Tc1 lymphocytes or CD8-positive Tc2 lymphocytes by contacting a T cell population with an anti-CD3 agonist antibody and an anti-CD30 agonist antibody (patent document 1), a method for expansion culture of tumor-specific T cells by obtaining a T cell population from a cancer patient and contacting the T cell population with an anti-CD3 agonist antibody, an anti-CD28 antibody, and a VEGF inhibitor (patent document 2) have been reported so far. However, T cells derived from healthy donor or patients have limited proliferative capacity, and the number of genetically modified T cells that can be produced from T cells that can be recovered by a single apheresis is limited. In addition, it has been reported that expansion culture of T cells by TCR stimulation causes a transient increase in the expression of CCR7 (CD197), which is a cell surface marker of naive cells and memory T cells, followed by disappearance within several days thereafter (non-patent document 1). In addition, it has been reported that being a memory T cell ("T cell persistence") is important for in vivo antitumor activity, and that a high in vivo antitumor effect was observed by increasing the number of CART cells to be administered, which is a memory T cell (non-patent document 2). Therefore, low CD197 expression in the cell population obtained by expansion culture of T cells was not suitable for cancer treatment.

On the other hand, since T cells differentiated from pluripotent stem cells such as iPS cell, ES cell, and the like (differentiated T cells) are made from pluripotent stem cells that can theoretically proliferate infinitely, modified T cells can be produced infinitely in theory. However, a method for expansion culture of iPS cells while maintaining pluripotency requires high cost and involves technical difficulties. Therefore, a method for obtaining differentiated T cells, particularly CD197-positive T cells, by proliferating a large number of iPS cells has a limitation. As a result, another approach to obtain a large number of T cells, particularly CD197-positive T cells, has been demanded.

### [Document List]

### [Patent documents]

patent document 1: WO 2003/038062
patent document 2: US-A-2014/0255368

### [Non-patent document]

non-patent document 1: Sallusto et al., Eur. J. Immunol. vol. 29, 2037-2045, 1999
non-patent document 2: Kaartinen et al., Cytotherapy vol. 19, 689-702, 2017

### [Summary of Invention]

### [Technical Problem]

The present invention aims to stably supply differentiated T cells, particularly CD197-positive T cells, and provides a production method, an expansion culture method, and a kit for expansion culture of CD3-positive cells with a T cell marker CD3 expressed on a cell membrane. Another object of the present invention is to provide a method for maintaining CD197-positive T cells and a kit for maintaining CD197-positive T cells.

### [Solution to Problem]

The present inventors have conducted intensive studies in an attempt to achieve the objects and found that CD8-positive T cells obtained from iPS cells transfected with TCR gene (iPS cell-derived CD8-positive T cells) can be efficiently proliferated by culturing the T cells in a medium containing an anti-CD30 agonist antibody on a culture container in which anti-CD3 agonist antibody and RetroNectin (registered trade mark) are immobilized. Furthermore, they have confirmed that the proliferation efficiency of the T cells and the antigen-specific cytotoxic activity are not affected even when the iPS cell-derived CD8-positive T cells are repeatedly proliferated by the culture method. In addition, they have found that, when the iPS cell-derived CD8-positive T cells are cultured using an anti-CD30 agonist antibody, the expression rate of CD197 becomes high as compared to the non-use of the anti-CD30 agonist antibody and the proliferated T cells are present as central memory T cells. Similar to the iPS cell-derived CD8-positive T cells, moreover, human peripheral blood-derived CD8-positive T cells and iPS cell-derived CD8-positive T cells transfected with anti-CD19-CAR gene (iPS cell-derived anti-CD19-CART cells) could also be proliferated efficiently by adding an anti-CD30 agonist antibody to the medium on a culture container in which anti-CD3 agonist antibody/RetroNectin (registered trade mark) are immobilized. Furthermore, they have found that when the human peripheral blood-derived CD8-positive T cells or the iPS cell-derived anti-CD19-CART cells are cultured using an anti-CD30 agonist antibody, the expression of CD197 is maintained for a long term and the human peripheral blood-derived CD8-positive T cells can survive in vivo for a long term as compared to the non-use of the anti-CD30 agonist antibody. Based on the above findings, they have completed the present invention.

Accordingly, the present invention provides the following.
[1] A method for producing a CD3-positive cell, comprising step (I) of culturing the CD3-positive cell in the presence of a CD3/TCR complex agonist, fibronectin or a variant thereof, and a CD30 agonist.
[2] The method of [1], further comprising step (II) of culturing the CD3-positive cell cultured in step (I) in the absence of a CD3/TCR complex agonist and fibronectin or a variant thereof, and in the presence of a CD30 agonist.
[3] The method of [1] or [2], wherein the CD3-positive cell is derived from a pluripotent stem cell.
[4] The method of [3], wherein the pluripotent stem cell is an iPS cell.
[5] The method of any one of [1] to [4], wherein the CD3-positive cell is a chimeric antigen receptor-expressing CD3-positive cell.
[6] The method of any one of [1] to [5], wherein the CD3-positive cell is a CD3-positive CD8-positive cell.
[7] The method of [6], wherein the CD3-positive CD8-positive cell is a CD3-positive CD8-positive CD4-negative cell.
[8] The method of any one of [1] to [7], wherein the CD3-positive cell is a γTCR-positive and/or δTCR-positive cell.
[9] The method of any one of [1] to [8], wherein the CD3/TCR complex agonist is a CD3 agonist and/or a TCR agonist.
[10] The method of [9], wherein the CD3 agonist is an anti-CD3 agonist antibody or a binding fragment thereof.
[11] The method of [10], wherein the anti-CD3 agonist antibody or a binding fragment thereof is an anti-CD3 agonist antibody or a binding fragment thereof produced from UCHT1 clone.
[12] The method of [9], wherein the TCR agonist is at least one selected from the group consisting of an anti-TCR antibody or a binding fragment thereof, an HLA/peptide complex or a multimer thereof, and an HLA/superantigen complex or a multimer thereof.
[13] The method of [10] or [11], wherein the anti-CD3 agonist antibody or a binding fragment thereof is immobilized on a culture container.
[14] The method of [13], wherein the anti-CD3 agonist antibody or a binding fragment thereof is immobilized by contacting 1 ng/ml - 50000 ng/ml of the anti-CD3 agonist antibody or a binding fragment thereof with the culture container.
[15] The method of any one of [1] to [14], wherein the fibronectin or a variant thereof is RetroNectin (registered trade mark).
[16] The method of [15], wherein the RetroNectin (registered trade mark) is immobilized on the culture container.
[17] The method of [16], wherein the RetroNectin (registered trade mark) is immobilized by contacting 1 - 150 µg/mL of the RetroNectin (registered trade mark) with the culture container.
[18] The method of any one of [1] to [17], wherein the CD30 agonist is at least one selected from the group consisting of an anti-CD30 agonist antibody or a binding fragment thereof and a CD30 ligand or a binding fragment thereof.
[19] The method of [18], wherein the anti-CD30 agonist antibody or a binding fragment thereof is contained in the medium.
[20] The method of [19], wherein a concentration of the anti-CD30 agonist antibody or a binding fragment thereof in the medium of 1 ng/ml - 1000 ng/ml.
[21] The method of any one of [1] to [20], wherein the medium comprises at least one selected from IL-7, IL-15, IL-18 and IL-21.
[22] The method of [21], wherein the medium comprises IL-7, IL-15, IL-18 and IL-21.
[23] The method of [21] or [22], wherein the medium further comprises TL1A and/or IL-12.
[24] The method of any one of [1] to [23], wherein the CD3-positive cell produced is further CD197-positive.
[25] A CD3-positive cell obtained by the method of any one of [1] to [24].
[26] A method for expansion culture of a CD3-positive cell, comprising a step of culturing the CD3-positive cell in the presence of a CD3/TCR complex agonist, fibronectin or a variant thereof, and a CD30 agonist.
[27] A kit for expansion culture of a CD3-positive cell, comprising the following:
   (1) a culture container in which a CD3/TCR complex agonist, and fibronectin or a variant thereof are immobilized, and
   (2) a medium comprising a CD30 agonist.
[28] A method for maintaining a CD3-positive cell as a CD3-positive CD197-positive cell, comprising a step of stimulating a CD30 signal in the CD3-positive cell.
[29] The method of [28], wherein the CD3-positive cell is a CD3-positive CD8-positive cell.
[30] The method of [29], wherein the CD3-positive CD8-positive cell is a CD3-positive CD8-positive CD4-negative cell.
[31] The method of any one of [28] to [30], wherein the medium used in the step of stimulation comprises at least one selected from IL-7, IL-15, IL-18 and IL-21.
[32] The method of [31], wherein the medium used in the step of stimulation comprises IL-7, IL-15, IL-18 and IL-21.
[33] The method of [31] or [32], wherein the medium used in the step of stimulation further comprises TL1A and/or IL-12.
[34] A kit for maintaining a CD3-positive cell as a CD3-positive CD197-positive cell, comprising a CD30 agonist.
[35] A chimeric antigen receptor comprising an antigen binding domain, a transmembrane domain and an intracellular signal transduction domain, wherein the intracellular signal transduction domain comprises a CD30 intracellular signal transduction domain or a mutant thereof.
[36] A nucleic acid comprising a polynucleotide encoding the chimeric antigen receptor of [35].
[37] A chimeric antigen receptor expression vector comprising the nucleic acid of [36].
[38] A chimeric antigen receptor-expressing cell comprising the chimeric antigen receptor expression vector of [37].
[39] The cell of [38], wherein the chimeric antigen receptor expression cell is a CD3-positive cell.
[40] A medicament comprising the cell of [38] or [39].
[41] The medicament of [40] for use in the prophylaxis or treatment of a tumor expressing an antigen recognized by the chimeric antigen receptor of [35].
[42] An agent for killing a cell expressing an antigen recognized by the chimeric antigen receptor of [35], wherein the agent comprises the cell of [38] or [39].
[43] The cell of [38] or [39] for use in the prophylaxis or treatment of a tumor expressing an antigen recognized by the chimeric antigen receptor of [35].
[44] Use of the cell of [38] or [39] in producing an agent for the prophylaxis or treatment of a tumor expressing an antigen recognized by the chimeric antigen receptor of [35].
[45] A method for preventing or treating a tumor expressing an antigen recognized by the chimeric antigen receptor of [35], comprising administering the cell of [38] or [39].

### [Advantageous Effects of Invention]

T cells can be efficiently produced or expansion cultured by culturing CD3-positive cells in the presence of a CD3/TCR complex agonist, fibronectin or a variant thereof, and a CD30 agonist. In addition, since the CD3-positive cells produced or expansion cultured by the above-mentioned culture are CD197-positive cells, long-term efficacy is expected when the cells are used for genetically modified T cell therapy. In addition, CD3-positive cells can be maintained for a long term as CD197-positive cells by stimulating the CD30 signal of the cells.

### [Brief Description of Drawings]

Fig. 1 shows concentrations suitable for immobilizing anti-CD3 agonist antibody and RetroNectin (registered trade mark) as measured by the ELISA method.
Fig. 2 shows iPSC-derived T cell counts (measured by the ATP amount) 12 days after stimulation with immobilized anti-CD3 agonist antibody/RetroNectin (registered trade mark).
Fig. 3 shows proliferation curves of iPSC-derived T cells stimulated with immobilized anti-CD3 agonist antibody/RetroNectin (registered trade mark) or anti-CD3/CD28 beads. The vertical axis shows cell counts, and the horizontal axis shows the number of days elapsed from the day when the above-mentioned stimulation was started.
Fig. 4 shows proliferation curves of iPSC-derived T cells for each number of stimulations with immobilized anti-CD3 agonist antibody/RetroNectin (registered trade mark).
Fig. 5 shows the antigen-specific cytotoxic activity of iPSC-derived T cells proliferated by stimulation with immobilized anti-CD3 agonist antibody/RetroNectin (registered trade mark).
Fig. 6 shows promoted proliferation of iPSC-derived T cells stimulated with immobilized anti-CD3 agonist antibody/RetroNectin (registered trade mark) by the addition of an anti-CD30 agonist antibody. The vertical axis shows cell counts, and the horizontal axis shows the number of days elapsed from the day when the above-mentioned stimulation was started.

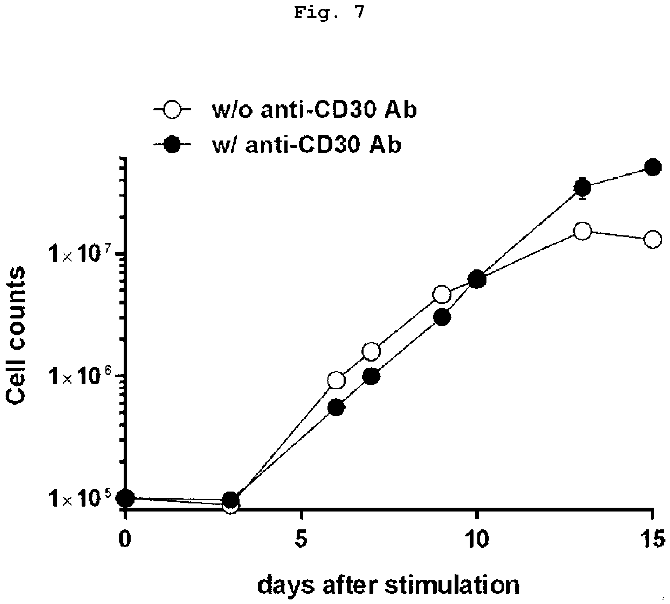

Fig. 7 shows the proliferation curves of iPSC-derived T cells that underwent a proliferation test in which the iPSC-derived T cells were stimulated (first stimulation) with immobilized anti-CD3 agonist antibody/RetroNectin (registered trade mark), or immobilized anti-CD3 agonist antibody/RetroNectin (registered trade mark) and anti-CD30 agonist antibody, and then the same stimulation (second stimulation) again after the completion of the test. The vertical axis shows cell counts, and the horizontal axis shows the number of days elapsed from the day when the above-mentioned second stimulation was started.
Fig. 8 shows the expression of CD197 and CD45RA on the cell membrane surface of iPSC-derived T cells 7 days after stimulation with immobilized anti-CD3 agonist antibody/RetroNectin (registered trade mark), or immobilized anti-CD3 agonist antibody/RetroNectin (registered trade mark) and anti-CD30 agonist antibody.
Fig. 9 shows the antigen-specific cytotoxic activity of iPSC-derived T cells proliferated by stimulation with immobilized anti-CD3 agonist antibody/RetroNectin (registered trade mark) and anti-CD30 agonist antibody.
Fig. 10 shows proliferation curves of human peripheral blood-derived CD8-positive T cells stimulated with immobilized anti-CD3 agonist antibody/RetroNectin (registered trade mark), or immobilized anti-CD3 agonist antibody/RetroNectin (registered trade mark) and anti-CD30 agonist antibody. The vertical axis shows cell counts, and the horizontal axis shows the number of days elapsed from the day when the above-mentioned stimulation was started.
Fig. 11 shows the expression of CD197 on the cell membrane surface of human peripheral blood-derived CD8-positive T cells after stimulation with anti-CD3/CD28 beads containing various ILs and anti-CD30 antibody.
Fig. 12 shows the number of human CD8-positive T cells surviving in the blood, spleen and bone marrow of irradiated immunodeficient mice 4 weeks after intravenous transplantation of human peripheral blood-derived CD8-positive T cells after stimulation with anti-CD3/CD28 beads containing various ILs and anti-CD30 antibody.
Fig. 13 shows proliferation curves of iPS cell-derived T cells (iPS-T) and iPS cell-derived anti-CD19-CART cells (iPS-CART anti-CD19) stimulated with immobilized anti-CD3 agonist antibody/RetroNectin (registered trade mark) and anti-CD30 agonist antibody. The vertical axis shows cell counts, and the horizontal axis shows the number of days elapsed from the day when the above-mentioned stimulation was started.
Fig. 14 shows proliferation curves of iPS cell-derived anti-CD19-CART cells stimulated with immobilized anti-CD3 agonist antibody/RetroNectin (registered trade mark), or immobilized anti-CD3 agonist antibody/RetroNectin (registered trade mark) and anti-CD30 agonist antibody. The vertical axis shows cell counts, and the horizontal axis shows the number of days elapsed from the day when the above-mentioned stimulation was started.
Fig. 15 shows the expression of CD197 on the cell membrane surface of iPS cell-derived anti-CD19-CART cells stimulated with immobilized anti-CD3 agonist antibody/RetroNectin (registered trade mark), or immobilized anti-CD3 agonist antibody/RetroNectin (registered trade mark) and anti-CD30 agonist antibody.
Fig. 16 shows the cytotoxic activity, against CD19-expressing Raji cells, of iPS cell-derived anti-CD19-CART cells stimulated with immobilized anti-CD3 agonist antibody/RetroNectin (registered trade mark) by the addition of anti-CD30 agonist antibody.
Fig. 17 shows the cytotoxic activity, against CD19-expressing Raji cells, of iPS cell-derived anti-CD19-CART cells (iCD19-CD30-CART) containing a CD30-derived intracellular domain.
Fig. 18 shows cell proliferation of γδT cells (iγδT cells) differentiated from non-T cell-derived iPS cells and stimulated with immobilized anti-CD3 agonist antibody/RetroNectin (registered trade mark) by the addition of anti-CD30 agonist antibody. The vertical axis shows the cell proliferation ratio, and the horizontal axis shows the number of days elapsed from the day when the above-mentioned stimulation was started.
Fig. 19 shows cell proliferation of anti-CD19-CARγδT cells (iCARγδT cells) stimulated with immobilized anti-CD3 agonist antibody/RetroNectin (registered trade mark) by the addition of anti-CD30 agonist antibody. The vertical axis shows cell counts, and the horizontal axis shows the number of days elapsed from the day when the above-mentioned stimulation was started.
Fig. 20 shows the antigen-specific cytotoxic activity of iCD19CAR/IL-15yδT cells proliferated by stimulation with immobilized anti-CD3 agonist antibody/RetroNectin (registered trade mark).
Fig. 21 shows the effect of increasing the survival days of human CD19-expressing tumor-bearing mice by iCD19CAR/IL-15γδT cells proliferated by stimulation with immobilized anti-CD3 agonist antibody/RetroNectin (registered trade mark).
Fig. 22 shows the antitumor effect of iCD19CAR/IL-15αβT cells proliferated by stimulation with immobilized anti-CD3 agonist antibody/RetroNectin (registered trade mark).
Fig. 23 shows the concentration suitable for immobilizing anti-CD3 agonist antibody (UCHT1) and RetroNectin (registered trade mark) as measured by the ELISA method.
Fig. 24 shows the proliferation rate of iPSC-derived T cells 13 days after stimulation with immobilized anti-CD3 agonist antibody (UCHT1)/RetroNectin (registered trade mark) and anti-CD30 agonist antibody.

### (Detailed Description of the Invention)

In the present specification, the "gene expression" encompasses both the synthesis of mRNA from a specific nucleotide sequence of the gene (also referred to as transcription or mRNA expression) and the synthesis of protein based on the information of the mRNA (also referred to as translation or protein expression). Unless otherwise specified, the "gene expression" or simple "expression" means expression of protein.

In the present specification, "positive" means that a protein or mRNA is expressed in an amount detectable by a method known in the art. Protein can be detected by an immunological assay using an antibody, such as ELISA method, immunostaining method, Western blot method, and flow cytometry. In addition, a reporter protein is expressed together with the protein, and the target protein may be detected by detecting the reporter protein. In addition, the presence of a protein can be detected by detecting the function of the protein (e.g., when the protein is a transcription factor, a gene whose expression is controlled by the protein is detected, and when the protein is an enzyme, a substrate or a product catalyzed by the protein is detected, and the like). mRNA can be detected by, for example, nucleic acid amplification method and/or nucleic acid detection method such as RT-PCR, microarray, biochip, RNAseq and the like.

In the present specification, "negative" means that the expression level of the protein or mRNA is less than the lower limit of detection by all or any of the above-mentioned known methods. The lower limit of detection of protein or mRNA expression may vary depending on each method.

In the present specification, the "culture" refers to maintaining, proliferating (growing) and/or differentiating cells in an in vitro environment. "Culturing" means maintaining, proliferating (growing) and/or differentiating cells extra-tissue or ex-vivo, for example, in a cell culture dish or flask.

In the present specification, the "expansion culture" means culturing for the purpose of proliferating a desired cell population and increasing the cell number. The increase in cell number may be achieved by increasing the number of cells by proliferation to exceed the decrease in number by death, and it does not require proliferation of all cells in the cell population. The increase in the cell number may be 1.1 times, 1.2 times, 1.5 times, 2 times, 3 times, 4 times, 5 times, 6 times, 7 times, 8 times, 9 times, 10 times, 15 times, 20 times, 30 times, 40 times, 50 times, 100 times, 300 times, 500 times, 1,000 times, 3,000 times, 5,000 times, 10,000 times, 100,000 times, or not less than 1,000,000 times, compared to that before the start of expansion culture.

In the present specification, "stimulation" means that a certain substance binds to various receptors and the like to activate a signal pathway at the downstream thereof.

In the present specification, "enrich" refers to increasing the amount of a specific constituent component in a composition such as cell composition and the like, and "enriched" refers to a cell population in which, when used to explain a cell composition, for example, cell population, the amount of a specific constituent component has increased as compared to the proportion of such constituent component in the cell population before the enrichment. For example, a composition such as a cell population and the like can be enriched with respect to a target cell type, and therefore, the proportion of the target cell type increases as compared to that of the target cell present in the cell population before enrichment. The cell population can also be enriched with respect to a target cell type by cell selection or a screening method known in the art. The cell population can also be enriched by the specific selection or screening process described in the present specification. In specific embodiments of the present invention, at least 20%, 30%, 40%, 50%, 60%, 70%, 80%, 85%, 90%, 95%, 97%, 98% or 99% of the target cell population is enriched by a method for enriching the target cell population.

In the present specification, the "cell population" means two or more cells of the same type or different types. The "cell population" also means a mass of cells of the same type or different types.

The present invention provides a production method or an expansion culture method of CD3-positive cells, including a step of culturing CD3-positive cells in the presence of a CD3/TCR complex agonist, fibronectin or a variant thereof, and a CD30 agonist (hereinafter sometimes to be referred to as the production method or expansion culture method of the present invention).

The CD3/TCR complex and CD30 can be respectively stimulated with a CD3/TCR complex agonist and a CD30 agonist.

In the production method or expansion culture method of the present invention, the CD3-positive cell to be cultured is not particularly limited as long as it expresses CD3 on the cell membrane. The CD3-positive cell is preferably a CD3-positive CD8-positive cell (CD3-positive CD8-positive CD4-positive cell or CD3-positive CD8-positive CD4-negative cell), more preferably, a CD3-positive CD8-positive CD4-negative cell. Another preferred CD3-positive cell is, for example, a CD3-positive CD4-positive cell (CD3-positive CD4-positive CD8-positive cell or a CD3-positive CD4-positive CD8-negative cell).

The CD3-positive cell to be cultured by the production method or expansion culture method of the present invention may be CD30-positive before culture, or differentiated to be CD30-positive during culture. Therefore, when the CD3-positive cell is CD30-positive before culture, the CD3-positive cell is preferably a CD3-positive CD8-positive CD30-positive cell (CD3-positive CD8-positive CD4-positive CD30-positive cell or CD3-positive CD8-positive CD4-negative CD30-positive cell), more preferably, a CD3-positive CD8-positive CD4-negative CD30-positive cell. Another preferred CD3-positive cell is, for example, a CD3-positive CD4-positive CD30-positive cell (CD3-positive CD4-positive CD8-positive CD30-positive cell or CD3-positive CD4-positive CD8-negative CD30-positive cell).

The CD3-positive cell obtained by production or expansion culture by the production method or expansion culture method of the present invention is not particularly limited as long as it expresses CD3 on the cell membrane, and may be, for example, a cell similar to the CD3-positive cell cultured in the production method or expansion culture method of the present invention. The CD3-positive cell after the production or expansion culture is preferably CD197-positive. The CD3-positive cell after the production or expansion culture is further CD197-positive, the cell is specifically a CD3-positive CD8-positive CD197-positive cell (CD3-positive CD8-positive CD4-positive CD197-positive cell or CD3-positive CD8-positive CD4-negative CD197-positive cell), more preferably, a CD3-positive CD8-positive CD4-negative CD197-positive cell. Alternatively, the cell is a CD3-positive CD4-positive CD30-positive CD197-positive cell (CD3-positive CD4-positive CD8-positive CD30-positive CD197-positive cell or CD3-positive CD4-positive CD8-negative CD30-positive CD197-positive cell). Among the CD3-positive cells, since the CD197-positive cell is classified into stem cell memory T cell or central memory T cell according to the presence or absence of CD45RA expression, and has high self-proliferation ability that can supply effector T cells, it is suitable for anti-tumor immunity.

The CD3-positive cell after the production or expansion culture is further preferably CD197-positive CD45RA-negative. When the CD3-positive cell after the production or expansion culture is further CD197-positive CD45RA-negative, the cell is specifically a CD3-positive CD8-positive CD197-positive CD45RA-negative cell (CD3-positive CD8-positive CD4-positive CD197-positive CD45RA-negative cell or CD3-positive CD8-positive CD4-negative CD197-positive CD45RA-negative cell), more preferably, a CD3-positive CD8-positive CD4-negative CD197-positive CD45RA-negative cell. Alternatively, the cell is a CD3-positive CD4-positive CD30-positive CD197-positive CD45RA-negative cell (CD3-positive CD4-positive CD8-positive CD30-positive CD197-positive CD45RA-negative cell or CD3-positive CD4-positive CD8-negative CD30-positive CD197-positive CD45RA-negative cell). Among the CD3-positive cells, the CD197-positive CD45RA-negative cell is also particularly called a central memory T cell.

CD3 is a molecule that is expressed during the maturation process of T cells, forms a larger complex (CD3/TCR complex) with the T cell receptor (TCR), and is also known as a T cell marker. It is a complex of 4 types of polypeptides of γ, δ, ε, and ζ chains. The TCR that forms a complex with CD3 is a molecule that transmits a signal into T cells, and examples include a dimer composed of two out of α chain (TCRα), β chain (TCRβ), γ chain (TCRγ), and δ chain (TCRδ), a heterodimer (αβTCR) composed of TCRα and TCRβ, a heterodimer (γδTCR) composed of TCRγ and TCRδ, and homo dimer composed of the same TCR chains. Like CD3, CD8 is expressed in vivo during the maturation process of T cells and is expressed together with CD4 in the early stages of maturation, but only the expression of CD4 is lost along with the differentiation into cytotoxic T cells. CD8 is a molecule that functions as a co-receptor of the CD3/TCR complex and recognizes MHC class I/antigen peptide, and is a homodimer consisting of α-chain polypeptides or a heterodimer consisting of two types of polypeptides of α- and β-chains. CD4 is expressed in vivo together with CD8 in the early stages of T cell maturation, but only CD8 expression is lost along with differentiates into helper T cells. Like CD8, CD4 also functions as a co-receptor of the CD3/TCR complex, but unlike CD8, it is a molecule that recognizes MHC class II/antigen peptide. CD30 is known as a molecule expressed in activated lymphocytes (T cells and B cells), has 6 cystein-rich pseudo-repeat motifs as extracellular domains, and has, as intracellular domain, a TNF receptor-related factor (TRAP) binding sequence that stimulates NFκB signal. CD197 and CD45RA are used as marker molecules for distinguishing, in T cells, naive T cell or stem cell memory T cell (CD197-positive, CD45RA-positive), central memory T cell (CD197-positive, CD45RA-negative), effector memory T cell (CD197-negative, CD45RA-negative), and effector T cell (CD197-negative, CD45RA-positive).

In the production method or expansion culture method of the present invention, the CD3-positive cells to be cultured may be cells collected from a mammal or cells obtained by differentiating pluripotent stem cells. Preferred are cells obtained by differentiating pluripotent stem cells.

When the CD3-positive cells are collected from a mammal, the cells can be isolated and collected, for example, by collecting peripheral blood mononuclear cells (PBMC) from the mammal by apheresis, and then using anti-CD3 antibody column, density gradient centrifugation method and the like. Mammals for collecting CD3-positive cells include humans and animals other than human (e.g., dog, cat, mouse, rat, hamster, guinea pig, rabbit, swine, bovine, goat, horse, sheep, monkey etc.), and human is preferable.

When the CD3-positive cell is a cell obtained by differentiation of pluripotent stem cells, the pluripotent stem cell include embryonic stem cell (ES cell), induced pluripotent stem cell (iPS cell), embryonal carcinoma cell (EC cell), and embryonic germ cell (EG cell). Preferred is ES cell or iPS cell (more preferred is human iPS cell).

When the pluripotent stem cell is an ES cell, it can be produced by a method known per se. Examples of the production method of ES cell include, but are not limited to, a method of culturing an inner cell mass at the blastocyst stage of a mammal (see, for example, Manipulating the Mouse Embryo: A Laboratory Manual, Second Edition, Cold Spring Harbor Laboratory Press (1994)), and a method of culturing early embryo produced by somatic cell nuclear transfer (Wilmut et al., Nature, 385, 810(1997); Cibelli et al., Science, 280, 1256(1998); Akira Iriya et al., Protein Nucleic Acid Enzyme, 44, 892(1999); Baguisi et al., Nature Biotechnology, 17, 456(1999); Wakayama et al., Nature, 394, 369(1998); Wakayama et al., Nature Genetics, 22, 127(1999); Wakayama et al., Proc. Natl. Acad. Sci. USA, 96, 14984(1999); RideoutIII et al., Nature Genetics, 24, 109(2000)) and the like. ES cells can be obtained from a predetermined institution, and can also be purchased as a commercial product. For example, human ES cell lines H1, H7, H9, H13 and H14 are available from WiCell Research Institute in the United States, human ES cell lines HES1 - 6 are available from ES Cell International in Australia, human ES cell lines SA002, SA181 and SA611 are available from Cellartis AB in Sweden, human ES cell lines HUES1 - 17 are available from HUES Cell Facility in the United States, human ES cell lines KhES-1 - KhES-5 are available from the Institute for Frontier Life and Medical Sciences, Kyoto University, and human ES cell lines SEES1 - SEES7 are available from the National Center for Child Health and Development. When ES cell is produced by somatic cell nuclear transfer, the type of somatic cells and the source for collecting somatic cells are the same as those in the production of iPS cells described below.

When the pluripotent stem cell is an iPS cell, the iPS cell can be produced by introducing a nuclear reprogramming substance into the somatic cell. The somatic cells that can be used as a starting material for producing iPS cells may be any cell other than germ cells derived from mammals (e.g., mouse or human). Examples include keratinizing epithelial cells (e.g., keratinized epidermal cell), mucosal epithelial cells (e.g., epithelial cell on tongue surface layer), exocrine gland epithelial cells (e.g., mammary cell), hormone secretory cells (e.g., adrenal medulla cell), metabolic/storage cells (e.g., hepatocyte), luminal epithelial cells that constitute the interface (e.g., type I alveolar cell), vascularluminal epithelial cells (e.g., vascular endothelial cell), ciliated cells with transporting capacity (e.g., airway epithelial cell), extracellular matrix secretion cells (e.g., fibroblast), contractile cells (e.g., smooth muscle cell), blood and immune system cells (e.g., T lymphocyte), cells relating to sensor (e.g., rod cell), autonomic nervous system neurons (e.g., cholinergic neuron), sensory organ and peripheral neuron-supporting cells (e.g., satellite cell), nerve cells and glial cells of central nervous system (e.g., astroglial cell), pigment cells (e.g., retinal pigment epithelial cell), and progenitor cells thereof (tissue progenitor cell) and the like. The degree of cell differentiation is not particularly limited, and both undifferentiated progenitor cells (including somatic stem cells) and finally-differentiated mature cells can be similarly used as the origin of the somatic cell in the present invention. As used herein, examples of the undifferentiated progenitor cell include tissue stem cells (somatic stem cells) such as fat-derived stroma (stem) cell, neural stem cell, hematopoietic stem cell, mesenchymal stem cell, pulp stem cell and the like.

Nuclear reprogramming substance to be introduced into somatic cells for the production of iPS cells includes combinations of various reprogramming genes reported so far (see, for example, WO 2007/069666, Nature Biotechnology, 26, 101-106 (2008), Cell, 126, 663-676 (2006), Cell, 131, 861-872

(2007), Nat. Cell Biol., 11, 197-203 (2009), Nature, 451, 141-146 (2008), Science, 318, 1917-1920 (2007), Stem Cells, 26, 1998-2005 (2008), Cell Research (2008) 600-603, Nature 454: 646-650 (2008), Cell Stem Cell, 2: 525-528(2008), WO2008/118820, Nat. Cell Biol., 11, 197-203 (2009), Nat. Cell Biol., 11, 197-203 (2009), Science, 324: 797-801 (2009)). In addition, a protein encoded by the above-mentioned reprogramming gene can also be introduced as a nuclear reprogramming substance into a somatic cell (Cell Stem Cell, 4: 381-384(2009), Cell Stem Cell, doi:10.1016/j.stem.2009.05.005 (2009)). In particular, a combination of the three factors Oct3/4, Sox2 and Klf4 is preferable, considering that the obtained iPS cells are used for therapeutic purposes.

iPS cell colony can be selected by a method using drug resistance and reporter activity as indices (Cell, 126, 663-676 (2006), Nature, 448, 313-317 (2007)) or a method including visual morphological observation (Cell, 131, 861-872 (2007)). Confirmation of iPS cell can be performed using the expression of various ES cell-specific genes and teratoma formation as indices.

Currently, there are various types of the iPS cell (iPSC), and iPS cell established by Yamanaka, et al. by introducing 4 factors of Oct3/4, Sox2, Klf4, c-Myc into mouse fibroblast (Takahashi K, Yamanaka S., Cell, (2006) 126: 663-676), iPSC derived from a human cell established by introducing similar 4 factors into human fibroblast (Takahashi K, Yamanaka S., et al., Cell, (2007) 131: 861-872.), Nanog-iPSc established by selecting with the expression of Nanog as an index after introduction of the above-mentioned 4 factors (Okita, K., Ichisaka, T., and Yamanaka, S. (2007). Nature 448, 313-317.), iPSC produced by a method free of c-Myc (Nakagawa M, Yamanaka S., et al., Nature Biotechnology, (2008) 26, 101 - 106), and iPSC established by introducing 6 factors by a virus-free method (Okita K et al., Nat. Methods 2011 May; 8(5):409-12, Okita K et al., Stem Cells. 31(3):458-66.) can also be used. In addition, iPSC established by Thomson et al. by introducing 4 factors of OCT3/4, SOX2, NANOG, and LIN28 (Yu J., Thomson JA. et al., Science (2007) 318: 1917-1920.), iPSC produced by Daley et al. (Park IH, Daley GQ. et al., Nature (2007) 451: 141-146), induced pluripotent stem cell produced by et al. (JP-A-2008-307007) and the like can also be used.

In addition, any of the iPSCs known in the art that are described in published papers (e.g., Shi Y., Ding S., et al., Cell Stem Cell, (2008) Vol3, Issue 5,568-574; Kim JB., Scholer HR., et al., Nature, (2008) 454, 646-650; Huangfu D., Melton, DA., et al., Nature Biotechnology, (2008) 26, No 7, 795-797), or patents (e.g., JP-A-2008-307007, JP-A-2008-283972, US2008-2336610, US2009-047263, WO2007/069666, WO2008/118220, WO2008/124133, WO2008/151058, WO2009/006930, WO2009/006997, WO2009/007852) can be used.

As the induced pluripotent stem cell line, various iPSC lines established by NIH, RIKEN, Kyoto University and the like can be used. Examples of the human iPSC line include HiPS-RIKEN-1A strain, HiPS-RIKEN-2A strain, HiPS-RIKEN-12A strain, Nips-B2 strain of RIKEN, 253G1 strain, 253G4 strain, 1201C1 strain, 1205D1 strain, 1210B2 strain, 1383D2 strain, 1383D6 strain, 201B7 strain, 409B2 strain, 454E2 strain, 606A1 strain, 610B1 strain, 648A1 strain, 1231A31 strain, FfI-01s04 and the like, and 1231A3 strain is preferable.

When a CD3-positive cell is a cell obtained by differentiating a pluripotent stem cell, the TCR expressed in the CD3-positive cell includes a dimer consisting of two out of TCRα, TCRβ, TCRγ and TCRδ, a heterodimer consisting of TCRα and TCRβ (αβTCR), a heterodimer consisting of TCRγ and TCRδ (γδTCR), and a homodimer consisting of the same TCR chain. While these TCRs may be endogenous dimers or exogenous dimers, a dimer containing exogenous TCRα and exogenous TCRβ is preferable.

In the present specification, the "exogenous TCR" refers to a heterodimer consisting of an exogenous TCRα and an exogenous TCRβ, a heterodimer consisting of an exogenous TCRγ and an exogenous TCRδ, and/or a homodimer consisting of the same exogenous TCR chain.

When TCR is a dimer containing endogenous TCRα and endogenous TCRβ, the pluripotent stem cell may be a pluripotent stem cell prepared from a cell containing a nucleic acid containing a base sequence encoding endogenous TCRα and a nucleic acid containing a base sequence encoding endogenous TCRβ. Examples of such cell include T cells (CD8-positive CD4-negative cell, CD8-negative CD4-positive cell, CD4-positive CD8-positive cell and the like). When TCR is a dimer containing exogenous TCRα and exogenous TCRβ, then the pluripotent stem cell may be a cell containing a nucleic acid containing a base sequence encoding an exogenous TCRα and nucleic acid containing a base sequence encoding an exogenous TCRβ.

In the present specification, the "exogenous TCR nucleic acid" refers to a nucleic acid containing a base sequence encoding an exogenous TCRα, nucleic acid containing a base sequence encoding an exogenous TCRβ, nucleic acid containing a base sequence encoding an exogenous TCRγ, and/or a nucleic acid containing a base sequence encoding an exogenous TCRδ.

The method for introducing an exogenous TCR nucleic acid (e.g., nucleic acid containing a base sequence encoding an exogenous TCRα and nucleic acid containing a base sequence encoding an exogenous TCRβ) into a pluripotent stem cell is not particularly limited. For example, the following method can be used.

When the exogenous TCR nucleic acid is in the form of a DNA, for example, vectors such as virus, plasmid, artificial chromosome, and the like can be introduced into pluripotent stem cells by methods such as lipofection, liposome, microinjection, and the like. Examples of the virus vector include retrovirus vector, lentivirus vector, adenovirus vector, adeno-associated virus vector, Sendai virus vector and the like. Examples of the artificial chromosome vector include human artificial chromosome (HAC), yeast artificial chromosome (YAC), bacterial artificial chromosome (BAC, PAC) and the like. As the plasmid, plasmid for mammalian cell can be used. The vector can contain control sequences such as promoter, enhancer, ribosome-binding sequence, terminator, polyadenylation sites and the like so that exogenous TCR can be expressed. Where necessary, selection marker sequences such as drug resistance gene (e.g., kanamycin resistance gene, ampicillin resistance gene, puromycin resistance gene and the like), thymidine kinase gene, diphtheria toxin gene and the like, reporter gene sequences such as fluorescent protein, β glucuronidase (GUS), FLAG and the like, and the like can be contained. Examples of the promoter include SV40 promoter, LTR promoter, CMV (cytomegalovirus) promoter, RSV (Rous sarcoma virus) promoter, MoMuLV (Moloney mouse leukemia virus) LTR, HSV-TK (herpes simplex virus thymidine kinase) promoter, EF-α promoter, CAG promoter and medicament responsive promoter. Examples of the drug responsive promoter include TRE promoter (CMV minimum promoter having a Tet-responsive sequence having 7 consecutive tetO sequences) that expresses a gene in the presence of the corresponding drug. When TRE promoter is used, it is preferable to use a mode that induces gene expression by simultaneously expressing a fusion protein of reverse tetR (rtetR) and VP16AD in the same cell in the presence of the corresponding drug (e.g., tetracycline or doxycycline). Further preferred is a vector having the TRE promoter and concurrently having a mode that expresses the fusion gene of rtetR and VP16AD in the same vector.

In the present invention, a polycistronic expression mode may also be used to simultaneously express exogenous TCRα and exogenous TCRβ. For polycistronic expression, the sequences encoding the genes may be linked by an IRES or foot-and-mouth disease virus (FMDV) 2A coding region.

In another other embodiment, the above-mentioned vector may have a transposon sequence before and after this expression cassette (gene expression unit containing a promoter, a gene sequence and a terminator) to excise as necessary the sequence encoding the exogenous TCR incorporated into the chromosome. The transposon sequence is not particularly limited, and exemplified by piggyBac. In another embodiment, to remove the expression cassette, the loxP sequence or the FRT sequence may be provided before and after the expression cassette.

When the exogenous TCR nucleic acid is in the form of an RNA, it may be introduced into pluripotent stem cells by a method such as electroporation, lipofection, microinjection, and the like.

In the production method or expansion culture method of the present invention, the CD3-positive cells to be cultured may also be cells expressing a chimeric antigen receptor.

In the present invention, the "chimeric antigen receptor (CAR)" means a fusion protein containing an antigen-binding domain, a transmembrane domain, and an intracellular signal transduction domain. The antigen-binding domain of CAR includes a short chain antibody (scFv) in which the light chain (VL) and heavy chain (VH) of the variable region of the antibody are linked in tandem via a linker (e.g., GS linker). The CD3-positive cells expressing CAR recognize the antigen in the scFV region and then transduce the recognition signal thereof into T cells through the intracellular signal transduction domain. Introduction of CAR into the CD3-positive cells makes it possible to impart specificity to the antigen of interest. In addition, since CAR can directly recognize antigen molecules without depending on HLA class I or class II, a high immune response can also be induced against cells with decreased HLA class I or class II gene expression.

Examples of the antigen targeted by the above-mentioned TCR and CAR include, but are not limited to, tumor antigens. The tumor antigen may be a tumor-specific antigen (TSA) or a tumor-associated antigen (TAA). Specific examples of such tumor antigen include one or more kinds of antigens selected from the group consisting of differentiated antigens such as MART-1/MelanA (MART-I), gp100 (Pmel 17), tyrosinase, TRP-1, TRP-2 and the like, tumor-specific multilineage antigens such as WT1, Glypican-3, MAGE-1, MAGE-3, BAGE, GAGE-1, GAGE-2, p15 and the like, fetal antigens such as CEA and the like, overexpressed tumor genes or mutated tumor suppressive genes such as p53, Ras, HER-2/neu and the like, unique tumor antigens caused by chromosome translocation such as BCR-ABL, E2A-PRL, H4-RET, IGH-IGK, MYL-RAR and the like, and virus antigens such as Epstein Barr virus antigen EBVA, human papilloma virus (HPV) antigens E6 and E7 and the like. As other tumor antigens, CD19, CD20, EGP2, erB2,3,4, GD2, GD3, Mesothelin, PSMA, 8H9, Lewis-Y, MUC1, TSP-180, MAGE-4, MAGE-5, MAGE-6, RAGE, NY-ESO, p185erbB2, p180erbB-3, c-met, nm-23H1, PSA, TAG-72, CA 19-9, CA 72-4, CAM 17.1, NuMa, K-ras, β-catenin, CDK4, Mum-1, p 15, p 16, 43-9F, 5T4, 791Tgp72, α-fetoprotein, β-HCG, BCA225, BTAA, CA 125, CA 15-3/CA 27.29/BCAA, CA 195, CA 242, CA-50, CAM43, CD68/P1, CO-029, FGF-5, G250, Ga733/EpCAM, HTgp-175, M344, MA-50, MG7-Ag, MOV18, NB/70K, NY-CO-1, RCAS1, SDCCAG16, TA-90/Mac-2 binding protein/cyclophilin C-associated protein, TAAL6, TAG72, TLP and TPS can be mentioned.

Examples of the transmembrane domain of CAR include a transmembrane domain derived from a protein selected from the group consisting of α chain, β chain or ζ chain of TCR, CD28, CD3ε chain, CD45, CD4, CD5, CD8, CD9, CD16, CD22, CD33, CD37, CD64, CD80, CD86, CD134, 4-1BB (CD137) and CD154, and the like. Among these, a transmembrane domain derived from CD8 is preferable. In addition, for example, a transmembrane domain derived from a molecule, from which the first intracellular signal transduction domain linked to an antigen binding domain is derived, is also preferably used. When, for example, the molecule from which the first intracellular signal transduction domain linked to an antigen binding domain is derived is CD28, the transmembrane domain may also be derived from CD28. Alternatively, an artificially-designed transmembrane domain may also be used.

Examples of the intracellular signal transduction domain of CAR include, but are not limited to, intracellular domains derived from one or more kinds of proteins selected from the group consisting of FcRγ chain, FcRβ chain, CD3γ chain, CD3δ chain, CD3ε chain, CD3ζ chain, CD5, CD22, CD79a, CD79b and CD66d. Among these, an intracellular domain derived from CD3ζ chain is preferable. When tyrosine phosphorylation of ITAM contained in the intracellular domain of the CD3ζ chain is triggered, a series of responses occur such as an increase in intracellular Ca²⁺ concentration, cytokine secretion, and the like, and finally, cell division occurs and CD3-positive cells show cytotoxic activity as CTL. The intracellular signal transduction domain may further contain an intracellular domain of a co-stimulating molecule. Examples of the co-stimulating molecule include, but are not limited to, intracellular domains of one or more kinds of proteins selected from the group consisting of CD27, CD28, 4-1BB (CD137), OX40, CD30, CD40, PD-1, ICOS, lymphocyte function-associated antigen-1 (LFA-1), CD2, CD7, LIGHT, NKG2C, B7-H3 and CD83. Among these, intracellular domains derived from CD28, 4-1BB (CD137), CD30 are preferable. CD28 can bind to promote T cell proliferation by increasing IL-2 production. Also, 4-1BB can promote differentiation into memory cells by suppressing apoptosis caused in the final stage of T cell activation. CD30 can further improve the proliferation of CD3-positive cells with a CD3 antibody and makes it possible to maintain the cells as CD197-positive cells for a long term. By selecting the type and number of co-stimulating molecule, the strength and duration of CAR activity can be controlled (e.g., Mol Ther. 2009; 17:1453-1464). When the intracellular signal transduction domain contains one or more kinds of intracellular domains, the order thereof is not limited.

A spacer may be incorporated between the antigen-binding domain of CAR and the transmembrane domain, or between the intracellular signal transduction domain of CAR and the transmembrane domain. As the spacer, a peptide consisting of not more than 300 amino acids, preferably 10 to 100 amino acids, and most preferably 25 to 50 amino acids, can be generally used. Specific examples thereof include, but are not limited to, a hinge region derived from IgG1, a peptide containing a CH2CH3 region of immunoglobulin and a part of CD3, and the like.

Specific examples of CAR include, but are not limited to, a first-generation CAR in which scFv and CD3ζ chain are linked via a spacer, a second-generation CAR in which a transmembrane domain and an intracellular domain derived from CD28 are incorporated between scFv of the first-generation CAR and the CD3ζ chain to enhance the T cell activation ability, and a third generation CAR in which an intracellular domain of co-stimulating molecule (4-1BB or OX40) different from CD28 is incorporated between the intracellular domain of CD28 of the second generation CAR and the CD3ζ chain.

In the production method or expansion culture method of the present invention, the CD3-positive cell to be cultured further may be cells that co-express CAR and a fusion protein of IL-15 and IL-15Rα (IL-15/IL-15Rα). The CAR expressed with IL-15/IL-15Rα may be similar to the above-mentioned CAR.

In the present invention, the "IL-15/IL-15Rα" means a fusion protein containing IL-15 and IL-15Rα. In the IL-15 signal transduction system, IL-15Rα expressed on antigen-presenting cells generally binds to IL-15 and IL-15 is presented to IL-15 receptor consisting of a common γ chain (yc) with IL-15Rβ on CD8-positive and CD4-negative cell (trans-presentation), whereby the cytotoxic activity of CD8-positive CD4-negative cell is maintained. Therefore, when the CD3-positive cell expressing IL-15/IL-15Rα is CD8-positive CD4-negative, the cell can transmit the IL-15 signal into its own cell via the IL-15 receptor. Alternatively, the CD3-positive cell expressing IL-15/IL-15Rα can transmit the IL-15 signal into other CD8-positive CD4-negative cells via the IL-15 receptor. As described above, since IL-15/IL-15Rα can maintain cytotoxic activity of CD8-positive CD4-negative cell, it is expected to show a continuous cytotoxic effect on cells targeted by CAR.

IL-15/IL-15Rα may be a transmembrane type protein or a secretor protein. It is known that, in IL-15Rα, the IL-15 binding domain of 1-65 amino acids from the N-terminal of the mature protein is the region responsible for binding to IL-15 (Wei X. et al., J. Immunol., 167: 277-282, 2001). Therefore, the transmembrane type protein may be a protein that retains the IL-15 binding domain and retains the transmembrane domain of IL-15Rα. On the other hand, the secretor protein may be a protein that maintains the IL-15 binding domain and lacks the transmembrane domain of IL-15Rα.

A spacer may be incorporated between IL-15 and IL-15Rα of IL-15/IL-15Rα. As the spacer, a peptide generally consisting of not more than 300 amino acids, preferably 10 - 100 amino acids, most preferably 20 - 50 amino acids, can be used. Specific examples thereof include, but are not limited to, GS linker and the like.

IL-15/IL-15Rα is not particularly limited as long as it is a peptide in which IL-15 and IL-15Rα are bonded via a spacer, and specific examples thereof include a peptide consisting of SEQ ID NO: 8. While IL-15/IL-15R is not particularly limited as long as it can bind to the IL-15 receptor and transmit the IL-15 signal into the cell, for example, a peptide containing an amino acid sequence having a homology of not less than about 90%, preferably not less than about 95%, more preferably not less than about 97%, particularly preferably not less than about 98%, most preferably not less than about 99%, with the amino acid sequence shown in SEQ ID NO: 8 can be mentioned. As used herein, the "homology" means the proportion (%) of the same amino acid and similar amino acid residue to all overlapping amino acid residues in the optimal alignment where two amino acid sequences are aligned using a mathematic algorithm known in the relevant technical field (preferably, the algorithm is such that a gap can be introduced into one or both of the sequences for the optimal alignment). The "similar amino acid" means amino acids having similar physicochemical properties and, for example, amino acids classified in the same group such as aromatic amino acids (Phe, Trp, Tyr), aliphatic amino acids (Ala, Leu, Ile, Val), polar amino acids (Gin, Asn), basic amino acids (Lys, Arg, His), acidic amino acids (Glu, Asp), amino acids having a hydroxyl group (Ser, Thr), amino acids having a small side chain (Gly, Ala, Ser, Thr, Met) and the like can be mentioned. It is predicted that the substitution with such similar amino acids does not change the phenotype of the protein (that is, conservative amino acid substitution). Specific examples of the conservative amino acid substitution are well known in the technical field and are described in various documents (e.g., Bowie et al., Science, 247: 1306-1310 (1990)). The homology of the amino acid sequence in the present specification can be calculated using homology calculation algorithm NCBI BLAST (National Center for Biotechnology Information Basic Local Alignment Search Tool), and under the following conditions (expectancy =10; accept gap; matrix =BLOSUM62; filtering =OFF).

When chimeric antigen receptor and/or IL-15/IL-15Rα are/is expressed in the CD3-positive cell cultured in the production method or expansion culture method of the present invention, a method for introducing the CAR nucleic acid and/or IL-15/IL-15Rα nucleic acid into the cells (e.g., pluripotent stem cell) is not particularly limited and, for example, the same method as the method of introducing TCR nucleic acid can be used.

When the CD3-positive cell to be cultured in the production method or expansion culture method of the present invention are cells obtained by differentiating pluripotent stem cells, the pluripotent stem cells can be differentiated into CD3-positive cells by a method known per se. Specific methods for differentiating pluripotent stem cells into CD3-positive cells are described, for example, in WO 2016/076415 and WO 2017/221975. A specific method for differentiating pluripotent stem cells into CD3-positive cells may include, for example, (1) a process for differentiating pluripotent stem cells into hematopoietic progenitor cells, and (2) a process for differentiating the hematopoietic progenitor cells into CD3-positive cells.

### (1) Process for differentiating induced pluripotent stem cells into hematopoietic progenitor cells

In step (1), the hematopoietic progenitor cell (HPC) means CD34-positive cell, preferably, CD34positive CD43-positive (DP) cell. In the step (1), hematopoietic progenitor cell and hematopoietic stem cell are not distinguished and show the same cell unless particularly indicated.

The method of differentiating pluripotent stem cells into hematopoietic progenitor cells is not particularly limited as long as it can cause differentiation into hematopoietic progenitor cells. Examples thereof include a method including culturing pluripotent stem cells in a medium for induction of hematopoietic progenitor cells, as described in, for example, WO 2013/075222, WO 2016/076415 and Liu S. et al., Cytotherapy, 17 (2015); 344-358 and the like.

In step (1), a medium used for induction into hematopoietic progenitor cells is not particularly limited. A medium used for culturing animal cells can be prepared into a basal medium. Examples of the basal medium include, but are not limited to, Dulbecco's Medium (e.g., IMDM), Eagle's medium (e.g., DMEM, EMEM, BME, MEM, αMEM), Ham's medium (e.g., F10 medium, F12 medium), RPMI medium (e.g., RPMI-1640 medium, RPMI-1630 medium), MCDB medium (e.g., MCDB104, 107, 131, 151, 153 medium), Fischer's medium, 199 medium, culture medium for primate ES cell (culture medium for primate ES/iPS cell, Reprocell), medium for mouse ES cell (TX-WES culture medium, Thromb-X), serum-free medium (mTeSR, Stemcell Technologies), ReproFF, StemSpan (registered trade mark) SFEM, StemSpan (registered trade mark) H3000, StemlineII, ESF-B medium, ESF-C medium, CSTI-7 medium, Neurobasal medium (Life Technologies, Inc.), StemPro-34 medium, StemFit (registered trade mark) (e.g., StemFit AK03N, StemFit AK02N) and the like. Furthermore, these media can be mixed as necessary and used and, for example, DMEM/F12 medium and the like can be mentioned. The basal medium used may contain a serum, or may be serum-free. The basal medium may be appropriately supplemented with 10 - 20% serum (fetal bovine serum (FBS), human serum, horse serum) or a serum replacement (KSR and the like), insulin, various vitamins, L-glutamine, various amino acids such as non-essential amino acid and the like, 2-mercaptoethanol, various cytokines (interleukins (IL-2, IL-7, IL-15 etc.), SCF (Stem cell factor), activin and the like), various hormones, various growth factors (Leukemia inhibitory factor (LIF), basic fibroblast growth factor (bFGF), TGF-β etc.), various extracellular matrices, various cell adhesion molecules, antibiotics such as penicillin/streptomycin, puromycin and the like, pH indicator such as phenol red and the like, and the like. If necessary, the basal medium may also contain Vitamin C (e.g., ascorbic acid), albumin, insulin, transferrin, selenium compound (e.g., sodium selenite), fatty acid, trace elements, 2-mercaptoethanol, thioglycerol (e.g., α-monothioglycerol (MTG)), lipids, L-alanyl-L-glutamine (e.g., Glutamax (registered trade mark)), growth factors, low-molecular-weight compounds, antibiotics, antioxidants, pyruvic acid, buffers, inorganic salts, and the like.

In step (1), "Vitamin C" means L-ascorbic acid and derivatives thereof, and "L-ascorbic acid derivative" means derivatives that become vitamin C by enzymatic reaction in the living body. Examples of the derivatives of L-ascorbic acid used in step (1) include vitamin C phosphate, ascorbic acid glucoside, ascorbyl ethyl, vitamin C ester, ascorbyl tetrahexyldecanoate, ascorbyl stearate, and ascorbyl 2-phosphate 6-palmitate. Preferred is vitamin C phosphate. Examples of the vitamin C phosphate (e.g., ascorbic acid 2-phosphate) include salts of L-ascorbic acid phosphate such as L-ascorbic acid phosphate Na and L-ascorbic acid phosphate Mg.

When Vitamin C is used, the Vitamin C is preferably added (supplied) every four days, every three days, every two days, or every day. Vitamin C is more preferably added every day. In one embodiment, Vitamin C is preferably added to the medium at an amount corresponding to 5 ng/ml to 500 ng/ml (e.g., an amount corresponding to 5 ng/ml, 10 ng/ml, 25 ng/ml, 50 ng/ml, 100 ng/ml, 200 ng/ml, 300 ng/ml, 400 ng/ml, or 500 ng/ml). In another embodiment, Vitamin C is added to the culture medium at an amount corresponding to 5 µg/ml - 500 µg/ml (e.g., an amount corresponding to 5 µg/ml, 10 µg/ml, 25 µg/ml, 50 µg/ml, 100 µg/ml, 200 µg/ml, 300 µg/ml, 400 µg/ml, 500 µg/ml).

The medium to be used in step (1) may be further supplemented with at least one kind of cytokine selected from the group consisting of BMP4 (Bone morphogenetic protein 4), VEGF (vascular endothelial growth factor), SCF (Stem cell factor), TPO (thrombopoietin), FLT-3L (Flt3 Ligand) and bFGF (basic fibroblast growth factor). It is more preferably a culture supplemented with BMP4, VEGF and bFGF, and further preferably a culture supplemented with BMP4, VEGF, SCF and bFGF.

When cytokine is used, its concentration in the medium may be, for example, 5 ng/ml - 500 ng/ml for BMP4, 5 ng/ml - 500 ng/ml for VEGF, 5 ng/ml - 500 ng/ml for SCF, 3 ng/ml - 300 ng/ml for TPO, 1 ng/ml - 100 ng/ml for FLT-3L, and 5 ng/ml - 500 ng/ml for bFGF.

The medium may be supplemented with a TGFβ inhibitor. The TGFβ inhibitor is a small molecule inhibitor that interferes with the signal transduction of TGFβ family and includes, for example, SB431542, SB202190 (both R.K. Lindemann et al., Mol. Cancer 2:20 (2003)), SB505124 (GlaxoSmithKline), NPC30345, SD093, SD908, SD208 (Scios), LY2109761, LY364947, LY580276 (Lilly Research Laboratories) and the like. For example, when the TGFβ inhibitor is SB431542, its concentration in the medium is preferably 0.5 µM - 100 µM.

The induced pluripotent stem cells may be cultured by adherent culture or suspension culture. In cases of adherent culture, the culturing may be carried out in a culture vessel coated with an extracellular matrix component, and/or may be co-cultured with feeder cells. While the feeder cell is not particularly limited, for example, fibroblast (mouse embryo fibroblast (MEF), mouse fibroblast (STO) and the like) can be mentioned. Feeder cells are preferably inactivated by a method known per se, for example, radiation (gamma-ray and the like) irradiation, treatment with anti-cancer agent (mitomycin C and the like) and the like. As the extracellular matrix component, fibrous proteins such as Matrigel (Niwa A, et al., PLoS One.6(7): e22261, 2011), gelatin, collagen, elastin and the like, glucosaminoglycan and proteoglycan such as hyaluronic acid, chondroitin sulfate and the like, cell adhesion proteins such as fibronectin, vitronectin, laminin and the like, and the like can be mentioned.

Suspension culture means culturing cells in a state of non-adhesion to a culture container and is not particularly limited. To improve adhesiveness to the cells, a culture container free of an artificial treatment (e.g., coating treatment with extracellular matrix and the like), or a culture container subjected to a treatment for artificially suppressing adhesion (e.g., coating treatment with polyhydroxyethyl methacrylic acid (poly-HEMA) or non-ionic surface-active polyol (Pluronic F-127 etc.)) can be used. In the suspension culture, embryoid (EB) is preferably formed and cultured.

Hematopoietic progenitor cell can also be prepared from a sac-like structure (to be also referred to as ES-sac or iPS-sac) obtained by culturing pluripotent stem cells. As used herein, the "sac-like structure" is a pluripotent stem cell-derived three-dimensional saccular (with spaces inside) structure, which is formed by an endothelial cell population and the like and contains hematopoietic progenitor cells in the inside thereof.

The temperature conditions in step (1) are not particularly limited. The temperature is, for example, about 37°C to about 42°C, preferably about 37°C to about 39°C. The culture period may be appropriately determined by those skilled in the art by monitoring of the number of hematopoietic progenitor cells and/or the like. The number of days of the culture is not limited as long as hematopoietic progenitor cells can be obtained. Examples of the culture period include at least 6 days, not less than 7 days, not less than 8 days, not less than 9 days, not less than 10 days, not less than 11 days, not less than 12 days, not less than 13 days, and not less than 14 days. The culture period is preferably 14 days. While a longer culture period generally does not pose a problem in the production of hematopoietic progenitor cells, it is preferably not more than 35 days, more preferably not more than 21 days. The culture may be carried out under low-oxygen conditions, and the low-oxygen condition in the present specification means, for example, oxygen concentration of 15%, 10%, 9%, 8%, 7%, 6%, 5% or lower than these.

### (2) Step of differentiating hematopoietic progenitor cells into CD3-positive cells

A method for differentiating hematopoietic progenitor cells into CD3-positive cells is not particularly limited as long as it can differentiate hematopoietic progenitor cells into CD3-positive cells. Examples thereof include a method for culturing hematopoietic progenitor cells under the same culture conditions as those in a method of inducing T cells from hematopoietic progenitor cells, as described in WO 2016/076415, WO 2017/221975 and the like.

In step (2), a medium for inducing differentiation into CD3-positive T cell is not particularly limited, and a medium used for culturing animal cells can be prepared into a basal medium. Examples of the basal medium include those similar to the basal medium used in the above-mentioned step (1). The medium may contain serum, or may be serum-free. If necessary, the basal medium may also contain Vitamin C (e.g., ascorbic acid), albumin, insulin, transferrin, selenium compound (e.g., sodium selenite), fatty acid, trace elements, 2-mercaptoethanol, thioglycerol (e.g., α-monothioglycerol (MTG)), lipids, amino acids, L-glutamine, L-alanyl-L-glutamine (e.g., Glutamax (registered trade mark)), non-essential amino acids, vitamins, growth factors, low-molecular-weight compounds, antibiotics (e.g., penicillin, streptomycin), antioxidants, pyruvic acid, buffers, inorganic salts, cytokines, and the like.

When vitamin C is used in step (2), Vitamin C may be the same as that described in step (2-1) and can be added similarly. In one embodiment, the concentration of vitamin C in the medium or culture medium is preferably 5 µg/ml - 200 µg/ml. In another embodiment, vitamin C is added to the culture medium at an amount corresponding to 5 µg/ml - 500 µg/ml (e.g., amount corresponding to 5 µg/ml, 10 µg/ml, 25 µg/ml, 50 µg/ml, 100 µg/ml, 200 µg/ml, 300 µg/ml, 400 µg/ml, 500 ug/ml).

In step (2), p38 inhibitor and/or SDF-1 (Stromal cell-derived factor 1) are/is preferable. In the present specification, the "p38 inhibitor" means a substance that inhibits the functions of p38 protein (p38 MAP kinase). Examples thereof include, but are not limited to, chemical inhibitor of p38, dominant-negative mutant of p38 or nucleic acid encoding same and the like.

Examples of the chemical inhibitor of p38 to be used in step (2) include, but are not limited to, SB203580 (4-(4-fluorophenyl)-2-(4-methylsulfonylphenyl)-5-(4-pyridyl)-1H-imidazole), and a derivative thereof, SB202190 (4-(4-fluorophenyl)-2-(4-hydroxyphenyl)-5-(4-pyridyl)-1H-imidazole) and a derivative thereof, SB239063 (trans-4-[4-(4-fluorophenyl)-5-(2-methoxy-4-pyrimidinyl)-1H-imidazol-1-yl]cyclohexanol) and a derivative thereof, SB220025 and a derivative thereof, PD169316, RPR200765A, AMG-548, BIRB-796, SClO-469, SCIO-323, VX-702 and FR167653. These compounds are commercially available and, for example, SB203580, SB202190, SB239063, SB220025 and PD169316 are available from Calbiochem, and SClO-469 and SCIO-323 are available from Scios and the like. The chemical inhibitor of p38 is preferably SB203580 (4-(4-fluorophenyl)-2-(4-methylsulfonylphenyl)-5-(4-pyridyl)-1H-imidazole), or a derivative thereof.

Examples of the dominant-negative mutant of p38 in step (2) include p38T180A obtained by point mutation of the 180-position threonine located in the DNA binding region of p38 to alanine, p38Y182F obtained by point mutation of the 182-position tyrosine of p38 in human and mouse to phenylalanine and the like. The p38 inhibitor is contained in a medium at, for example, about 1 µM - about 50 µM. When SB203580 is used as the P38 inhibitor, it may be contained in a medium at 1 µM - 50 µM, 5 µM - 30 µM, 10 µM - 20 µM.

SDF-1 in step (2) may be not only SDF-1α or a mature form thereof, but also an isoform such as SDF-1β, SDF-1γ, SDF-1δ, SDF-1ε, SDF-1φ and the like or a mature form thereof, or a mixture of these at any ratio or the like. Preferably, SDF-1α is used. SDF-1 is sometimes referred to as CXCL-12 or PBSF.

In step (2), one or several amino acids in the amino acid sequence of SDF-1 may be substituted, deleted, added and/or inserted as long as it has the activity as the chemokine (SDF-1 with such substitution, deletion, addition and/or insertion of amino acid is to be also referred to as "SDF-1 mutant"). Similarly, sugar chain may be substituted, deleted and/or added in SDF-1 or SDF-1 mutant. Examples of the mutant of the above-mentioned SDF-1 include those maintaining at least 4 cysteine residues (Cys30, Cys32, Cys55 and Cys71 in human SDF-1α) and having not less than 90% identity with amino acid sequence of a natural substance, though the amino acid mutation is not limited thereto. SDF-1 may be obtained from a mammal, for example, human or non-human mammal such as monkey, sheep, bovine, horse, swine, dog, cat, rabbit, rat, mouse and the like. For example, the protein registered as GenBank accession number:NP_954637 can be used as human SDF-1α, and the protein registered as GenBank accession number:NP_000600 can be used as SDF-1β.

SDF-1 may be commercially available, purified from nature, or produced by peptide synthesis or genetic engineering techniques. SDF-1 is contained in a medium within the range of, for example, about 10 ng/ml to about 100 ng/ml. In addition, SDF-1 alternative having an SDF-1-like activity can also be used instead of SDF-1. Examples of such SDF-1 alternative include CXCR4 agonist, and a low-molecular-weight compound having a CXCR4 agonist activity and the like may be added to the medium instead of SDF-1.

The culture medium used in step (2) may be further supplemented with at least one kind, preferably all, of cytokine selected from the group consisting of SCF, TPO (thrombopoietin), FLT-3L and IL-7. The concentration of these is, for example, 10 ng/ml to 100 ng/ml for SCF, 10 ng/ml to 200 ng/ml for TPO, 1 ng/ml to 100 ng/ml for IL-7, and 1 ng/ml to 100 ng/ml for FLT-3L.

In step (2), the hematopoietic progenitor cells may be cultured by adherent culture or suspension culture. In cases of adherent culture, a coated culture vessel may be used, and/or the hematopoietic progenitor cells may be co-cultured with feeder cells and/or the like. Examples of the feeder cells for the co-culture include a bone-marrow stromal cell line, OP9 cells (available from Riken BioResource Center). The OP9 cell is preferably OP9-DL4 cell or OP9-DL1 cell, which constantly expresses DLL4 or DLL1 (e.g., Holmes R I and Zuniga-Pflucker J C. Cold Spring Harb Protoc. 2009(2)). In step (2), in cases where OP9 cells are used as the feeder cells, DLL1, or a separately-prepared DLL4 or DLL1, or a fusion protein of DLL4 or DLL1, and Fc or the like, may be added to the medium to perform the co-culture. When feeder cells are used, the feeder cells are preferably appropriately replaced during the culture. The replacement of the feeder cells may be carried out by transferring the subject cells that are being cultured onto feeder cells that are preliminarily plated. The replacement may be carried out every five days, every four days, every three days, or every two days. When hematopoietic progenitor cells are obtained by suspension culture of embryoid, it is preferable to perform adhesion culture after dissociation into single cells. While the cells may be co-cultured with feeder cells, culturing is preferably carried out without using feeder cells.

In the case of adhesion culture and when a culture container is coated, examples of the coating agent include Matrigel (Niwa A, et al. PLos One, 6(7):e22261, 2011)), collagen, gelatin, laminin, heparan sulfuric acid proteoglycan, RetroNectin, fusion protein of DLL4 or DLL1, or DLL4 or DLL1, and Fc region of antibody (hereinafter sometimes referred to as Fc) and the like (e.g., DLL4/Fc chimera), entactin, and/or combination of these, and a combination of RetroNectin and fusion protein of DLL4 and Fc etc. is preferable.

In step (2), the culture temperature conditions are not limited. The temperature is, for example, about 37°C to about 42°C, preferably about 37 to about 39°C. The culture period may be appropriately determined by those skilled in the art by monitoring of the number of CD3-positive cells and the like. The number of days of the culture is not limited as long as CD3-positive cells can be obtained. Examples of the culture period include at least not less than 10 days, not less than 12 days, not less than 14 days, not less than 16 days, not less than 18 days, not less than 20 days, not less than 22 days, and not less than 23 days. The culture period is preferably 21 days. In addition, not more than 90 days is preferable, and not more than 42 days is more preferable.

The cells obtained by the above steps include CD3-positive cells. A specific method for differentiating pluripotent stem cells into CD3-positive cells may further include the following step (3).

### (3) Step of obtaining CD3-positive CD8-positive CD4-negative cell (or CD3-positive CD8-positive CD4-negative CD30-positive cell) or step of enriching CD3-positive cell

Examples of the method for obtaining CD3-positive CD8-positive CD4-negative cell (or CD3-positive CD8-positive CD4-negative CD30-positive cell) include the methods described in WO 2016/076415, WO 2017/221975 and the like. CD3-positive cell may also be enriched using a similar method.

In step (3), a medium used for obtaining CD3-positive CD8-positive CD4-negative cell (or CD3-positive CD8-positive CD4-negative CD30-positive cell) or enriching CD3-positive cell is not particularly limited, and a medium used for culturing animal cells can be prepared into a basal medium. Examples of the basal medium include those similar to the basal medium used in the above-mentioned step (1). The medium may contain serum, or may be serum-free. If necessary, the basal medium may also contain Vitamin C (e.g., ascorbic acid), albumin, insulin, transferrin, selenium compound (e.g., sodium selenite), fatty acid, trace elements, 2-mercaptoethanol, thioglycerol (e.g., α-monothioglycerol (MTG)), lipids, amino acids, L-glutamine, L-alanyl-L-glutamine (e.g., Glutamax (registered trade mark)), non-essential amino acids, vitamins, growth factors, low-molecular-weight compounds, antibiotics (e.g., penicillin, streptomycin), antioxidants, pyruvic acid, buffers, inorganic salts, cytokines, hormone, and the like.

When Vitamin C is used in step (3), Vitamin C may be similar to those described in step (1) and can be added similarly. In one embodiment, the concentration of Vitamin C in the medium or culture medium is preferably 5 µg/ml - 200 µg/ml. In another embodiment, vitamin C is added in an amount corresponding to 5 µg/ml - 500 µg/ml in the culture medium (e.g., amount corresponding to 5 µg/ml, 10 µg/ml, 25 µg/ml, 50 µg/ml, 100 µg/ml, 200 µg/ml, 300 µg/ml, 400 µg/ml, 500 µg/ml).

When hormone is used in step (3), examples of the hormone include corticosteroid. Corticosteroid is glucocorticoid or a derivative thereof, and cortisone acetate, hydrocortisone, fludrocortisone acetate, predonisolone, triamcinolone, methylprednisolone, dexamethasone, betamethasone, and beclometasone dipropionate are recited as examples. Preferred is dexamethasone. When corticosteroid is dexamethasone, its concentration in the medium is 1 nM - 100 nM.

In step (3), the medium contains a CD3/TCR complex agonist. The CD3/TCR complex agonist is not particularly limited as long as it is a molecule capable of transducing a signal from a CD3/TCR complex to a CD3-positive cell by specifically binding to at least a part of the CD3/TCR complex. Examples of the CD3/TCR complex agonist include CD3 agonist and/or TCR agonist. As the CD3 agonist, an anti-CD3 agonist antibody or a binding fragment thereof can be mentioned, and as the TCR agonist, at least one selected from the group consisting of an anti-TCR agonist antibody or a binding fragment thereof, an MHC/antigen peptide complex or a multimer thereof, and an MHC/superantigen complex or a multimer thereof can be mentioned. When an anti-CD3 agonist antibody is used, the anti-CD3 agonist antibody includes both a polyclonal antibody and a monoclonal antibody, preferably a monoclonal antibody. The antibody may belong to any immunoglobulin class of IgG, IgA, IgM, IgD and IgE, preferably IgG. As the anti-CD3 agonist antibody, an antibody (OKT3) produced from OKT3 clone, an antibody (UCHT1) produced from UCHT1 clone and the like can be mentioned, and it is preferably UCHT1. When the anti-CD3 agonist antibody is OKT3, OKT3 includes RYTMH (SEQ ID NO: 9), YINPSRGYTNYNQKFKD (SEQ ID NO: 10), and YYDDHYCLDY (SEQ ID NO: 11) as complementarity determining regions 1 - 3 in the heavy chain variable region, and SASSSVSYMN (SEQ ID NO: 12), DTSKLAS (SEQ ID NO: 13), and QQWSSNPFT (SEQ ID NO: 14) as complementarity determining regions 1 - 3 in the light chain variable region. In addition, OKT3 preferably includes a heavy chain variable region containing the amino acid sequence shown in SEQ ID NO: 21 and a light chain variable region containing the amino acid sequence shown in SEQ ID NO: 22. When the anti-CD3 agonist antibody is UCHT1, UCHT1 includes GYSFTGYTMN (SEQ ID NO: 15), LINPYKGVST (SEQ ID NO: 16), and SGYYGDSDWYFDV (SEQ ID NO: 17) as complementarity determining regions 1 - 3 in the heavy chain variable region, and RASQDIRNYLN (SEQ ID NO: 18), YTSRLHS (SEQ ID NO: 19), and QQGNTLPWT (SEQ ID NO: 20) as complementarity determining regions 1 - 3 in the light chain variable region. In addition, UCHT1 preferably includes a heavy chain variable region containing the amino acid sequence shown in SEQ ID NO: 23 and a light chain variable region containing the amino acid sequence shown in SEQ ID NO: 24. The concentration of anti-CD3 agonist antibody in the medium is, for example, 10 ng/ml - 1000 ng/ml, preferably 50 ng/ml - 800 ng/ml, more preferably 250 ng/ml - 600 ng/ml.

When cytokine is used in step (3), IL-2 and IL-7 and the like can be mentioned as the cytokine. When cytokine is IL-2, the concentration thereof in the medium is 10 U/ml - 1000 U/mL, and when it is IL-7, the concentration thereof in the medium is 1 ng/ml - 1000 ng/mL.

In step (3), the culture temperature conditions are not particularly limited. The temperature is, for example, about 37°C to about 42°C, preferably about 37°C to about 39°C. The culture period may be appropriately determined by those skilled in the art by monitoring of the number of CD3-positive cells and the like. The number of days of the culture is not limited as long as CD3-positive cell cells can be obtained. The culture period is, for example, at least 1 day or more, 2 days or more, 3 days or more, 4 days or more, 5 days or more, and preferably 6 days. It is preferably 28 days or less, more preferably 14 days or less.

As described above, by differentiating pluripotent stem cells, CD3-positive cells to be cultured by the production method or expansion culture method of the present invention can be obtained.

The production method or expansion culture method of the present invention includes a step of culturing CD3-positive cells in the presence of a CD3/TCR complex agonist, fibronectin or a variant thereof, and a CD30 agonist (hereinafter to be referred to as step (I) of the present invention).

In step (I) of the present invention, the CD3/TCR complex agonist may be the same as the CD3/TCR complex agonist used in the step (3).

In step (I) of the present invention (or the step (3)), when the CD3/TCR complex agonist to be used is an anti-CD3 agonist antibody or an anti-TCR antibody, the polyclonal antibodies thereof can be produced, for example, by the following method. For example, in the case of polyclonal antibody to CD3, mammals such as rabbit, mouse, rat, goat, guinea pig, hamster and the like are immunized (1 to several boosters every about 1 - 4 weeks from the initial immunization) with a mixture of a partial peptide (e.g., γ, δ, ε, ζη chain) containing CD3 or an epitope thereof and complete or incomplete Freund's adjuvant (FCA or FIA) as an immunization antigen, and in the case of polyclonal antibody to TCR, the mammals are immunized with a mixture of a partial peptide (e.g., α, β, γδ chain) containing TCR or an epitope thereof and complete or incomplete Freund's adjuvant (FCA or FIA) as an immunization antigen. About 3 to 10 days after each additional immunization, the antibody titer of the partially collected serum is measured using a conventionally known antigen-antibody reaction, and its increase is confirmed. In addition, about 3-10 days after final immunization, whole blood is collected and antiserum is purified. The polyclonal antibody can also be purified as a single immunoglobulin class by using a conventional separation technique such as salting out (e.g., ammonium sulfate fractionation), centrifugation, dialysis, and column chromatography.

When the anti-CD3 agonist antibody or anti-TCR antibody used in step (I) of the present invention (or the step (3)) is a monoclonal antibody, the monoclonal antibody can be generally obtained from hybridomas (fusion cells) produced by cell fusion. That is, as in the above-mentioned polyclonal antibody, it can be produced by isolating antibody-producing cells from a mammal immunosensitized to a partial peptide containing CD3 or TCR or an epitope thereof, fusing same with myeloma cells to form hybridoma, cloning the hybridoma, and selecting, as a marker antigen, a clone that produces an antibody that shows specific affinity for CD3 or TCR. In addition, an antibody-producing cell produced by reacting CD3 with splenocytes or lymphocytes isolated in advance in a culture medium can also be used. In this case, human-derived antibody-producing cells can also be prepared.

Hybridoma that secretes monoclonal antibody can be prepared according to the method of Kohler and Milstein (Nature, Vol. 256, pp. 495-497, 1975) or a modified method thereof. That is, monoclonal antibody is prepared by culturing hybridoma obtained by fusing antibody-producing cells contained in splenocytes, thymocytes, lymph node cells, peripheral lymphocytes, myeloma cells or tonsillaria, preferably splenocytes, obtained from animals immunosensitized as described above, with preferably myeloma cells of allogenic mammals such as mouse, rat, guinea pig, hamster, rabbit or human, more preferably mouse, rat or human. Culture can be performed in vitro or in vivo in mammals such as mouse, rat, guinea pig, hamster, rabbit, preferably mouse or rat, more preferably intraperitoneally in mice, and the antibody can be obtained from the culture supernatant or ascites of mammal.

Examples of the myeloma cell used for cell fusion include mouse-derived myeloma (e.g., P3-NSI-1-Ag4-1, P3-X63-Ag8-U1, P3-X63-Ag8-653, SP2/0-Ag14, BW5147 and the like), rat-derived myeloma (e.g., 210RCY3-Ag1.2.3 and the like), human-derived myeloma (e.g., U-266AR1, GML500-6TG-A1-2, UC729-6, CEM-AGR, D1R11 and CEM-T15 and the like) and the like.

A hybridoma clone that produces the monoclonal antibody can be screened for by culturing hybridoma in, for example, a micro titer plate, and measuring the reactivity of the culture supernatant in the well, which showed proliferation, to CD3 by radioimmunoassay, enzyme immunoassay, fluorescence immunoassay and the like.

The isolation and purification of the monoclonal antibody can be performed by subjecting the antibody-containing culture supernatant or ascites produced by the method described above to ion exchange chromatography, affinity column chromatography such as an anti-immunoglobulin column or protein G column.

The monoclonal antibody used in step (I) of the present invention (or the step (3)) of the present invention is not may be obtained by any method without limited to the production method. While a monoclonal antibody generally has a sugar chain having a different structure according to the kind of mammal to be immunosensitized, the monoclonal antibody in the present invention is not limited by the structural difference of the sugar chain and includes monoclonal antibodies derived from any mammals.

The anti-CD3 agonist antibody or anti-TCR antibody includes the polyclonal antibody, natural antibody such as monoclonal antibody (mAb), chimeric antibody (humanized antibody) that can be produced using gene recombination technology, single strand antibody, and binding fragments of these antibodies. The binding fragment of an antibody means a region of a part of the antibody having specific binding activity, and specifically includes Fab, Fab', F(ab')₂, scAb, scFv, scFv-Fc, and the like.

In addition, those skilled in the art can produce a fusion antibody of an anti-CD3 agonist antibody or an anti-TCR antibody or a binding fragment thereof with other peptide or protein, or a modified antibody to which a modifying agent is bound. Other peptides and proteins used for fusion are not particularly limited as long as they do not reduce the binding activity of the antibody. For example, human serum albumin, various tag peptides, artificial helix motif peptides, maltose binding proteins, glutathione S transferase, various toxins, other peptides or proteins that can promote multimerization, and the like can be mentioned. The modifying agent used for the modification is not particularly limited as long as it does not reduce the binding activity of the antibody, and examples thereof include polyethylene glycol, sugar chain, phospholipid, liposome, low-molecular-weight compound and the like.

As the anti-CD3 agonist antibody, a reagent that can be purchased can also be used. The anti-CD3 agonist antibody that can be purchased is not particularly limited, and, for example, an antibody (OKT3) produced from an OKT3 clone (anti-human CD3 functional grade purified (Clone: OKT3) (eBioscience)), an antibody (UCHT1) produced from UCHT1 clone (CD3 antibody (Clone: UCHT1) (GeneTex)), and the like can be used, and UCHT1 can be preferably used.

When the CD3/TCR complex agonist used in step (I) of the present invention (or the step (3)) is an MHC/antigen peptide complex, the MHC/antigen peptide complex is not particularly limited as long as it is a molecule specifically recognized by CD3/TCR complex of CD3-positive cells and the molecule can transmit a signal into CD3-positive cells.

The MHC constituting the MHC/antigen peptide complex may be MHC class I or MHC class II, preferably MHC class I.

In step (I) of the present invention (or the step (3)), MHC class I is not particularly limited as long as it is a molecule that forms a complex with an antigen peptide, is recognized by the CD3/TCR complex and CD8, and transmits a signal into CD3-positive cells. MHC class I is, for example, a dimer composed α chain (HLA-A, HLA-B, HLA-C, HLA-E, HLA-F or HLA-G for human, H-2K, H-2D or H-2L for mouse) and β₂ microglobulin, preferably, a dimer composed of HLA-A, HLA-B, HLA-C, HLA-E, HLA-F or HLA-G, and β₂ microglobulin, more preferably, a dimer composed of HLA-A, HLA-B or HLA-C, and β₂ microglobulin. In the production method or the expansion culture method of the present invention, specific examples of the MHC class I include, but are not limited to, HLA-A*02:01, HLA-A*24:02, HLA-A*01:01, and the like. The antigen peptide is not particularly limited as long as it is a peptide presented by MHC class I. As the antigen peptide presented by MHC class I, for example, HER-2/neu, MART-1, NY-ESO-1, Gp-100, MUC-1, p53, prostate-specific antigen (PSA), hTERT, WT1, survivin, CEA, MAGE-3, peptide derived from protein derived from a group of viruses strongly related to the development of malignant tumor, and the like can be mentioned, and WT1-derived peptide is preferable. As specific WT1-derived peptide, RMFPNAPYL (SEQ ID NO: 1), SLGEQQYSV (SEQ ID NO: 2), CMTWNQMNL (SEQ ID NO: 3) (modified peptide thereof CYTWNQMNL (SEQ ID NO: 4)) and the like can be mentioned.

In step (I) of the present invention (or the step (3)), MHC class II is not particularly limited as long as it is a molecule that forms a complex with an antigen peptide, is recognized by the CD3/TCR complex and CD4, and transmits a signal into CD3-positive cells. MHC class II is, for example, HLA-DR, HLA-DQ or HLA-DP for human, and H-2A or H-2B for mouse, each of which is a dimer composed of α chain and β chain (e.g., HLA-DR is composed of HLA-DRA as α chain and HLA-DRB1 as β chain), preferably, HLA-DR, HLA-DQ or HLA-DP. The antigen peptide is not particularly limited as long as it is a peptide presented by MHC class II. As the antigen peptide presented by MHC class II, for example, WT1, PSA, MAGE-3, CEA, survivin, tyrosinase, peptide derived from protein derived from a group of viruses strongly related to the development of malignant tumor, and the like can be mentioned, and WT1-derived peptide is preferable.

MHC and/or antigen peptide (hereinafter to be indicated as MHC and the like) may be a chemically-synthesized protein or a protein biochemically synthesized in a cell-free translation system. Alternatively, the protein may be a recombinant protein produced from a transformant incorporating a nucleic acid having a base sequence that encodes MHC and the like.

When MHC and the like are produced according to a known method of peptide synthesis, for example, any of a solid phase synthesis method and a liquid phase synthesis method may be used. The desired protein can be produced by condensing an amino acid derivative having a protecting group attached to a carboxy group and a functional group of a side chain, and an amino acid derivative having a protected amino group and a protected functional group of a side chain, and removing the protecting groups.

Condensation and removal of protecting groups are performed according to a method known per se, for example, the methods described in (1) - (5) below.
(1) M. Bodanszky and M.A. Ondetti: Peptide Synthesis, Interscience Publishers, New York (1966)
(2) Schroeder and Luebke: The Peptide, Academic Press, New York (1965)
(3) Nobuo Izumiya, et al.: Peptide Gosei-no-Kiso to Jikken (Basics and experiments of peptide synthesis), published by Maruzen Co. (1975)
(4) Haruaki Yajima and Shunpei Sakakibara: Seikagaku Jikken Koza (Biochemical Experiment) 1, Tanpakushitsu no Kagaku (Chemistry of Proteins) IV, 205 (1977)
(5) Haruaki Yajima, ed.: Zoku Iyakuhin no Kaihatsu (A sequel to Development of Pharmaceuticals), Vol. 14, Peptide Synthesis, published by Hirokawa Shoten.

MHC and the like thus obtained can be purified or isolated by a known method of purification. Here, as examples of the method of purification, solvent extraction, distillation, column chromatography, liquid chromatography, recrystallization, combinations thereof and the like can be mentioned.

When the thus-obtained MHC and the like are in a free form, the free form can be converted into a suitable salt form by a known method or an analogue thereto, and on the other hand, when the MHC and the like are obtained in the form of a salt, it can be converted into the free form or in the form of a different salt by a known method or an analogue thereto.

Furthermore, MHC and the like can also be produced by culturing a transformant comprising a nucleic acid encoding the same, and separating and purifying MHC and the like from the obtained culture. The nucleic acid that encodes MHC and the like may be DNA or RNA, or DNA/RNA chimera, and preferably DNA. In addition, the nucleic acid may be double-stranded or single-stranded. In the case of double strands, double-stranded DNA, double-stranded RNA or DNA:RNA hybrid may be used. In the case of a single strand, it may be a sense strand (that is, coding strand), or an antisense strand (that is, non-coding strand).

Examples of the DNA encoding MHC and the like include genome DNA, cDNA derived from cells of warm-blooded animal (e.g., human, bovine, monkey, horse, swine, sheep, goat, dog, cat, guinea pig, rat, mouse, rabbit, hamster, bird and the like), synthetic DNA and the like. cDNA encoding MHC and the like can be directly amplified by Polymerase Chain Reaction (hereinafter to be abbreviated as "PCR method") by using, as a template, a total RNA or mRNA fraction prepared from any cell of the animals [e.g., hepatocyte, splenocyte, nerve cell, glial cell, pancreatic β cell, myelocyte, mesangial cell, Langerhans' cell, epidermal cell, epithelial cell, goblet cell, endothelial cell, smooth muscle cell, fibroblast, fibrocyte, myocyte, adipocyte, immunocyte (e.g., macrophage, T cell, B cell, natural killer cell, mast cell, neutrophil, basophil, eosinophil, monocyte), megakaryocyte, synovial cell, chondrocyte, bone cell, osteoblast, osteoclast, mammary gland cell, hepatocyte, interstitial cell, or a corresponding progenitor cell, stem cell or cancer cell thereof etc.] or any tissue in which these cells are present [e.g., brain or any portion of brain (e.g., olfactory bulb, amygdaloid nucleus, basal ganglia, hippocampus, thalamus, hypothalamus, cerebral cortex, medulla oblongata, cerebellum), spinal cord, hypophysis, stomach, pancreas, kidney, liver, gonad, thyroid, gallbladder, bone marrow, adrenal gland, skin, lung, gastrointestinal tract (e.g., large intestine and small intestine), blood vessel, heart, thymus, spleen, submandibular gland, peripheral blood, prostate, testis, ovary, placenta, uterus, bone, joint, adipose tissue (e.g., brown adipose tissue, white adipose tissue), skeletal muscle, etc.], and by Polymerase Chain Reaction (hereinafter abbreviated as "PCR method") and Reverse Transcriptase-PCR (hereinafter abbreviated as "RT-PCR method"). Alternatively, cDNA encoding MHC and the like can also be cloned by colony or plaque hybridization method or PCR method and the like from a cDNA library prepared by inserting the above-mentioned total RNA or a fragment of mRNA into a suitable vector. The vector used for the library may be any of a bacteriophage, a plasmid, a cosmid, a phagemid and the like.

A DNA encoding MHC and the like can be cloned by amplifying a synthesized DNA primer having a part of a base sequence encoding the MHC and the like by PCR method, or hybridizing a DNA incorporated into a suitable expression vector with a labeled DNA fragment or synthetic DNA encoding a part or whole region of MHC and the like. The hybridization can be performed by a method known per se or a method analogous thereto, for example, by the method described in Molecular Cloning, 2nd ed. (J. Sambrook et al., Cold Spring Harbor Lab. Press, 1989) and the like. When a commercially available library is used, the hybridization can be performed according to the attached instruction manual. The hybridization can be preferably performed under stringent conditions.

As examples of the highly stringent conditions, conditions of a hybridization reaction in 6×SSC (sodium chloride/sodium citrate) at 45°C followed by washing in 0.2×SSC/0.1% SDS at 65°C once or more and the like can be mentioned. Those skilled in the art are able to easily obtain desired stringency by changing the salt concentration of the hybridization solution, hybridization reaction temperature, probe concentration, probe length, the number of mismatches, hybridization reaction time, the salt concentration of the washing solution, washing temperature and the like as appropriate. When a commercially available library is used, hybridization can be conducted according to the method described in the instruction manual attached to the library.

An expression vector comprising DNA that encodes MHC and the like can be produced by, for example, cutting out a desired DNA fragment from the DNA that encodes MHC and the like, and joining the DNA fragment downstream of a promoter in an appropriate expression vector.

As an expression vector, Escherichia coli-derived plasmids (e.g., pBR322, pBR325, pUC12, pUC13); animal cell expression plasmids (e.g., pA1-11, pXT1, pRc/CMV, pRc/RSV, pcDNAI/Neo); animal virus vectors such as retrovirus, vaccinia virus, adenovirus, lentivirus and the like; and the like are used.

The promoter may be any promoter, as long as it is appropriate for the host used to express the gene.

For example, when the host is an animal cell, SRa promoter, SV40 promoter, LTR promoter, CMV (cytomegalovirus) promoter, RSV (Rous sarcoma virus) promoter, MoMuLV (Moloney murine leukaemia virus) LTR, HSV-TK (herpes simplex virus thymidine kinase) promoter and the like are used. Of these, CMV promoter, SRα promoter and the like are preferred.

When the host is a bacterium of the genus Escherichia, the trp promoter, the lac promoter, the recA promoter, the λP_{L} promoter, the lpp promoter, the T7 promoter and the like are preferred.

Expression vectors used herein include, in addition to the above, expression vectors that optionally comprises an enhancer, a splicing signal, a polyA addition signal, a selection marker, an SV40 replication origin (hereinafter also abbreviated as SV40 ori), and the like. As examples of the selection markers, the dihydrofolate reductase gene (hereinafter also abbreviated as dhfr) [methotrexate (MTX) resistance], the ampicillin resistance gene (hereinafter also abbreviated as amp^{r}), the neomycin resistance gene (hereinafter also abbreviated as neo^{r}, G418 resistance), and the like can be mentioned. In particular, when a dhfr gene defective Chinese hamster cell is used and the dhfr gene is used as the selection marker, a target gene can also be selected using a thymidine-free medium.

Where necessary, a base sequence encoding a signal sequence suitable for a host (signal codon) may be added (or substituted with native signal codon) to the 5'-terminal side of a DNA encoding MHC and the like. For example, when the host is the genus Escherichia, PhoA signal sequence, OmpA signal sequence and the like are used; when the host is an animal cell, insulin signal sequence, α-interferon signal sequence, antibody molecule signal sequence and the like are used.

MHC and the like can be produced by transforming a host with an expression vector containing the above-mentioned DNA encoding MHC and the like, and culturing the obtained transformant.

As the host, for example, the genus Escherichia, animal cell and the like are used.

As the genus Escherichia, for example, K12 DH1 [Proc. Natl. Acad. Sci. USA, vol. 60,160(1968)], JM103 [Nucleic Acids Research, vol. 9, 309(1981)], JA221 [Journal of Molecular Biology, vol. 120, 517(1978)], HB101 [Journal of Molecular Biology, vol. 41, 459(1969)], C600 [Genetics, vol. 39, 440(1954)] and the like are used.

As the animal cell, for example, monkey COS-7 cell, monkey Vero cell, Chinese hamster ovary cell (hereinafter to be abbreviated as CHO cell), dhfr gene defective CHO cell (hereinafter to be abbreviated as CHO(dhfr⁻) cell), mouse L cell, mouse AtT-20 cell, mouse myeloma cell, rat GH3 cell, human FL cell, and the like may be used.

Transformation can be carried out according to the kind of host in accordance with a known method.

The genus Escherichia can be transformed, for example, in accordance with the methods described in Proc. Natl. Acad. Sci. USA, vol. 69, 2110(1972), Genee, vol. 17, 107(1982) and the like.

Animal cells can be transformed according to, for example, the method described in Saibo Kogaku, extra issue 8, Shin Saibo Kogaku Jikken Protocol, 263-267 (1995), published by Shujunsha, or Virology, 52, 456 (1973).

Culture of a transformant can be carried out according to the kind of host in accordance with a known method.

As an example of the medium used for culturing a transformant whose host is a bacterium of the genus Escherichia, a M9 medium supplemented with glucose and a cas amino acid [Miller, Journal of Experiments in Molecular Genetics, 431-433, Cold Spring Harbor Laboratory, New York 1972] is preferable. As required, in order to increase promoter efficiency, a chemical agent such as 3β-indolylacrylic acid may be added to the medium.

Cultivation of a transformant whose host is a bacterium of the genus Escherichia is normally carried out at about 15°C to about 43°C for about 3 to about 24 hours. Where necessary, the culture may be aerated or agitated.

As a medium used when culturing a transformant whose host is an animal cell, for example, minimum essential medium (MEM) containing about 5 - about 20% fetal bovine serum [Science, vol. 122, 501(1952)], Dulbecco's modified Eagle medium (DMEM) [Virology, vol. 8, 396(1959)], RPMI1640 medium [The Journal of the American Medical Association, vol. 199, 519(1967)], 199 medium [Proceeding of the Society for the Biological Medicine, vol. 73, 1(1950)] or the like is used. The medium's pH is preferably about 6 to about 8. Cultivation is normally carried out at about 30°C to about 40°C for about 15 to about 60 hours. As necessary, the culture may be aerated or agitated.

As described above, MHC and the like can be produced in a cell of the transformant or outside the cell.

MHC and the like can be separated and purified from the culture obtained by cultivating the transformant according to a method known per se.

For example, when MHC or the like is extracted from a cultured bacterium or cytoplasm of cell, a method is used as appropriate wherein bacteria or cells are collected from the culture by a known means, suspended in an appropriate buffer solution, and disrupted by means of sonication, lysozyme and/or freeze-thawing and the like, after which a crude extract of soluble protein is obtained by centrifugation or filtration. The buffer solution may comprise a protein denaturing agent such as urea or guanidine hydrochloride and a surfactant such as Triton X-100^{™}. In addition, when MHC or the like is secreted outside the bacteria (cell), a method of separating a culture supernatant by centrifugation, filtration or the like from a culture, and the like are used.

Isolation and purification of MHC and the like contained in the thus-obtained soluble fraction and culture supernatant can be conducted according to a method know per se. Useful methods include methods based on solubility, such as salting-out and solvent precipitation; methods based mainly on molecular weight differences, such as dialysis, ultrafiltration, gel filtration, and SDS-polyacrylamide gel electrophoresis; methods based on charge differences, such as ion exchange chromatography; methods based on specific affinity, such as affinity chromatography; methods based on hydrophobicity differences, such as reversed-phase high performance liquid chromatography; and methods based on isoelectric point differences, such as isoelectric focusing. These methods can be combined as appropriate.

The MHC/antigen peptide complex obtained as described above may be a multimer. By multimerizing the MHC/antigen peptide complex, a higher agonist effect on TCR can be expected. The method for multimerizing the MHC/antigen peptide complex of the present invention is known. For example, the biotin-modified MHC/antigen peptide complex can be tetramerized via a tetramer of avidin or streptavidin. Alternatively, it can also be multimerized by ligating the MHC/antigen peptide complex to dextran.

When the CD3/TCR complex agonist used in step (I) of the present invention (or the step (3)) is an MHC/superantigen complex, the MHC/superantigen complex is a complex of MHC class II and superantigen. As the superantigen, Staphylococcal enterotoxin, Streptococcal pyrogenic exotoxin, toxic shock syndrome toxin and the like can be mentioned. MHC and superantigen may be produced by a method the same as the method described for the MHC and the like. The MHC/superantigen complex may be a multimer like the MHC/antigen peptide complex. The method for multimerizing the MHC/superantigen complex of the present invention is the same as the method for multimerizing the MHC/antigen peptide complex.

The CD3/TCR complex agonist used in step (I) of the present invention may be present in any form as long as it can contact the CD3/TCR complex on the CD3-positive cellular surface during culture. For example, it may be contained in the medium during culture, or may be immobilized on a culture container, and is preferably immobilized on a culture container.

When the CD3/TCR complex agonist is contained in the medium, the medium is not particularly limited as long as CD3-positive cells can be cultured therein. For example, Glasgow's Minimal Essential medium (GMEM) medium, IMDM medium, medium 199 medium, Eagle's Minimum Essential medium (EMEM) medium, αMEM medium, Dulbecco's modified Eagle's medium (DMEM) medium, Ham's F12 medium, RPMI 1640 medium, Fischer's medium, Neurobasal medium (Life Technologies) and mixed media of these, and the like are included. The medium may contain a serum, or may be serum-free. When a serum is contained, the concentration of the serum (e.g., fetal bovine serum (FBS), human serum and the like) is such that the lower limit is generally not less than 1%, preferably not less than 5%, and the upper limit is generally not more than 20%, preferably not more than 15%. Where necessary, the medium may contain one or more serum substitutes such as Knockout Serum Replacement (KSR) (serum replacement of FBS in culturing ES cells), N2 supplement (Invitrogen), B27 supplement (Invitrogen), albumin, insulin, transferrin, selenium compounds (e.g., sodium selenite), vitamin Cs (e.g., ascorbic acid) apotransferrin, fatty acid, collagen precursor, trace element, 2-mercaptoethanol, 3'-thiolglycerol and the like, and one or more substances such as lipid, amino acid, L-glutamine, L-alanyl-L-glutamine (e.g., Glutamax (registered trade mark)), non-essential amino acid, vitamin, growth factor, low-molecular-weight compound, antibiotic (e.g., penicillin, streptomycin), antioxidant, pyruvic acid, buffering agent, inorganic salts, selenium acid, progesterone, putrescine and the like. When the CD3/TCR complex agonist is contained in the medium, the concentration of the CD3/TCR complex agonist may be such that the lower limit is not less than 0.3 ng/ml, preferably not less than 3 ng/ml, and the upper limit is not more than 10000 ng/ml, preferably not more than 1000 ng/ml. Particularly, when the CD3/TCR complex agonist is an anti-CD3 agonist antibody, the concentration of the anti-CD3 agonist antibody in the medium is, for example, 10 ng/ml - 1000 ng/ml. The culture can be performed, for example, in a CO₂ incubator under a CO₂ concentration atmosphere of about 1 - about 10%, preferably about 2 - about 5% at about 30 - about 40°C, preferably about 37°C. The culture period in a medium containing CD3/TCR complex agonist can be appropriately determined by those skilled in the art by monitoring the number of CD3-positive cells and the like. The number of days is not limited as long as CD3-positive cells can be obtained. The culture period is, for example, at least 6 hr or more, 12 hr or more, 16 hr or more, 24 hr or more, 48 hr or more, 72 hr or more, preferably 16 - 72 hr. In addition, 14 days or more is preferable, and 7 days or more is more preferable.

When Vitamin C is used, the Vitamin C is preferably added (supplied) every four days, every three days, every two days, or every day. Vitamin C is more preferably added every day. In one embodiment, Vitamin C is preferably added to the medium at an amount corresponding to 5 ng/ml to 500 ng/ml (e.g., an amount corresponding to 5 ng/ml, 10 ng/ml, 25 ng/ml, 50 ng/ml, 100 ng/ml, 200 ng/ml, 300 ng/ml, 400 ng/ml, or 500 ng/ml). In another embodiment, Vitamin C is added to the culture medium at an amount corresponding to 5 µg/ml - 500 µg/ml (e.g., an amount corresponding to 5 µg/ml, 10 µg/ml, 25 µg/ml, 50 µg/ml, 100 µg/ml, 200 µg/ml, 300 µg/ml, 400 µg/ml, 500 µg/ml).

The medium used in step (I) of the present invention may further contain cytokine. The cytokine contained in the medium is not particularly limited and at least one selected from IL-7, IL-15, IL-18 and IL-21 can be mentioned. It is preferable to contain all of IL-7, IL-15, IL-18 and IL-21. The concentration of cytokine is such that the lower limit is not less than 0.1 ng/mL, preferably not less than 10 ng/mL, and the upper limit is not more than 1000 ng/mL, preferably not more than 300 ng/mL. When these cytokines are used, the concentration in the medium is 1 - 100 ng/ml for IL-7, 1 - 100 ng/ml for IL-15, 5 - 500 ng/ml for IL-18, and 2 - 200 ng/ml for IL-21.

The medium used in step (I) of the present invention may further contain TL1A and/or IL-12. In this case, the concentration of TL1A in the medium is 5 - 500 ng/ml, and the concentration of IL-12 in the medium is 5 - 500 ng/ml.

The medium used in step (I) of the present invention may contain a TNF family cytokine as cytokine. Examples of the TNF family cytokine include TNF-α, TNF-β, lymphotoxin α, Fas ligand, TRAIL, TWEAK, TL1A, RANK ligand, OX40 ligand, APRIL, AITRL, BAFF, 4-1BBL and CD40 ligand and the like, and TL1A is preferable. When TL1A is used, the concentration thereof in the medium may be 5 ng/ml - 500 ng/ml.

As the apoptosis inhibitor used in step (I) of the present invention, a protease inhibitor, for example, caspase inhibitor, can be mentioned. As the caspase inhibitor, Pan Caspase FMK inhibitor Z-VAD (N-benzyloxycarbonyl-Val-Ala-Asp(O-Me) fluoromethylketone) is preferable, and the concentration thereof in the medium may be 1 - 1000 µM.

When the CD3/TCR complex agonist is immobilized on a culture container, the culture container is not particularly limited as long as CD3-positive cells can be cultured therein, and container, slide, bead or a combination of these can be used. Examples of the culture container include flask, tissue culture flask, dish, petri dish, tissue culture dish, multidish, microplate, microwell plate, multiplate, muliwell plate, micro slide, chamber slide, petri dish, tube, tray, culture bag, roller bottle, micro bead and the like.

The CD3/TCR complex agonist can be immobilized on a culture container by a known means. For example, a CD3/TCR complex agonist can be immobilized on a culture container by dissolving the CD3/TCR complex agonist in a solvent (e.g., PBS and the like), adding same to the culture container, and allowing to stand at 4°C overnight. The concentration of the CD3/TCR complex agonist solution for immobilizing the CD3/TCR complex agonist on the culture container can be appropriately determined by those skilled in the art according to the kind of the CD3/TCR complex agonist. In one embodiment of the present invention, for example, when the CD3/TCR complex agonist is an anti-CD3 agonist antibody or a binding fragment thereof, a 0.3 - 10000 ng/ml solution of the anti-CD3 agonist antibody or a binding fragment thereof can be generally contacted with the culture container, and 3 - 5000 ng/ml is more preferable, 3 - 3000 ng/ml is further preferable, and 3 - 600 ng/ml is particularly preferable. In another embodiment of the present invention, the concentration of an anti-CD3 agonist antibody or a binding fragment thereof in a solution is, for example, 10 ng/ml - 10000 ng/ml, preferably 50 ng/ml - 5000 ng/ml, more preferably 200 ng/ml - 4000 ng/ml. In still another embodiment of the present invention, the concentration of an anti-CD3 agonist antibody or a binding fragment thereof in a solution is, for example, 1 - 50000 ng/ml, preferably 3 - 30000 ng/ml, more preferably 30 - 30000 ng/ml, further preferably 300 - 30000 ng/ml. When the anti-CD3 agonist antibody or a binding fragment thereof is an antibody (OKT3) or a binding fragment thereof produced from an OKT3 clone, the concentration thereof in a solution is, for example, 1 ng/ml - 50000 ng/ml, preferably 10 ng/ml - 10000 ng/ml, more preferably 50 ng/ml - 5000 ng/ml, further preferably 200 ng/ml - 4000 ng/ml. When the anti-CD3 agonist antibody or a binding fragment thereof is an antibody (UCHT1) or a binding fragment thereof produced from a UCHT1 clone, the concentration thereof in a solution is, for example, 1 ng/ml - 50000 ng/ml, preferably 3 ng/ml - 30000 ng/ml, more preferably 30 ng/ml - 30000 ng/ml, further preferably 300 ng/ml - 30000 ng/ml.

In step (I) of the present invention, fibronectin is not particularly limited as long as it is a molecule capable of binding to CD3-positive cells. The variant of fibronectin is not particularly limited as long as it is a molecule capable of binding to VLA-5 and VLA-4 on the surface of CD3-positive cells, and examples thereof include RetroNectin (registered trade mark).

Fibronectin or a variant thereof to be used in step (I) of the present invention may be present in any form as long as they can contact CD3-positive cells during culture, like the CD3/TCR complex agonist. For example, they may be contained in the medium during culture, or may be immobilized on a culture container, and are preferably immobilized on a culture container.

When fibronectin or a variant thereof is contained in a medium, the medium may be the same as that containing a CD3/TCR complex agonist. The presence or absence of serum, additive and the like may be the same as that in the medium containing a CD3/TCR complex agonist. When fibronectin or a variant thereof is contained in a medium, the lower limit of the concentration of fibronectin or a variant thereof may be not less than 10 ng/ml, preferably not less than 100 ng/ml, and the upper limit may be not more than 10000 µg/ml, preferably not more than 1000 µg/ml.

When fibronectin or a variant thereof is immobilized on a culture container, the culture container may be the same as that on which the CD3/TCR complex agonist is immobilized. In addition, immobilization of fibronectin or a variant thereof on the culture container may be the same as that of immobilizing the CD3/TCR complex agonist. The immobilizing of fibronectin or a variant thereof on a culture container may be the same as that of CD3/TCR complex agonist. The concentration of the solution of fibronectin or a variant thereof when immobilizing fibronectin or a variant thereof on a culture container may be appropriately determined by those skilled in the art according to the fibronectin or a variant thereof. For example, when fibronectin or a variant thereof is RetroNectin (registered trade mark), a solution of 0.1 - 10000 µg/mL of RetroNectin (registered trade mark) is preferably contacted with the culture container. In this case, the concentration of the RetroNectin solution is more preferably 0.1 - 1000 µg/mL, further preferably 1 - 300 µg/mL, particularly preferably 1 - 150 µg/mL.

In step (I) of the present invention, the CD30 agonist is not particularly limited as long as it is a molecule capable of transducing a signal from a CD30 into a cell by specifically binding to CD30. Examples of the CD30 agonist include at least one selected from the group consisting of anti-CD30 agonist antibody or a fragment bonded thereto and CD30 ligand or a fragment bonded thereto.

The kind, production method and the like of the anti-CD30 agonist antibody and a binding fragment thereof used in step (I) of the present invention may be the same as those of the anti-CD3 agonist antibody and anti-TCR antibody.

The CD30 ligand or a binding fragment thereof used in step (I) of the present invention is, for example, CD153. The CD30 ligand or a binding fragment thereof may be produced by a method similar to the production method of MHC and/or antigen peptide.

The CD30 agonist used in step (I) of the present invention may be present in any form as long as it can be present in contact with CD30 during culturing. For example, it may be contained in the medium during culture, or may be immobilized on a culture container, and is preferably contained in the medium.

When a CD30 agonist is contained in a medium, the medium may be the same as that containing a CD3/TCR complex agonist. The presence or absence of serum, additive and the like may be the same as that in the medium containing a CD3/TCR complex agonist. When a CD30 agonist is contained in a medium, the concentration of the CD30 agonist in the medium can be appropriately determined by those skilled in the art according to the kind of the CD30 agonist. For example, when the CD30 agonist is an anti-CD30 agonist antibody or a binding fragment thereof, the concentration of the anti-CD30 agonist antibody or a binding fragment thereof in the medium is generally 1 ng/ml - 10000 ng/ml, preferably 1 ng/ml - 1000 ng/ml, more preferably 3 ng/ml - 300 ng/ml, further preferably 30 ng/ml - 300 ng/ml.

When the CD30 agonist is immobilized on a culture container, the culture container may be the same as that on which the CD3/TCR complex agonist is immobilized. In addition, a method of immobilizing the CD30 agonist on the culture container may be the same as that of immobilizing the CD3/TCR complex agonist. The lower limit of the concentration of a CD30 agonist solution when the CD30 agonist is immobilized on a culture container may be not less than 0.1 ng/ml, preferably not less than 1 ng/ml, and the upper limit may be not more than 10000 ng/ml, preferably not more than 1000 ng/ml.

The production method or expansion culture method of the present invention may further include a step of culturing the CD3-positive cells, which were cultured in step (I) of the present invention, in the absence of a CD3/TCR complex agonist, and fibronectin or a variant thereof, and in the presence of a CD30 agonist (hereinafter to be referred to as step (II) of the present invention).

As the medium in step (II) of the present invention, the medium used in the above-mentioned step (I) can be used. The medium may contain a serum, or may be serum-free. When a serum is contained, the concentration of the serum (e.g., fetal bovine serum (FBS), human serum and the like) is such that the lower limit is generally not less than 1%, preferably not less than 5%, and the upper limit is generally not more than 20%, preferably not more than 15%. Where necessary, the medium may contain one or more serum substitutes such as Knockout Serum Replacement (KSR) (serum replacement of FBS in culturing ES cells), N2 supplement (Invitrogen), B27 supplement (Invitrogen), albumin, insulin, transferrin, selenium compounds (e.g., sodium selenite), vitamin Cs (e.g., ascorbic acid) apotransferrin, fatty acid, collagen precursor, trace element, 2-mercaptoethanol, 3'-thiolglycerol and the like, and one or more substances such as lipid, amino acid, L-glutamine, Glutamax (Invitrogen), non-essential amino acid, vitamin, growth factor, low-molecular-weight compound, antibiotic, antioxidant, pyruvic acid, buffering agent, inorganic salts, selenium acid, progesterone, putrescine and the like. The culture can be performed, for example, in a CO₂ incubator under a CO₂ concentration atmosphere of about 1 - about 10%, preferably about 2 - about 5% at about 30 - about 40°C, preferably about 37°C. The culture period can be appropriately determined by those skilled in the art by monitoring the number of CD3-positive cells and the like. The number of days is not limited as long as CD3-positive cells can be obtained. The culture period is, for example, not less than 3 days, not less than 5 days, not less than 7 days, not less than 10 days, not less than 14 days, not less than 21 days, preferably not less than 5 days and not more than 15 days. It is preferably not more than 30 days, more preferably not more than 21 days.

When Vitamin C is used, the Vitamin C is preferably added (supplied) every four days, every three days, every two days, or every day. The Vitamin C is more preferably added every day. In one embodiment, Vitamin C is added to the medium at an amount corresponding to 5 ng/ml to 500 ng/ml (e.g., an amount corresponding to 5 ng/ml, 10 ng/ml, 25 ng/ml, 50 ng/ml, 100 ng/ml, 200 ng/ml, 300 ng/ml, 400 ng/ml, or 500 ng/ml). In another embodiment, Vitamin C is added to the culture medium at an amount corresponding to 5 µg/ml - 500 µg/ml (e.g., an amount corresponding to 5 µg/ml, 10 µg/ml, 25 µg/ml, 50 µg/ml, 100 µg/ml, 200 µg/ml, 300 µg/ml, 400 µg/ml, 500 µg/ml).

The medium used in step (II) of the present invention may further contain cytokine. The cytokine is not particularly limited and at least one selected from IL-7, IL-15, IL-18 and IL-21 can be mentioned. It is preferable to contain all of IL-7, IL-15, IL-18 and IL-21. The concentration of cytokine may be such that the lower limit is not less than 0.1 ng/mL, preferably not less than 10 ng/mL, and the upper limit is not more than 1000 ng/mL, preferably not more than 300 ng/mL. When these cytokines are used, the concentration in the medium may be 1 ng/ml - 100 ng/ml for IL-7, 1 ng/ml - 100 ng/ml for IL-15, 5 ng/ml - 500 ng/ml for IL-18, and 2 ng/ml - 200 ng/ml for IL-21.

The medium used in step (II) of the present invention may further contain TL1A and/or IL-12. In this case, the concentration of TLLA in the medium may be 5 ng/ml - 500 ng/ml, and the concentration of IL-12 in the medium may be 5 ng/ml - 500 ng/ml.

The medium used in step (II) of the present invention may further contain an apoptosis inhibitor. As the apoptosis inhibitor, a protease inhibitor can be mentioned, for example, caspase inhibitor. As the caspase inhibitor, Pan Caspase FMK inhibitor Z-VAD (N-benzyloxycarbonyl-Val-Ala-Asp(O-Me) fluoromethylketone) (hereinafter sometimes referred to as "Z-VAD-FMK") is preferable, and the concentration thereof in the medium may be 1 µM - 1000 µM, preferably 1 µM - 500 µM, more preferably 1 µM - 200 µM, particularly preferably 1 µM - 50 µM.

In one embodiment of the present invention, the above-mentioned step (I) and step (II) may be repeated in this order.

The present invention also provides CD3-positive cells obtained by the production method or expansion culture method of the present invention (hereinafter to be referred to as the CD3-positive cell of the present invention). The CD3-positive cell of the present invention is the same as the CD3-positive cell obtained by the production method or expansion culture method of the present invention.

The present invention also provides a kit for expansion culture of CD3-positive cells containing the following (hereinafter to be referred to as the kit of the present invention):
(1) a culture container having a CD3/TCR complex agonist, and fibronectin or a variant thereof
   immobilized thereon, and
(2) a medium containing a CD30 agonist.

The CD3/TCR complex agonist, fibronectin or a variant thereof, CD30 agonist, culture container and medium contained in the kit of the present invention may be the same as those described for the production method or expansion culture method of the present invention.

The present invention also provides a method for maintaining CD3-positive cells as CD3-positive CD197-positive cells, including a step of stimulating a CD30 signal in the CD3-positive cells (hereinafter to be referred to as the maintaining method of the present invention).

Generally, when peripheral blood-derived naive T cells are stimulated with an antigen presented by dendritic cell, naive T cells are activated by intracellular signals, repeat proliferation and maturation, and sequentially differentiate into stem cell memory T cells, central memory T cells, effector memory T cells, and effector T cells. Effector T cells produce cytokine in the case of CD3-positive CD4-positive CD8-negative cells (helper T cells), and kill target cells via antigen in the case of CD3-positive CD4-negative CD8-positive cell (cytotoxic T cell), but cannot survive for a long time. However, by stimulating the CD30 signal in T cells, the T cells can be maintained as self-proliferating naive T cells or stem cell memory T cells (CD197-positive, CD45RA-positive) or central memory T cells (CD197-positive, CD45RA-negative) that can differentiate into effector T cells.

The CD3-positive cells cultured by the maintaining method of the present invention may be the same as the CD3-positive cells cultured by, the production method or expansion culture method of the present invention. Preferably, the CD3-positive cell is a CD3-positive CD197-positive cell.

The maintaining method of the present invention includes a step of stimulating the CD30 signal in the CD3-positive cells. The method of stimulating the CD30 signal in the CD3-positive cells is not particularly limited as long as the signal can be transmitted to the downstream via an intracellular domain of CD30. Examples of the method include a method including a step of culturing CD3-positive cells in the presence of a CD30 agonist (hereinafter to be referred to as the maintaining method (1) of the present invention). The CD3-positive cell can be maintained as CD3-positive CD197-positive cells by the maintaining method (1) of the present invention.

In one embodiment of the maintaining method (1) of the present invention, CD3-positive cells can be maintained as CD3-positive CD197-positive CD45RA-negative cells.

The CD30 agonist and culture conditions used in the maintaining method (1) of the present invention may be the same as those used in the production method or expansion culture method of the present invention.

The present invention also provides a kit containing a CD30 agonist for maintaining CD3-positive cells as CD3-positive CD197-positive cells (hereinafter to be referred to as the maintaining kit of the present invention).

The CD30 agonist contained in the maintaining kit of the present invention may be the same as the CD30 agonist used in the maintaining method (1) of the present invention.

In the maintaining method of the present invention, another method for stimulating a CD30 signal in the CD3-positive cell is, for example, a method including a step of culturing CD3-positive cells that express a chimeric antigen receptor containing the intracellular domain of CD30, in the presence of an antigen that stimulates the chimeric antigen receptor (hereinafter to be referred to as the maintaining method (2) of the present invention). The signal can be transmitted to the downstream via an intracellular domain of CD30 by stimulating a chimeric antigen receptor containing the intracellular domain of CD30, and CD3-positive cells that express a chimeric antigen receptor containing the intracellular domain of CD30 can be maintained as CD3-positive CD197-positive cells.

In one embodiment of the maintaining method (2) of the present invention, CD3-positive cells that express a chimeric antigen receptor containing the intracellular domain of CD30 can be maintained as CD3-positive CD197-positive CD45RA-negative cells.

The culture conditions used in the maintaining method (2) of the present invention may be the same as those used in the production method or expansion culture method of the present invention.

The antigen that stimulates the chimeric antigen receptor used in the maintaining method (2) of the present invention is not particularly limited as long as a signal can be transmitted to the downstream via an intracellular domain of CD30 contained in the chimeric antigen receptor. The antigen that stimulates the chimeric antigen receptor may be the same as the antigen targeted by the chimeric antigen receptor of the chimeric antigen receptor-expressing CD3-positive cells cultured by the production method or expansion culture method of the present invention.

The present invention also provides a chimeric antigen receptor containing an antigen binding domain, a transmembrane domain and an intracellular signal transduction domain, wherein the intracellular signal transduction domain contains an intracellular domain of CD30 or a mutant thereof (hereinafter to be referred to as the chimeric antigen receptor of the present invention).

The antigen binding domain contained in the chimeric antigen receptor of the present invention may be the same as the antigen binding domain contained in the chimeric antigen receptor expressed on the CD3-positive cells cultured in the production method or expansion culture method of the present invention.

The transmembrane domain contained in the chimeric antigen receptor of the present invention may be the same as the transmembrane domain contained in a chimeric antigen receptor expressed on the CD3-positive cells cultured in the production method or expansion culture method of the present invention.

The intracellular signal transduction domain contained in the chimeric antigen receptor of the present invention contains an intracellular domain of CD30 or a mutant thereof. The intracellular domain of CD30 is not particularly limited as long as the antigen binding domain contained in the chimeric antigen receptor of the present invention recognizes an antigen and retains the function of transmitting the recognition signal thereof into cells. For example, a peptide containing the amino acid sequence shown in SEQ ID NO: 6 can be mentioned.

While the mutant of the intracellular domain of CD30 is not particularly limited as long as it can retain the above-mentioned function, for example, a peptide containing an amino acid sequence having a homology of not less than about 90%, preferably not less than about 95%, more preferably not less than about 97%, particularly preferably not less than about 98%, most preferably not less than about 99%, with the amino acid sequence shown in SEQ ID NO: 6 can be mentioned. As used herein, the "homology" may be the same as that in the amino acid sequence of "IL-15/IL-15Rα".

In addition, the mutant of the intracellular domain of CD30 also includes, for example, a protein containing (1) an amino acid sequence wherein one or more (preferably, 1 - about 100, preferably 1 - about 50, further preferably 1 - about 10, particularly preferably 1 - several (2, 3, 4 or 5)) amino acids in the amino acid sequence shown in SEQ ID NO: 6 are deleted, (2) an amino acid sequence wherein one or more (preferably, 1 - about 100, preferably 1 - about 50, further preferably 1 - about 10, particularly preferably 1 - several (2, 3, 4 or 5)) amino acids are added to the amino acid sequence shown in SEQ ID NO: 6, (3) an amino acid sequence wherein one or more (preferably, 1 - about 50, preferably 1 - about 10, further preferably 1 - several (2, 3, 4 or 5)) amino acids are inserted into the amino acid sequence shown in SEQ ID NO: 6, (4) an amino acid sequence wherein one or more (preferably, 1 - about 50, preferably 1 - about 10, further preferably 1 - several (2, 3, 4 or 5)) amino acids are substituted by other amino acids in the amino acid sequence shown in SEQ ID NO: 6 or (5) an amino acid sequence which is a combination thereof and the like. When the amino acid sequence is inserted, deleted or substituted as mentioned above, the position of insertion or deletion or substitution is not particularly limited as long as the function of the intracellular domain of CD30 is maintained.

The intracellular signal transduction domain contained in the chimeric antigen receptor of the present invention may contain an intracellular domain of CD30 or a mutant thereof, and further an intracellular domain derived from other protein. The intracellular domain to be further contained may be the same as the intracellular domain contained in an intracellular signal transduction domain contained in the chimeric antigen receptor expressed on CD3-positive cells cultured in the production method or expansion culture method of the present invention. The intracellular domain to be further contained is not particularly limited, and an intracellular domain derived from CD28 or CD3ζ chain is preferable.

The chimeric antigen receptor of the present invention may have a spacer incorporated in between an antigen binding domain and a transmembrane domain, or between an intracellular signal transduction domain and a transmembrane domain. The spacer may be the same as the spacer contained in the chimeric antigen receptor expressed on CD3-positive cells cultured in the production method or expansion culture method of the present invention.

Specific examples of the chimeric antigen receptor of the present invention include a chimeric antigen receptor containing scFv recognizing CD19 as an antigen binding domain, transmembrane domain of CD8 as a transmembrane domain, intracellular domain derived from CD28 as an intracellular signal transduction domain, an intracellular domain derived from CD30, and an intracellular domain derived from CD3ζ chain. The order of the above-mentioned intracellular domains contained in the intracellular signal transduction domain is not particularly limited and, for example, the order of the intracellular domain derived from CD28, the intracellular domain derived from CD30, and the intracellular domain derived from CD3ζ chain is adopted. More specifically, the chimeric antigen receptor in the present invention is composed of, for example, the amino acid sequence shown in SEQ ID NO: 7.

The chimeric antigen receptor of the present invention may be a chemically-synthesized protein or a protein biochemically synthesized in a cell-free translation system, or may be a recombinant protein produced from a transformant incorporating a nucleic acid having a base sequence that encodes the chimeric antigen receptor of the present invention. The chimeric antigen receptor of the present invention can be similarly produced and isolated and purified according to the production method of MHC and/or antigen peptide used in the production method or expansion culture method of the present invention.

The present invention also provides a nucleic acid containing a polynucleotide encoding the chimeric antigen receptor of the present invention (hereinafter to be referred to as the nucleic acid of the present invention).

The nucleic acid of the present invention may be a DNA or an RNA, or DNA/RNA chimera. Preferred is a DNA. In addition, the nucleic acid may be double-stranded or single-stranded. In the case of double strands, double-stranded DNA, double-stranded RNA or DNA:RNA hybrid may be used. In the case of single strand, it may be a sense strand (i.e., coding strand), or an antisense strand (i.e., non-coding strand).

The nucleic acid of the present invention can be obtained by the Polymerase Chain Reaction (hereinafter abbreviated as "PCR method"). First, a genome DNA or cDNA encoding each domain of an antigen binding domain, a transmembrane domain and an intracellular signal transduction domain contained in the chimeric antigen receptor of the present invention. cDNA can also be directly amplified by PCR method and Reverse Transcriptase-PCR (hereinafter to be abbreviated as "RT-PCR method") by using, as a template, a total RNA or mRNA fraction prepared from cells. Using the obtained genome DNA or cDNA as a template, a nucleic acid encoding each domain of an antigen binding domain, a transmembrane domain and an intracellular signal transduction domain can be amplified by the PCR method using a synthetic DNA primer consisting of a part of the base sequence encoding each domain and a base sequence encoding a spacer. The nucleic acid of the present invention can be obtained by repeating the PCR method using the obtained respective domains as templates, and the synthetic DNA primers.

The present invention also provides a chimeric antigen receptor gene transfer vector containing the nucleic acid of the present invention (hereinafter to be referred to as the transgene vector of the present invention).

The transgene vector of the present invention can be produced, for example, by linking the nucleic acid of the present invention to the downstream of a promoter in an appropriate gene transfer vector. The gene transfer vector, promoter, and other elements may be the same as vector, promoter, and other elements used when introducing exogenous TCR into pluripotent stem cells in the production method or expansion culture method of the present invention.

The present invention also provides a chimeric antigen receptor-expressing cell containing the transgene vector of the present invention (hereinafter to be referred to as the chimeric antigen receptor-expressing cell of the present invention).

The chimeric antigen receptor-expressing cell of the present invention can be produced by introducing the transgene vector of the present invention into cells and culturing the cells. As the cell into which the transgene vector of the present invention is introduced, a CD3-positive cell or a progenitor cell thereof (hematopoietic stem cell, common lymphoid progenitor cell, lymphoblast and the like), or a pluripotent stem cell can be mentioned. As the pluripotent stem cell, ES cell, iPS cell, EC cell, and EG cell can be mentioned, and ES cell or iPS cell is preferred.

The transgene vector of the present invention may be introduced into cells by methods such as lipofection, liposome, microinjection and the like.

The chimeric antigen receptor-expressing cell of the present invention is preferably a CD3-positive cell. The CD3-positive cell is preferably a CD3-positive CD8-positive cell (CD3-positive CD8-positive CD4-positive cell or CD3-positive CD8-positive CD4-negative cell), more preferably a CD3-positive CD8-positive CD4-negative cell. Another preferable CD3-positive cell mentioned above is, for example, a CD3-positive CD4-positive cell (CD3-positive CD4-positive CD8-positive cell or CD3-positive CD4-positive CD8-negative cell).

When the chimeric antigen receptor-expressing cell of the present invention is a cell produced by introducing the transgene vector of the present invention into pluripotent stem cells, the pluripotent stem cell can be differentiated into a CD3-positive cell according to a method known per se. A specific method for differentiating a pluripotent stem cell into a CD3-positive cell may be the same as the method for differentiating the CD3-positive cell to be cultured into a pluripotent stem cell in the production method or expansion culture method of the present invention.

The thus-obtained chimeric antigen receptor-expressing cell of the present invention is imparted with the specificity for the antigen of interest by the chimeric antigen receptor, and can show cytotoxic activity against tumors expressing the antigen of interest. The chimeric antigen receptor can directly recognize an antigen molecule without relying on HLA class I or class II. Thus, the chimeric antigen receptor-expressing cell of the present invention can cause a high immune response even for tumors with reduced expression of HLA class I or class II gene.

Therefore, the present invention also provides a medicament containing the chimeric antigen receptor-expressing cell of the present invention as an active ingredient (hereinafter sometimes to be referred to as the medicament of the present invention). A medicament containing the chimeric antigen receptor-expressing cell of the present invention can be used for the prophylaxis or treatment of tumors expressing an antigen recognized by the chimeric antigen receptor of the present invention, and can be administered, for example, to mammals (e.g., mouse, rat, hamster, rabbit, cat, dog, bovine, sheep, monkey, human), preferably human. Therefore, in one embodiment of the present invention, the medicament of the present invention for use for the prophylaxis or treatment of tumors expressing an antigen recognized by the chimeric antigen receptor of the present invention is provided. In addition, a method for preventing or treating tumors expressing an antigen recognized by the chimeric antigen receptor of the present invention, including administering the chimeric antigen receptor-expressing cell of the present invention preferably in the form of a medicament containing the cell is provided.

The tumor that can be prevented or treated by the chimeric antigen receptor expression cell of the present invention is not particularly limited as long as it expresses an antigen recognized by the chimeric antigen receptor of the present invention. Tumor is described in, for example, "Daniel Baumhoer et al., Am J. Clin Pathol, 2008, 129, 899-906" and the like. Tumor includes benign tumor, malignant tumor (also referred to as "cancer"), and tumor that may be diagnosed or determined to be benign or malignant. Specific examples of tumor include, but are not limited to, liver cancer (e.g., hepatoma), ovarian cancer (e.g., ovary clear cell adenocarcinoma), childhood cancer, lung cancer (e.g., squamous cell carcinoma, small cell lung cancer), testis cancer (e.g., nonseminomas germ cell tumor), soft tissue tumor (e.g., liposarcoma, malignant fibrous histiocytoma), uterine cancer (e.g., cervix intraepithelial tumor, cervix squamous cell carcinoma), melanoma, adrenal gland tumor (e.g., adrenal gland adenoma), neurotic tumor (e.g., schwannoma), gastric cancer (e.g., adenocarcinoma of stomach), renal cancer (e.g., Grawitz tumor), breast cancer (e.g., invasive lobular carcinoma, mucous cancer), thyroid cancer (e.g., medullar cancer), laryngeal cancer (e.g., squamous cell carcinoma), urinary bladder cancer (e.g., invasive transitional cell carcinoma) and the like.

The chimeric antigen receptor-expressing cell of the present invention may be cultured and/or stimulated using an appropriate medium and/or a stimulating molecule before administration to a test subject. Examples of the stimulating molecule include, but are not limited to, cytokines, suitable protein, other components and the like. Examples of the cytokines include IL-2, IL-7, IL-12, IL-15, IFN-γ and the like, and IL-2 can be preferably used. While the concentration of IL-2 in the medium is not particularly limited, for example, it is preferably 0.01 U/mL - 1×10⁵ U/mL, more preferably 1 U/mL - 1×10⁴ U/mL. Examples of the suitable protein include an antigen recognized by chimeric antigen receptor, CD3 ligand, CD28 ligand, anti-CD3 antibody, anti-CD30 antibody, and anti-IL-4 antibody. Besides these, a lymphocyte stimulating factor such as lectin and the like can also be added. Furthermore, serum or plasma may be added to the medium. While the amount of addition to these media is not particularly limited, 0% by volume - 20% by volume can be mentioned. In addition, the amount of serum or plasma to be used can be changed according to the culturing stage. For example, serum or plasma concentration can be reduced stepwise. The origin of serum or plasma may be either autologous or allogeneic, and autologous one is preferable from the aspect of safety.

The medicament of the present invention is preferably used by parenteral administration to the test subject. Examples of the method for parenteral administration include intravenous, intraarterial, intramuscular, intraperitoneal, and subcutaneous administration and the like. While the dose is appropriately selected according to the condition, body weight, age and the like of the test subject, the medicament is generally administered such that the cell number is generally 1×10⁶ - 1×10¹⁰ cells, preferably 1×10⁷ - 1×10⁹ cells, more preferably 5×10⁷ - 5×10⁸ cells, per dose to a test subject with body weight 60 kg. The T cells may be administered once or in plural portions. The medicament of the present invention can be formulated into a known form suitable for parenteral administration, for example, injection or injecting agent. The medicament of the present invention may contain saline, phosphate buffered saline (PBS), medium and the like to maintain the cells stably. The medium is not particularly limited, and examples thereof include, but are not limited to, media such as RPMI, AIM-V, X-VIVO10 and the like. The medicament may contain a pharmaceutically acceptable carrier (e.g., human serum albumin), preservative and the like for stabilizing purposes.

Furthermore, since the chimeric antigen receptor-expressing cell of the present invention can kill cells that express an antigen recognized by the chimeric antigen receptor of the present invention, it can be used as a killing agent for cells expressing the antigen. Such killing agent can be produced and used in the same manner as the medicament.

The present invention also includes embodiments of the use of the chimeric antigen receptor-expressing cell of the present invention in the production of prophylactic or therapeutic agents for tumor that expresses an antigen recognized by the chimeric antigen receptor of the present invention, according to the medicament containing the chimeric antigen receptor-expressing cell of the present invention. Prophylactic or therapeutic agents for tumor can be produced by a method known per se. For example, they can be produced in a known form suitable for parenteral administration, such as injection, injectable agent, or the like, as in the above-mentioned preparation method of the medicament of the present invention.

While the present invention is explained in more detail in the following by referring to Examples, they are mere exemplifications and do not limit the present invention.

### [Example]

### [Example 1]

### 1. Preparation of iPS cell

As the iPS cell, Ff-I01s04 strain provided by the Center for iPS Cell Research and Application (CiRA), Kyoto University, was used. iPS cell culture was performed according to the protocol distributed by CiRA, "Human iPS cell culture under feeder-free conditions".

### 2. Introduction of T cell receptor (TCR) gene into iPS cell

As TCR gene, TAK1-derived HLA-A*24:02-restricted WT1-specific TCR (WT1-TCR) gene supplied by Professor Masataka Yasukawa, Graduate School of Medicine, Ehime University, was used. Gene transfer into iPS cells was performed by incorporation into CS-UbC-RfA-IRES2-hKO1 lentivirus vector supplied by Inst. of Physical and Chemical Research and infecting the iPS cells.

### 3. Differentiation of iPS cells with WT1-TCR gene introduced thereinto into CD8-positive T cells (CTL)

Differentiation of iPS cells with WT1-TCR gene introduced thereinto into CD8-positive T cells (CTL) was performed according to a known method (WO 2017/221975). The present cells were CD3-positive, CD8-positive. In the following, the cells were used as iPS cell-derived T cells.

### 4. Reagent, antibody

RetroNectin (registered trade mark) was purchased from Takara Bio Inc. As the anti-CD3 agonist antibody, anti-human CD3 functional grade purified (Clone: OKT3) purchased from eBioscience or CD3 antibody (Clone: UCHT1) purchased from GeneTex was used. Unless particularly indicated, OKT3 was used. As the anti-CD30 agonist antibody, CD30 agonist antibody purchased from R&D systems was used.

### 5. Immobilization of anti-CD3 agonist antibody and RetroNectin (registered trade mark) on culture plate

The anti-CD3 agonist antibody and RetroNectin (registered trade mark) dissolved in PBS to necessary concentrations were added to a 96 well plate at 50 µL/well, and the plate was allowed to stand at 4°C overnight. The cells were washed with PBS and subjected to the test.

### 6. ELISA for detection of immobilized anti-CD3 agonist antibody and immobilized RetroNectin (registered trade mark)

For the detection of immobilized anti-CD3 agonist antibody, HRP Conjugated Goat anti-Mouse IgG2a Detection Antibody contained in the Mouse IgG2a ELISA Quantitation Set purchased from Bethyl Laboratories was used. For the detection of immobilized RetroNectin (registered trade mark), peroxidase-labeled anti-RetroNectin antibody contained in the RetroNectin EIA kit purchased from Takara Bio Inc. was used. Each detection antibody was added to a immobilized plate, the plate was allowed to stand for 1 hr at room temperature. After washing with PBS containing 0.05% Tween-20, 1-Step^{™} Ultra TMB-ELISA Substrate Solution (ThermoFisher) was added. After standing for 15 min at room temperature, 1M sulfuric acid was added to discontinue the reaction, and the absorbance at 450 nm was measured using a plate reader.

### 7. Proliferation test

iPS cell-derived T cells prepared to 100,000 cells/200 µL in a medium prepared by adding cytokine and the like of Table 1 to α-MEM medium containing 15% FBS were seeded in a plate with an anti-CD3 agonist antibody (3, 30, 300, 3000 ng/ml) and RetroNectin (registered trade mark) (0, 1.85, 2.25, 16.7, 50, 150 µg/ml) immobilized thereon, and cultured for 3 days under 5% CO₂/37°C.

On day 3 of culture, the cells were harvested from the plate, and the number of the cells was measured using TC20 (Bio-Rad). The cells were suspended in an appropriate amount of a medium obtained by adding cytokine and the like in Table 2 to α-MEM medium containing 15% FBS, added to a non-immobilized 96 well plate and cultured at 5% CO₂/37°C. Thereafter, the cells were collected from the plate on any of days 5, 6, 7, 8, 9, 10, 12, 14 and 16 once each timing and 4 to 7 times in total, and the number of cells was measured. The cells were suspended in an appropriate amount, added to a non-immobilized plate and cultured at 5% CO₂/37°C.

**[Table 1]**

| product name | manufacturer | Final cone |
|---|---|---|
| ITS (100x) (Insulin-Transferrin-Selenium Supplements) | Invitrogen | 1 x |
| Ascorbic acid 2-phosphate | sigma | 50 µg/ml |
| IL-7 | Peprotech | 10 ng/ml |
| IL-15 | Peprotech | 10 ng/ml |
| IL-2 | Peprotech | 10 ng/ml |
| IL-21 | Peprotech | 20 ng/ml |
| IL-12 | Merck | 50 ng/ml |
| IL-18 | MBL | 50 ng/ml |
| Z-VAD-FMK | R&D | 10 µM |

**[Table 2]**

| product name | manufacturer | Final conc |
|---|---|---|
| ITS (100x) (Insulin-Transferrin-Selenium Supplements) | Invitrogen | 1 x |
| Ascorbic acid 2-phosphate | sigma | 50 µg/ml |
| IL-7 | Peprotech | 10 ng/ml |
| IL-15 | Peprotech | 10 ng/ml |

### 8. ATP test

The cells on day 12 of culture in the proliferation test and ATP in the culture supernatant were measured according to the standard protocol and using CellTiter-Glo(R) Luminescent Cell Viability Assay purchased from Promega KK.

### 9. Measurement of WT1 antigen-specific cytotoxic activity

HLA-A*24:02-positive LCL cells were purchased from RIKEN BioResource center. The cells were cultured in a RPMI1640 medium containing 10% FBS. Synthesis of WT1 antigen peptide (CMTWNQMNL: SEQ ID NO: 3) was outsourced to Scrum Inc. The cytotoxicity test was evaluated according to the standard protocol and using DELFIA immunoassay purchased from Perkin Elmer Inc.

### 10. Proliferation test with addition of anti-CD30 agonist antibody

An anti-CD30 agonist antibody diluted to a final concentration of 0, 30, 100, 300 ng/mL was added to the culture medium during suspending of the cells. The rest was the same as in the method of "7. Proliferation test".

In the proliferation test of iPS cell-derived T cells by multiple stimulations with immobilized anti-CD3 agonist antibody/RetroNectin (registered trade mark) and anti-CD30 agonist antibody, an anti-CD30 agonist antibody diluted to a final concentration of 100 ng/mL was added to the culture medium during suspending of the cells. The rest was the same as in the method of "7. Proliferation test".

### 11. Detection of CD197 and CD45RA on cell membrane surface

The cells on day 7 of culture were harvested, stained with the antibody of Table 3, and detected using LSRFortessa^{™} X-20 (BD Bioscience) flow cytometry.

**[Table 3]**

| Target | Fluorescent | clone | Vender |
|---|---|---|---|
| CD5 | APC/Cy7 | UCHT2 | BioLegend |
| CD8a | PerCP/Cy5.5 | SK1 | BioLegend |
| CD8b | PE/Cy7 | SID18BEE | eBioscience |
| CD197 | AF647 | G043H7 | BioLegend |
| CD45RA | FITC | HI100 | BioLegend |

### [Experimental Example 1]

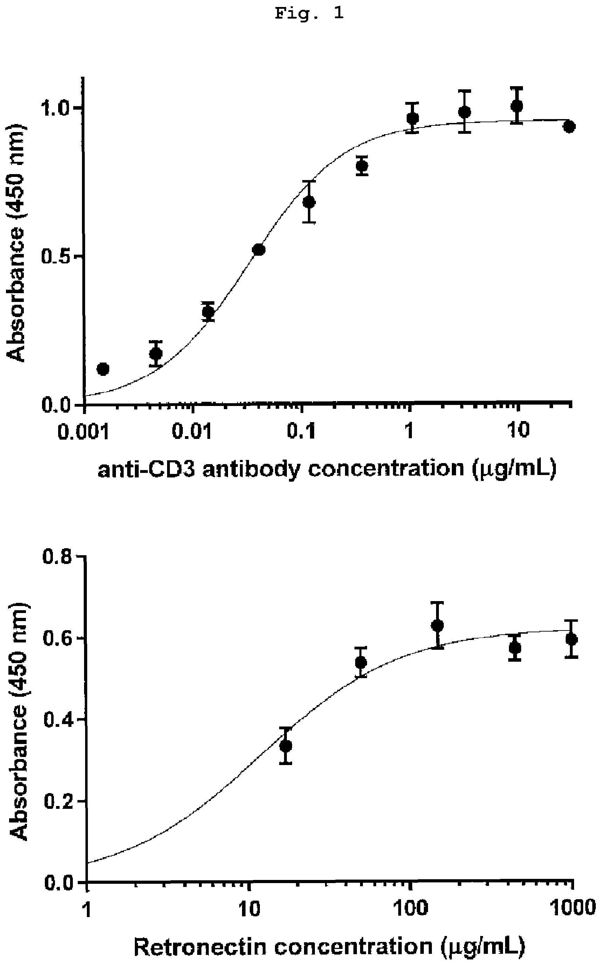

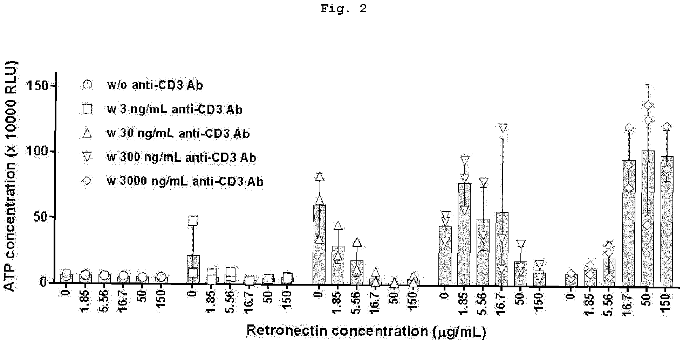

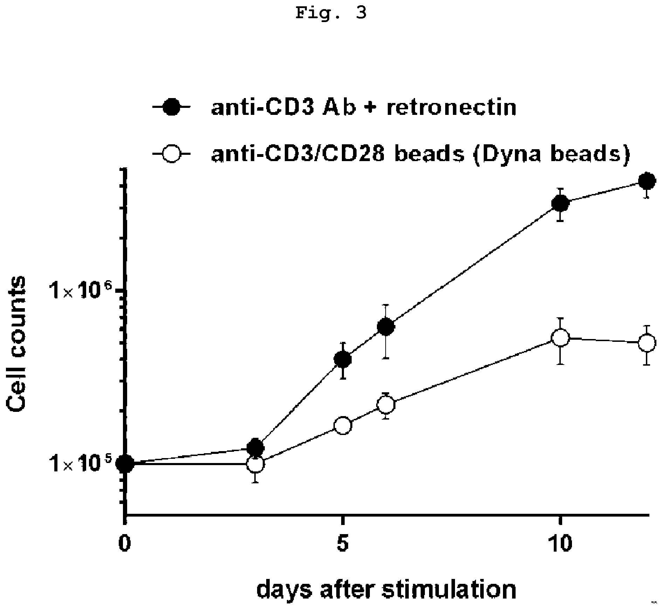

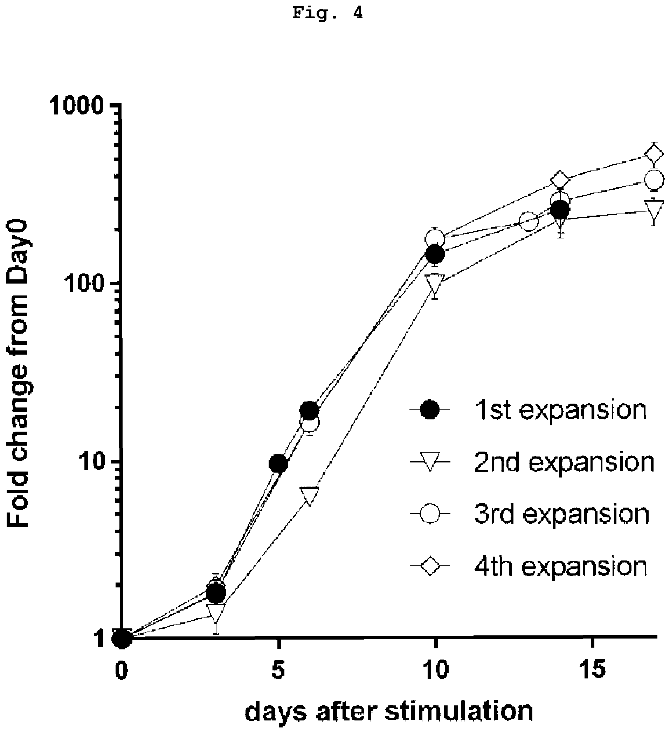

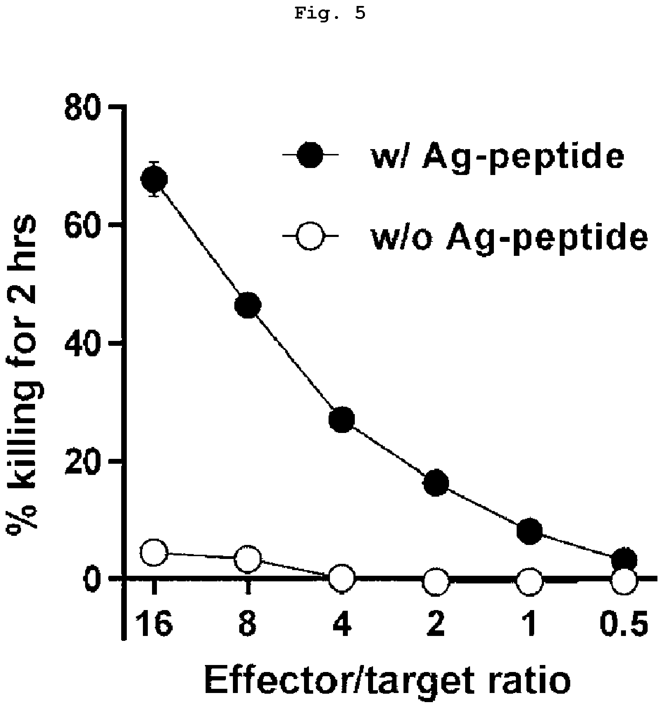

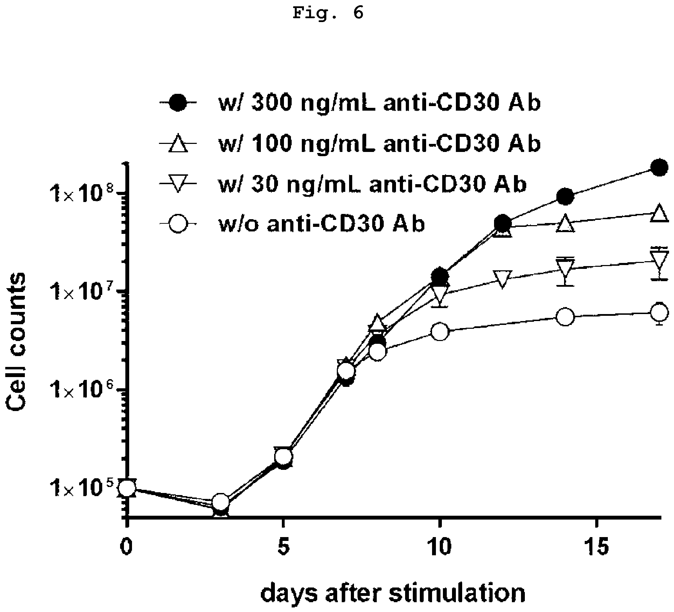

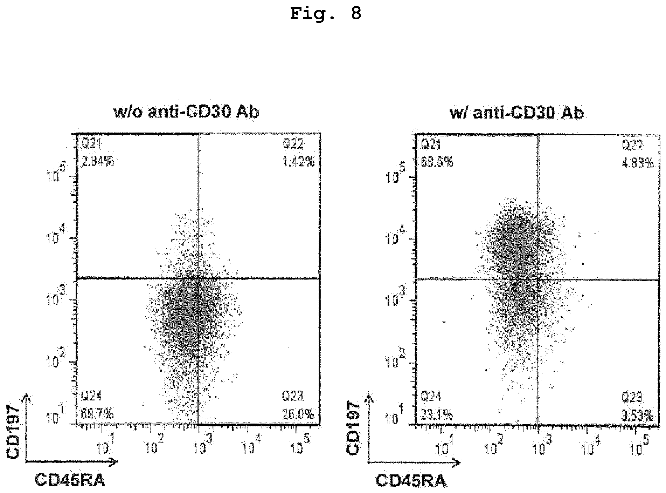

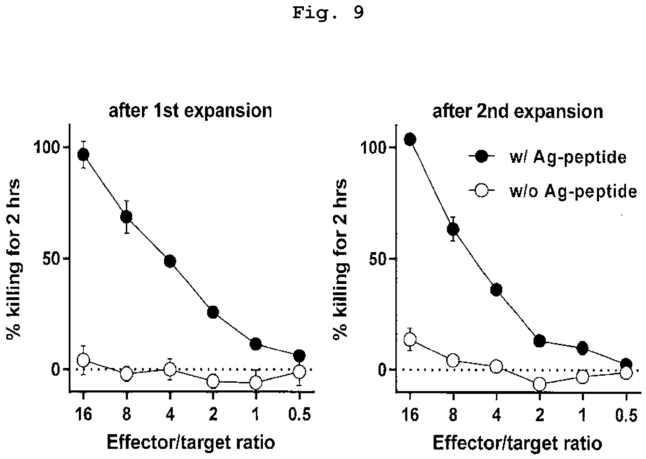

1. Measurement of concentration of anti-CD3 agonist antibody and concentration of RetroNectin (registered trade mark) suitable for immobilizing on culture plate
   The concentration of an anti-CD3 agonist antibody and RetroNectin (registered trade mark) suitable for immobilizing on a culture plate was measured by the ELISA method (Fig. 1). Concentration-dependent immobilizing of an anti-CD3 agonist antibody was confirmed between 3 ng/mL and 3000 ng/mL. Concentration-dependent immobilizing of RetroNectin (registered trade mark) was confirmed between 16.7 µg/mL and 150 µg/mL.
2. Verification of concentration range of anti-CD3 agonist antibody and RetroNectin (registered trade mark) necessary for immobilized anti-CD3 agonist antibody and immobilized RetroNectin (registered trade mark) suitable for proliferation of iPS cell-derived T cells
   iPS cell-derived T cells were stimulated for 3 days on a plate with an anti-CD3 agonist antibody (0, 3, 30, 300, 3000 ng/mL) and RetroNectin (registered trade mark) (0, 1.85, 5.56, 16.7, 50, 150 µg/mL) immobilized thereon, and cultured on a non-immobilized plate for 9 days, and the amount of ATP was measured (Fig. 2).
3. Proliferation test of iPS cell-derived T cells stimulated with immobilized anti-CD3 agonist antibody/RetroNectin (registered trade mark)
   iPS cell-derived T cells prepared at 100,000 cells/200 µL were stimulated for 3 days on a 96 well plate with an anti-CD3 agonist antibody (3000 ng/mL) and RetroNectin (registered trade mark) (150 µg/mL) immobilized thereon, or with anti-CD3/CD28 beads (Dynabeads) in which the ratio of the number of iPS cell-derived T cells and the number of bead particles is 1:1, and the number of cells cultured without stimulation was measured over time (Fig. 3). The iPS cell-derived T cells were proliferated more by stimulating with immobilized anti-CD3 agonist antibody/RetroNectin (registered trade mark) than with anti-CD3/CD28 beads.
4. Proliferation test of iPS cell-derived T cells stimulated multiple times with immobilized anti-CD3 agonist antibody/RetroNectin (registered trade mark)
   The cell proliferation reaction of iPS cell-derived T cells to multiple times of stimulation with immobilized anti-CD3 agonist antibody/RetroNectin (registered trade mark) was confirmed. One proliferation test was performed by stimulating iPS cell-derived T cells for 3 days on an immobilized plate and culturing the cells for 11-15 days on a non-immobilized plate. After the completion of the test, the number of cells was adjusted and subjected to the next test. The iPS cell-derived T cells responded to the fourth stimulation with immobilized anti-CD3 agonist antibody/RetroNectin (registered trade mark) and proliferated (Fig. 4).
5. Verification of WT1 antigen-specific cytotoxic activity of WT1-TCR gene-transferred iPS cell-derived T cells proliferated by stimulation with immobilized anti-CD3 agonist antibody/RetroNectin (registered trade mark)
   The WT1 antigen-specific cytotoxic activity of WT1-TCR gene-transferred iPS cell-derived T cells proliferated by the stimulation with immobilized anti-CD3 agonist antibody/RetroNectin (registered trade mark) was evaluated. The WT1 antigen-specific cytotoxic activity was evaluated based on the difference between the cytotoxicity against LCL added with WT1 antigen peptide and the cytotoxicity against LCL free of the addition. The WT1-TCR gene-transferred iPS cell-derived T cells scarcely had non-specific cytotoxic activity and showed WT1 antigen-specific cytotoxic activity alone (Fig. 5).
6. Proliferation test of iPS cell-derived T cells by stimulation with anti-CD30 agonist antibody
   Whether addition of an anti-CD30 agonist antibody (0, 30, 100, 300 ng/mL) during stimulation with immobilized anti-CD3 agonist antibody/RetroNectin (registered trade mark) influences proliferation of iPS cell-derived T cells was verified. As the immobilized 96 well plate, one on which an anti-CD3 agonist antibody (3000 ng/ml) and RetroNectin (registered trade mark) (150 ug/ml) were immobilized was used. iPS cell-derived T cells were proliferated more by the addition of an anti-CD30 agonist antibody. The cells were proliferated in an anti-CD30 agonist antibody concentration dependent manner (Fig. 6).
7. Proliferation test of iPS cell-derived T cells by multiple times of stimulation with immobilized anti-CD3 agonist antibody/RetroNectin (registered trade mark) and anti-CD30 agonist antibody
   Whether iPS cell-derived T cells show a proliferation reaction by multiple times of stimulation with immobilized anti-CD3 agonist antibody/RetroNectin (registered trade mark) and anti-CD30 agonist antibody was verified. The cells were seeded in a plate containing anti-CD3 antibody (3000 ng/ml) and RetroNectin (registered trade mark) (150 µg/ml) immobilized thereon and cultured at 5% CO₂/37°C for 3 days.
   On day 3 of culture, the cells were harvested from the plate, and the number of the cells was measured using TC20 (Bio-Rad). The cells were suspended in an appropriate amount of a medium obtained by adding cytokine and the like in Table 2 to α-MEM medium containing 15% FBS, added to a non-immobilized plate and cultured at 5% CO₂/37°C. Thereafter, the cells were collected from the plate once each on days 6, 7, 9, 10, 14 and 16, and the number of cells was measured. The cells were suspended in an appropriate amount, added to a non-immobilized plate and cultured at 5% CO₂/37°C. As the culture medium, one added with an anti-CD30 agonist antibody diluted to the final concentration of 100 ng/mL, and one free of an anti-CD30 agonist antibody were used. The above-mentioned stimulation from day 0 to day 16 of culture was repeated twice. Even by the second stimulation, the iPS cell-derived T cells were proliferated by the stimulation with immobilized anti-CD3 agonist antibody/RetroNectin (registered trade mark) and anti-CD30 agonist antibody as compared to the stimulation with immobilized anti-CD3 agonist antibody/RetroNectin (registered trade mark) alone (Fig. 7).
8. Detection of CD197 and CD45RA on membrane surface of iPS cell-derived T cell after stimulation with immobilized anti-CD3 agonist antibody/RetroNectin (registered trade mark) and anti-CD30 agonist antibody
   After stimulation with immobilized anti-CD3 agonist antibody/RetroNectin (registered trade mark), or stimulation with immobilized anti-CD3 agonist antibody/RetroNectin (registered trade mark) and anti-CD30 agonist antibody, the expression of CD197 and CD45RA on the membrane surface of iPS cell-derived T cells on day 7 was measured by flow cytometer. In the cell population stimulated with anti-CD3 agonist antibody/RetroNectin (registered trade mark) and anti-CD30 agonist antibody, CD197-positive CD45RA-negative central memory-like iPS cell-derived T cells were in the majority (Fig. 8) .
9. Verification of WT1 antigen-specific cytotoxic activity of WT1-TCR gene-transferred iPS cell-derived T cells proliferated by stimulation with immobilized anti-CD3 agonist antibody/RetroNectin and anti-CD30 agonist antibody
   WT1-TCR gene-transferred iPS cell-derived T cells proliferated by the stimulation with immobilized anti-CD3 agonist antibody/RetroNectin (registered trade mark) and anti-CD30 agonist antibody were applied to HLA-A*24:02-positive LCL cells in the presence or absence of WT1 antigen peptide, and the WT1 antigen-specific cytotoxic activity was evaluated. The WT1-TCR gene-transferred iPS cell-derived T cells scarcely had non-specific cytotoxic activity and showed WT1 antigen-specific cytotoxic activity alone (Fig. 9).

### [Example 2]

### 1. Preparation of human peripheral blood-derived CD8-positive T cells

Human peripheral blood was healthy donor-derived peripheral blood mononuclear cell purchased from Precision Bioservices. Using CD8+ T cell isolation kit, human (Milteny), CD8-positive T cells were isolated.

### 2. Proliferation test of human peripheral blood-derived CD8-positive T cells

By a method similar to that in [Example 1] 7. Proliferation test, the test was conducted.

### 3. Culture of human peripheral blood-derived CD8-positive T cells used for mouse engraftment test

To human peripheral blood-derived CD8-positive T cells suspended at a final concentration of 100,000 cells/mL in 3 kinds of media obtained by adding additives shown in Table 4 to α-MEM medium containing 15% FBS was added Dynabeads T-Activator CD3/CD28 (gibco) adjusted to a 3-fold amount of the number of cells, and the cells were cultured at 5% CO₂/37°C for 3 days. The cells were harvested from the plate on day 3 of culture and suspended in an appropriate amount of a medium obtained by adding cytokine and the like shown in Table 5 to α-MEM medium containing 15% FBS, and cultured at 5% CO₂/37°C. Thereafter, the cells were collected from the plate on day 10 of culture, and the number of cells was measured. The cells were suspended in an appropriate amount and administered to the mice. On days 6, 10, 13, 17 of culture, the cells were collected from the plate, suspended in an appropriate amount, and subjected to a CD197 expression experiment.

**[Table 4]**

| product name | manufacturer | Final conc | w IL2 | w IL7/ IL15 | w IL7/IL15/ anti-CD30Ab |
|---|---|---|---|---|---|
| Insulin-Transferrin-Selenium Supplements | Invitrogen | 1 x | + | + | + |
| Ascorbic acid 2-phosphate | sigma | 50 µg/ml | + | + | + |
| IL-2 | Peprotech | 15 ng/ml | + | | |
| IL-7 | Peprotech | 10 ng/ml | | + | + |
| IL-15 | Peprotech | 10 ng/ml | | + | + |
| IL-21 | Peprotech | 20 ng/ml | | + | + |
| IL-12 | Merck | 50 ng/ml | | + | + |
| IL-18 | MBL | 50 ng/ml | | + | + |
| TL-1A | Peprotech | 50 ng/ml | | + | + |
| Z-VAD-FMK | R&D | 10 µM | | + | + |
| Human CD30 Antibody | R&D | 300 ng/ml | | | + |

| | | | | | |
|---|---|---|---|---|---|
| w: with (additive added to each group is shown with +.) | | | | | |

**[Table 5]**

| product name | manufacturer | Final conc | w IL2 | w IL7/ IL15 | w IL7/IL15/ anti-CD30Ab |
|---|---|---|---|---|---|
| Insulin-Transferrin-Selenium Supplements | Invitrogen | 1 x | + | + | + |
| Ascorbic acid 2-phosphate | sigma | 50 µg/ml | + | + | + |
| IL-2 | Peprotech | 15 ng/ml | + | | |
| IL-7 | Peprotech | 10 ng/ml | | + | + |
| IL-15 | Peprotech | 10 ng/ml | | + | + |
| Human CD30 Antibody | R&D | 300 ng/ml | | | + |

| | | | | | |
|---|---|---|---|---|---|
| w: with (additive added to each group is shown with +.) | | | | | |

### 4. Administration of human peripheral blood-derived CD8-positive T cells to mouse and recovery of cells engrafted cells

8-week-old male NSG mouse (Japan Charles River), which was irradiated with 2 Gy of gamma rays before the cell administration on the same day, was used. 5,000,000 cells adjusted in Culture of human peripheral blood-derived CD8-positive T cells used for mouse engraftment test in [Example 2] 3. were intravenously administered to mice, and the mice were bred for 4 weeks. Thereafter, the mice were euthanized and bone marrow cells, splenocytes, and leukocytes in the blood were collected from the mice.

### 5. Detection of human peripheral blood-derived CD8-positive T cells engrafted into mouse tissue

The collected cells were stained with the antibody of Table 3, and detected using LSRFortessa^{™} X-20 (BD Bioscience) flow cytometry.

### [Experimental Example 2]

### 1. Proliferation test of human peripheral blood-derived CD8-positive T cells by stimulation with anti-CD30 agonist antibody

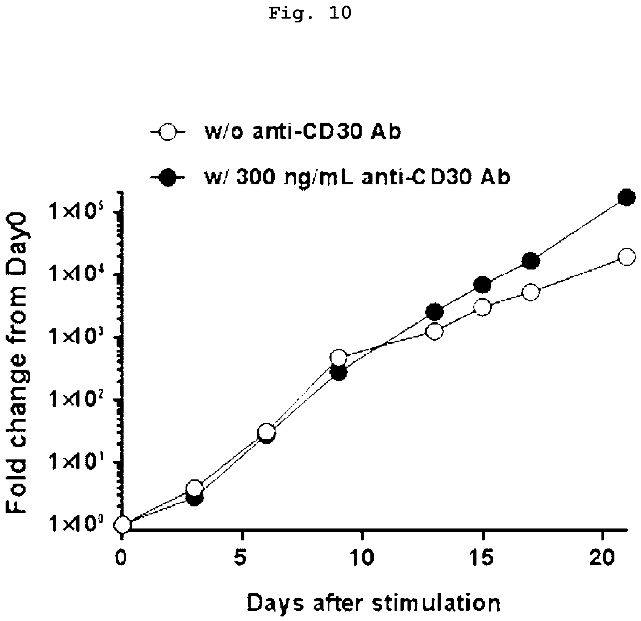

Whether addition of an anti-CD30 agonist antibody during stimulation with immobilized anti-CD3 antibody/RetroNectin (registered trade mark) influences proliferation of human peripheral blood-derived CD8-positive T cells was confirmed. Addition of the anti-CD30 agonist antibody promoted proliferation of CD8-positive T cells in human peripheral blood (Fig. 10).

### 2. Detection of CD197 on membrane surface of human peripheral blood-derived CD8-positive T cell after stimulation with anti-CD3/CD28 bead and anti-CD30 agonist antibody

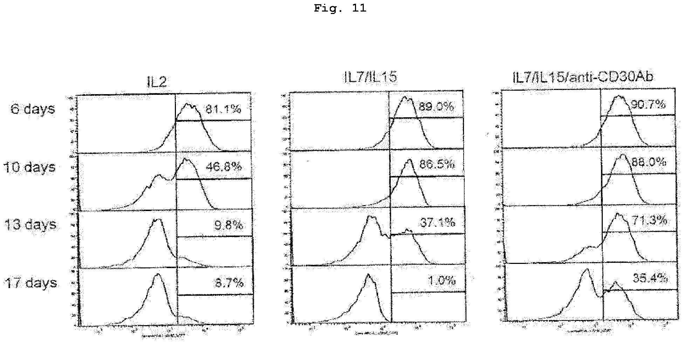

CD197 expression on the cell membrane surface of human peripheral blood-derived CD8-positive T cells stimulated with anti-CD3/CD28 beads in IL-2-containing medium or IL-7/IL-15-containing medium, IL-7/IL-15/anti-CD30 antibody-containing medium on days 6, 10, 13, 17 of culture was measured by flow cytometer. On day 6, CD197 expression was confirmed in all groups. However, CD197 expression almost disappeared on day 13 in the IL-2-containing medium group and on day 17 in the IL-7/IL-15-containing medium group, whereas CD197 expression was maintained even on day 17 in the IL-7/IL-15/anti-CD30 antibody-containing medium group (Fig. 11).

### 3. Evaluation of engraftment of human peripheral blood-derived CD8-positive T cells after stimulation with anti-CD3/CD28 bead and anti-CD30 agonist antibody

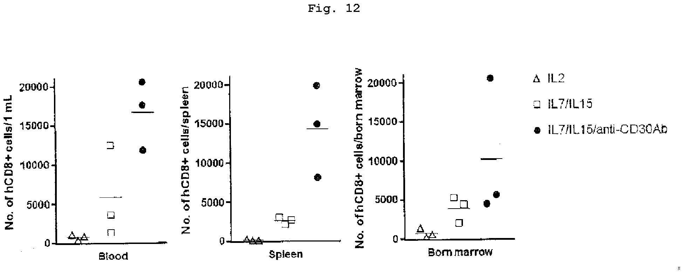

Human peripheral blood-derived CD8-positive T cells stimulated with anti-CD3/CD28 beads in IL-2-containing medium or IL-7/IL-15-containing medium, IL-7/IL-15/anti-CD30 antibody-containing medium on day 10 of culture were intravenously administered to immunodeficient mice, and engraftment in blood and immune tissues was confirmed. On day 28 after administration, blood, spleen and bone marrow were collected from the mice, and human CD8-positive T cells contained therein were detected by flow cytometry. Almost no human CD8-positive T cells were detected in the IL-2-containing medium group in any of the organs, whereas human CD8-positive T cells were detected in the IL-7/IL-15-containing medium group and IL-7/IL-15/anti-CD30 antibody containing medium group. In the IL-7/IL-15/anti-CD30 antibody-containing medium group, a larger number of human CD8-positive T cells were detected. Therefore, it was shown that human peripheral blood-derived CD8-positive T cells cultured in a medium containing IL-7/IL-15/anti-CD30 antibody can not only maintain the expression of CD197 for a long time during culture, but also can survive for a long time in vivo (Fig. 12).

### [Example 3]

### 1. Proliferation test of iPS cell-derived anti-CD19-CART cells

iPS cell-derived anti-CD19-CART cells prepared to 100,000 cells/200 µL in a medium prepared by adding cytokine and the like of Table 1 to α-MEM medium containing 15% FBS were seeded in a plate containing anti-CD3 antibody and RetroNectin immobilized thereon and cultured at 5% CO₂/37°C for 3 days. The cells were harvested from the plate on day 3 of culture and the number of the cells was measured using TC20 (Bio-Rad). The cells were suspended in an appropriate amount of a medium obtained by adding cytokine and the like shown in Table 2 to α-MEM medium containing 15% FBS, added to a non-immobilized plate and cultured at 5% CO₂/37°C. Thereafter, the cells were collected from the plate 4 to 7 times on any of days 5, 6, 7, 8, 9, 10, 12, 14, 16 of culture, and the number of cells was measured. The cells were suspended in an appropriate amount, added to a non-immobilized plate and cultured at 5% CO₂/37°C.

### 2. Anti-CD19-CAR gene

As an anti-CD19-CAR gene, an oligo DNA encoding a polypeptide (SEQ ID NO: 5) designed to align in the order shown in Table 6 from the N-terminal was artificially synthesized.

**[Table 6]**

| order from N-terminal | gene | amino acid number |
|---|---|---|
| 1 | lead sequence of immunoglobulin heavy chain | 22 |
| 2 | variable region of anti-CD19 antibody (FMC60) light chain | 104 |
| 3 | GGGGS linker | 15 |
| 4 | variable region of anti-CD19 antibody (FMC60) heavy chain | 120 |
| 5 | CD8-derived sequence (including transmembrane region) | 83 |
| 6 | intracellular domain region of CD28 | 41 |
| 7 | intracellular domain region of 4-1BB | 47 |
| 8 | intracellular domain region of CD3ζ | 112 |

### 3. Production of retrovirus vector carrying anti-CD19-CAR gene

The artificial oligo DNA synthesized in [Example 3] 2. was incorporated into the multicloning site of pMEI-5 retrovirus vector. The production of the virus vector was outsourced to Unitech.

### 4. Production of iPS cell-derived anti-CD19-CART cells

The iPS cell-derived T cells produced in [Example 1] 3. were infected with the retrovirus vector carrying an anti-CD19-CAR gene and produced in [Example 3] 3., whereby iPS cell-derived anti-CD19-CART cells was produced.

### [Experimental Example 3]

### 1. Proliferation test of iPS cell-derived anti-CD19-CART cells stimulated with immobilized anti-CD3 agonist antibody/RetroNectin (registered trade mark)

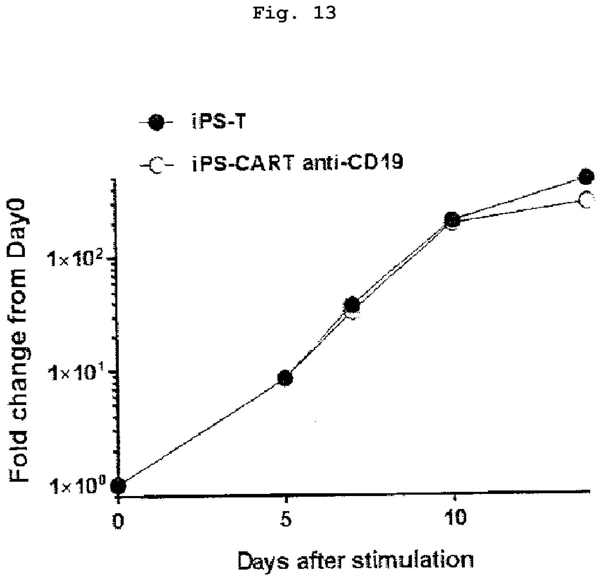

The iPS cell-derived T cells produced in [Example 1] 3. and the iPS cell-derived anti-CD19-CART cells produced in [Example 3] 4. were stimulated for 3 days with immobilized anti-CD3 antibody/RetroNectin (registered trade mark), and the number of cells cultured without stimulation was measured over time. The iPS cell-derived anti-CD19-CART cells were proliferated to the same level as iPS cell-derived T cells by the stimulation with immobilized anti-CD3 antibody/RetroNectin (registered trade mark) (Fig. 13).

### 2. Proliferation test of iPS cell-derived anti-CD19-CART cells by stimulation with anti-CD30 agonist antibody

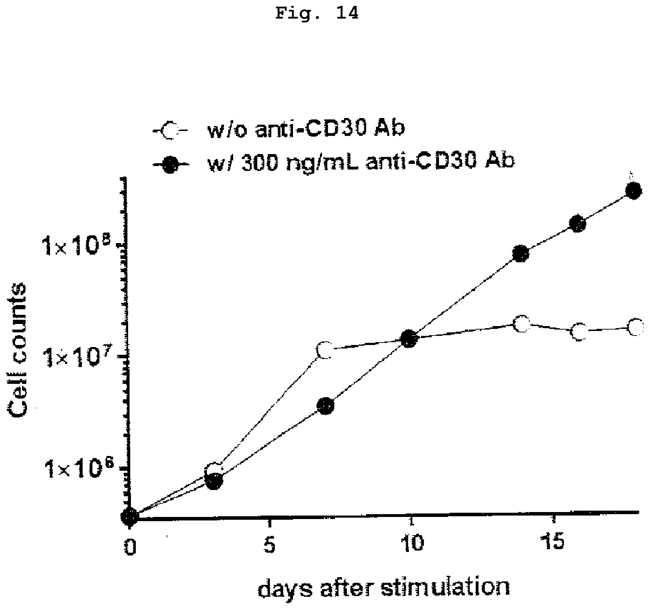

Whether addition of an anti-CD30 agonist antibody during stimulation with immobilized anti-CD3 antibody/RetroNectin (registered trade mark) influences proliferation of iPS cell-derived anti-CD19-CART cells was confirmed. Addition of the anti-CD30 agonist antibody promoted proliferation of iPS cell-derived anti-CD19-CART cells (Fig. 14).

### 3. Detection of CD197 on membrane surface of iPS cell-derived anti-CD19-CART cell after stimulation with immobilized anti-CD3 agonist antibody/RetroNectin (registered trade mark) and anti-CD30 agonist antibody

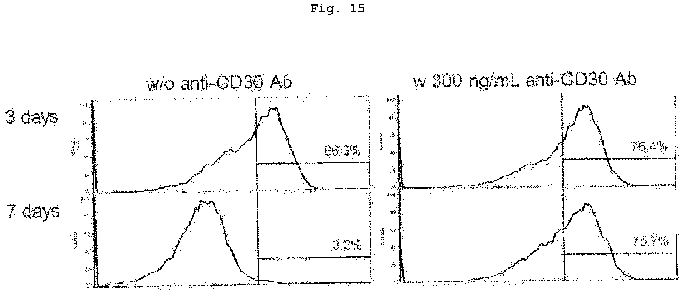

CD197 expression on the cell membrane surface of iPS cell-derived anti-CD19-CART cells on days 3 and 7 after stimulation with immobilized anti-CD3 antibody/RetroNectin (registered trade mark), or after stimulation with immobilized anti-CD3 antibody/RetroNectin (registered trade mark)/anti-CD30 agonist antibody was measured by flow cytometer. On day 3, CD197 expression was confirmed in both groups. However, CD197 expression almost disappeared on day 7 in the anti-CD3 antibody/RetroNectin (registered trade mark) stimulation group, but it was confirmed that the CD197 expression was maintained in the anti-CD3 antibody/RetroNectin (registered trade mark)+anti-CD30 agonist antibody stimulation group (Fig. 15).

### 4. Verification of Raji cytotoxic activity of iPS cell-derived anti-CD19-CART cell after stimulation with immobilized anti-CD3 agonist antibody/RetroNectin (registered trade mark) and anti-CD30 agonist antibody

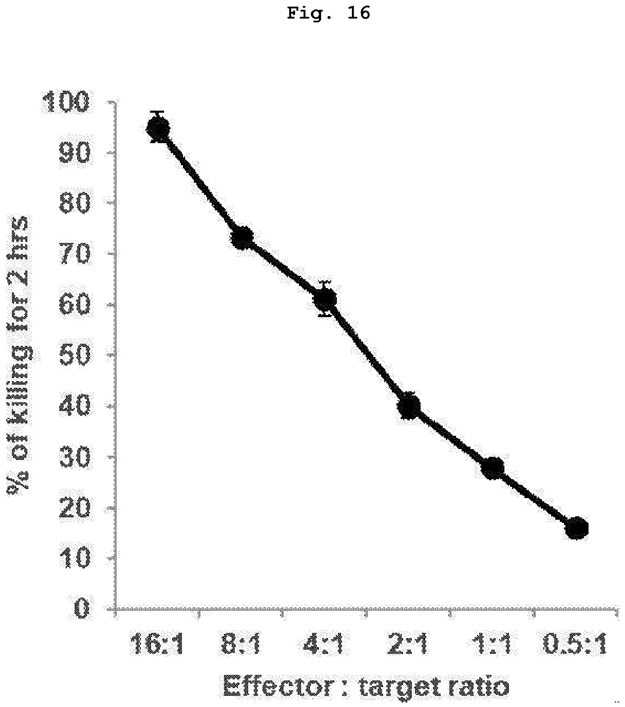

The cytotoxic activity of iPS cell-derived anti-CD19-CART cells obtained by proliferation in [Experimental Example 3] 1. against CD19-positive cancer cells was evaluated by the cytotoxic activity against CD19-positive Raji cells. The iPS cell-derived anti-CD19-CART cells showed cytotoxic activity against CD19-expressing Raji cells (Fig. 16).

### [Example 4]

### 1. Anti-CD19-CAR gene containing CD30-derived intracellular domain

As anti-CD19-CAR gene, an oligo DNA encoding a polypeptide (SEQ ID NO: 7) designed to align in the order shown in Table 7 from the N-terminal was artificially synthesized.

**[Table 7]**

| order from N-terminal | gene | amino acid number |
|---|---|---|
| 1 | lead sequence of immunoglobulin heavy chain | 22 |
| 2 | variable region of anti-CD19 antibody (FMC60) light chain | 104 |
| 3 | GGGGS linker | 15 |
| 4 | variable region of anti-CD19 antibody (FMC60) heavy chain | 120 |
| 5 | CD8-derived sequence (including transmembrane region) | 83 |
| 6 | intracellular domain region of CD28 | 41 |
| 7 | intracellular domain region of CD30 (SEQ ID NO: 6) | 87 |
| 8 | intracellular domain region of CD3ζ | 112 |

### 2. Production of retrovirus vector carrying anti-CD19-CAR gene containing CD30-derived intracellular domain

The artificial oligo DNA synthesized in [Example 4] 1. was incorporated into the multicloning site of pMY retrovirus vector. Using FLYRD18 cells for retrovirus vector production, a virus vector was produced.

### 3. Production of iPS cell-derived anti-CD19-CART cells (iCD19-CD30-CART) containing CD30-derived intracellular domain

The iPS cell-derived T cells produced in [Example 1]3. were infected with the retrovirus vector carrying an anti-CD19-CAR gene and produced in [Example 4] 2., whereby iPS cell-derived anti-CD19-CART cells (iCD19-CD30-CART) containing a CD30-derived intracellular domain were produced.

### [Experimental Example 4]

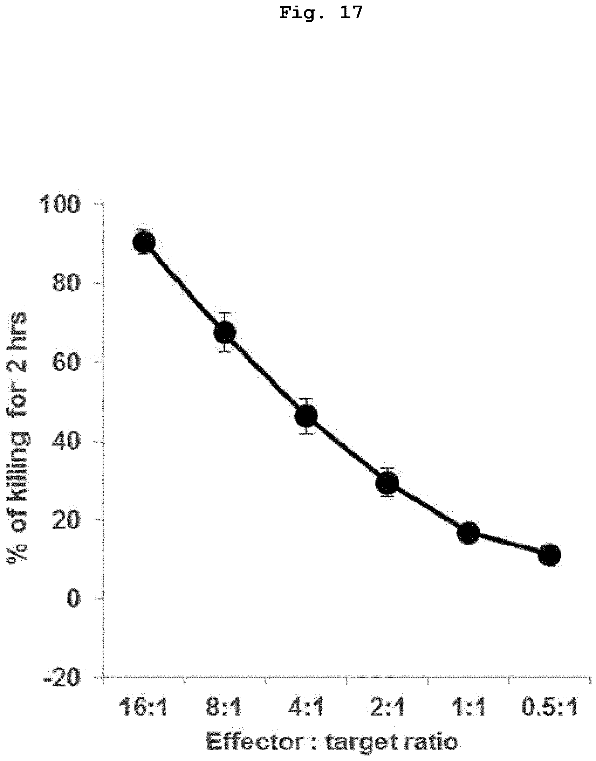

The cytotoxic activity of iCD19-CD30-CART obtained in [Example 4] 3. against CD19-positive cancer cells was evaluated by the cytotoxic activity against CD19-positive Raji cells. The iCD19-CD30-CART showed cytotoxic activity against CD19-expressing Raji cells (Fig. 17).

### [Example 5]

### 1. Preparation of iPS cell

Similar to [Example 1], as the iPS cell, Ff-I01s04 strain provided by the Center for iPS Cell Research and Application (CiRA), Kyoto University, was used. iPS cell culture was performed according to the protocol distributed by CiRA, "Human iPS cell culture under feeder-free conditions".

### 2. Differentiation of iPS cells into γδTCR-positive T cells (γδT cells)

Differentiation of iPS cells into y5TCR-positive T cells (γδT cells) was performed according to a known method (WO 2017/221975) as in [Example 1]. As the anti-CD3 antibody used in the differentiation step, 3000 ng/mL UCHT1 (manufactured by GeneTex) was used. The obtained CD3-positive cells were γδT cells (hereinafter to be referred to as "iPS cell-derived γδT cells (iγδT cells)").

### 3. Expansion culture of iPS cell-derived γδT cells

The iγδT cells obtained in [Example 5] 2. were suspended at 2,000,000 cells/mL in a medium obtained by adding cytokine shown in Table 8 to α-MEM medium containing 15% FBS, and the suspension was seeded in a plate containing anti-CD3 antibody (UCHT1) and RetroNectin (registered trade mark) immobilized thereon and cultured at 5% CO₂/37°C for 3 days. The cells were harvested from the plate on day 3 of culture and the number of the cells was measured using NucleoCounter NC-200 (ChemoMetec). The cells were suspended in an appropriate amount of a medium obtained by adding cytokine and the like shown in Table 9 to α-MEM medium containing 15% FBS, added to a non-immobilized G-Rex 6-well plate (WILSONWOLF) and cultured at 5%CO₂/37°C. Thereafter, a part of the cells were collected from the plate 4 to 6 times on any of days 5, 6, 7, 8, 9, 10, 11, 14, 17 of culture, and the number of cells was measured. The cells were immobilized on the culture plate with anti-CD3 antibody and RetroNectin (registered trade mark) by the following method. An anti-CD3 antibody (UCHT1, final concentration 3000 ng/mL) and RetroNectin (final concentration 150 µg/mL) dissolved in PBS at necessary concentrations were added to the plate and the plate was stood overnight at 4°C. The plate was washed with PBS and subjected to the test.

**[Table 8]**

| product name | manufacturer | Final conc | w/ anti-CD30 Ab | w/o anti-CD30 Ab |
|---|---|---|---|---|
| Insulin-Transferrin-Selenium Supplements | Invitrogen | 1 x | + | + |
| Ascorbic acid 2-phosphate | sigma | 50 µg/ml | + | + |
| IL-2 | Peprotech | 15 ng/ml | + | + |
| IL-7 | Peprotech | 10 ng/ml | + | + |
| IL-15 | Peprotech | 10 ng/ml | + | + |
| IL-21 | Peprotech | 20 ng/ml | + | + |
| IL-12 | Merck | 50 ng/ml | + | + |
| IL-18 | MBL | 50 ng/ml | + | + |
| TL-1A | Peprotech | 50 ng/ml | + | + |
| Z-VAD-FMK | R&D | 10 µM | + | + |
| Human CD30 Antibody | R&D | 300 ng/ml | | + |

| | | | | |
|---|---|---|---|---|
| w/: with, w/o without Additive added to each group is shown with "+". | | | | |

**[Table 9]**

| product name | manufacturer | Final conc | w/ anti-CD30 Ab | w/o anti-CD30 Ab |
|---|---|---|---|---|
| Insulin-Transferrin-Selenium Supplements | Invitrogen | 1 x | + | + |
| Ascorbic acid 2-phosphate | sigma | 50 µg/ml | + | + |
| IL-2 | Peprotech | 15 ng/ml | + | + |
| IL-7 | Peprotech | 10 ng/ml | + | + |
| IL-15 | Peprotech | 10 ng/ml | + | + |
| Human CD30 Antibody | R&D | 300 ng/ml | + | + |

| | | | | |
|---|---|---|---|---|
| w/: with, w/o without Additive added to each group is shown with "+". | | | | |

### [Experimental Example 5]

### 1. Proliferation test of iPS cell-derived γδT cells by stimulation with anti-CD30 agonist antibody

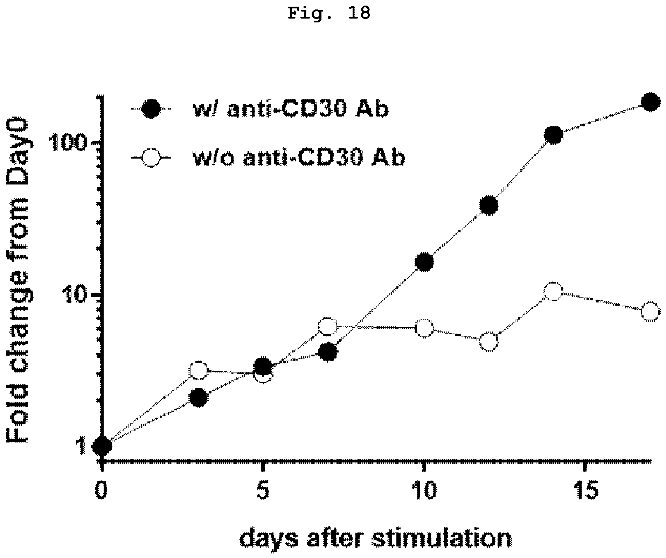

Whether addition of an anti-CD30 agonist antibody (300 ng/mL) to the stimulation method using immobilized anti-CD3 agonist antibody/RetroNectin (registered trade mark) in [Example 5] 3. influences proliferation of iPS cell-derived γδT cells was verified. iPS cell-derived T cells were proliferated by the addition of an anti-CD30 agonist antibody (Fig. 18).

### [Example 6]

### 1. IL-15Rα/IL-15 gene

As IL-15Rα/IL-15 gene, an oligo DNA encoding a polypeptide (SEQ ID NO: 8) designed to align in the order shown in Table 10 from the N-terminal was artificially synthesized.

**[Table 10]**

| order from N-terminal | gene | amino acid number |
|---|---|---|
| 1 | lead sequence of human IL-2 | 23 |
| 2 | C-terminal sequence of human IL-15 | 114 |
| 3 | GGGGS linker | 24 |
| 4 | C-terminal sequence of human IL-15RA | 239 |

### 2. Production of retrovirus vector carrying IL-15Rα/IL-15 gene

The artificial oligo DNA synthesized inn [Example 6] 1. was incorporated into the multicloning site of pMY retrovirus vector. Using FLYRD18 cells for retrovirus vector production, a virus vector was produced.

### 3. Production of iPS cell-derived anti-CD19-CAR/IL-15γδT cells

The iPS cell-derived γδT cells (iγδT cells) produced in [Example 5] 2. were infected with the retrovirus vector carrying anti-CD19-CAR gene and produced in [Example 3] 3. and the retrovirus vector carrying IL-15Rα/IL-15 gene and produced in [Example 6] 1., whereby iPS cell-derived anti-CD19-CAR/IL-15γδT cells (iCD19CAR/IL-15γδT cells) were produced.

### 4. Expansion culture of iPS cell-derived anti-CD19-CAR/IL-15γδT cells

By a method similar to that in [Example 5]3., expansion culture of iCD19CAR/IL-15γδT cells was performed except that IL-2 was not added.

### [Experimental Example 6]

### 1. Proliferation test of iPS cell-derived anti-CD19-CAR/IL-15γδT cells by stimulation with anti-CD30 agonist antibody

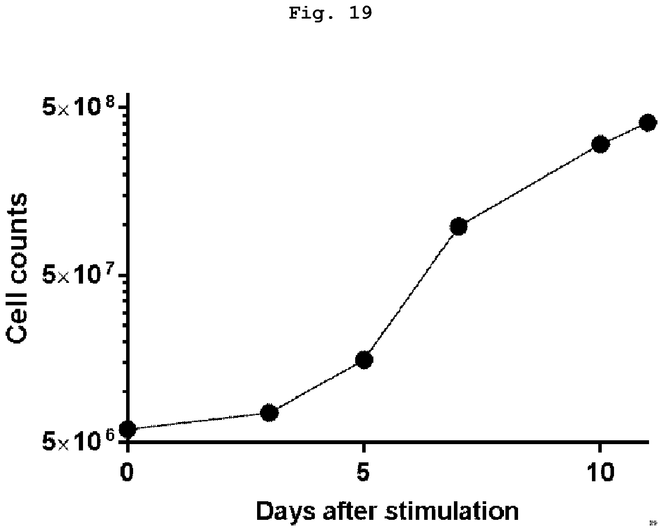

Whether addition of an anti-CD30 agonist antibody (0, 30, 100, 300 ng/mL) during stimulation with immobilized anti-CD3 agonist antibody/RetroNectin (registered trade mark) in [Example 6] 4. influences proliferation of iCD19CAR/IL-15γδT cells was verified. The cells were proliferated more by the addition of an anti-CD30 agonist antibody (Fig. 19).

### 2. Study of cytotoxic activity of iPS cell-derived anti-CD19-CAR/IL-15γδT cell after stimulation with immobilized anti-CD3 agonist antibody/RetroNectin (registered trade mark) and anti-CD30 agonist antibody

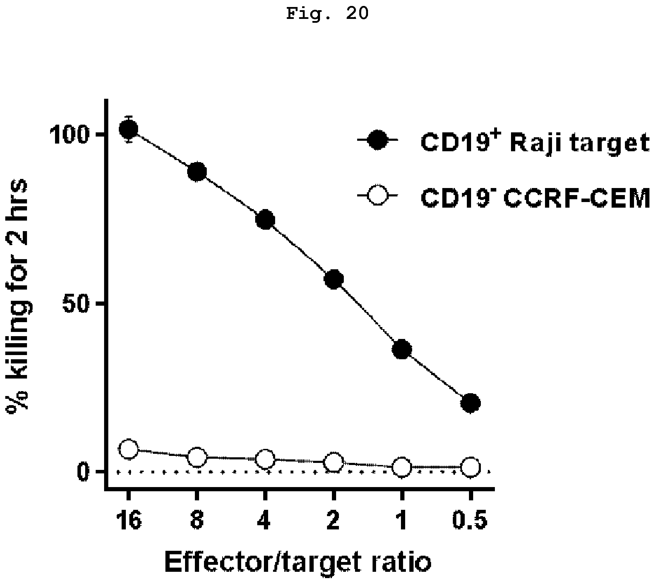

The cytotoxic activity of iCD19CAR/IL-15γδT cells obtained in [Example 6] 4., was evaluated. Using CD19-positive Raji cell and CD19 negative CCRF-CEN cell as target cells, iCD19CAR/IL-15γδT cells were mixed at a proportion of 0.5, 1, 2, 4, 8, 16-fold relative to the target cell. The cytotoxic activity of iCD19CAR/IL-15γδT cells was evaluated based on the target cell death 2 hr later. It was shown that iCD19CAR/IL-15γδT cells expansion cultured in a medium containing an anti-CD30 agonist antibody has cytotoxic activity against CD19-positive Raji cell, does not have cytotoxic activity against CD19-negative CCRF-CEN cells (Fig. 20).

### 3. Number of survival day-increasing effect of iPS cell-derived anti-CD19-CAR/IL-15γδT cells after stimulation with immobilized anti-CD3 agonist antibody/RetroNectin (registered trade mark) and anti-CD30 agonist antibody

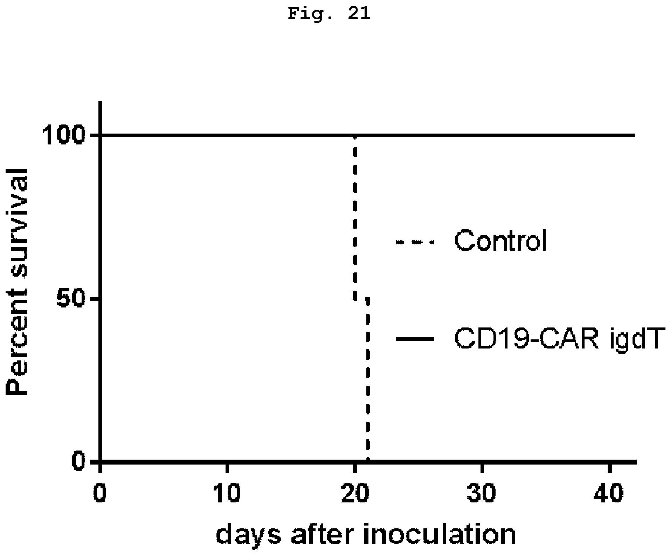

5×10⁵ Nalm6 cells (ATCC) were transplanted to NOD/Shi-scid, IL-2RyKO (NOG) mice (Central Institute for Experimental Animals, female, 7-8-week-old) from the tail vein to prepare Nalm6 xenograft mice. On day 4 post-transplantation, a suspension of iCD19CAR/IL-15y5T cells (5×10⁶ (cells)) obtained in [Example 6] 4. in 0.1 mL of HBSS-buffer or an equal amount of HBSS-buffer was administered from the tail vein, and the number of days of survival was confirmed. All mice transplanted with CD19-positive Nalm6 cancer cells via the tail vein died within 3 weeks in the control administration group, whereas all mice survived for at least 6 weeks in the iCD19CAR/IL-15γδT cell administration group expansion cultured in a medium containing an anti-CD30 agonist antibody (Fig. 21).

### [Example 7]

### 1. Production of iPS cell-derived anti-CD19-CAR/IL-15αβT cells

iPS cell-derived anti-CD19-CART cells produced by the method of [Example 3] 4. were infected with the retrovirus vector carrying IL15Rα/IL-15 gene of the produced in [Example 6] 2. to produce iPS cell-derived anti-CD19-CAR/IL-15αβT cells (iCD19CAR/IL-15αβT cells).

### 2. Expansion culture of iCD19CAR/IL-15αβT cells by using immobilized anti-CD3 agonist antibody/RetroNectin (registered trade mark)

The iCD19CAR/IL-15αβB cells obtained in [Example 7] 1. were cultured by a method similar to that in [Example 6] 4. until day 7. An anti-human CD30 antibody was not added.

### [Experimental Example 7]

### 1. In vivo antitumor effect of iCD19CAR/IL-15αβB cells after stimulation with immobilized anti-CD3 agonist antibody/RetroNectin (registered trade mark)

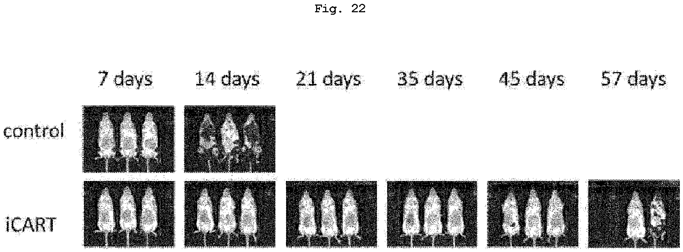

5×10⁵ luciferase-expressing Nalm6 cells (ATCC) were transplanted to NOD/Shi-scid, IL-2RyKO (NOG) mice (Central Institute for Experimental Animals, female, 7-8-week-old) from the tail vein to prepare luciferase-expressing Nalm6 xenograft mice. On day 4 post-transplantation, a suspension of iCD19CAR/IL-15αβB cells (5×10⁶ cells) obtained in [Example 7] 2. in 0.1 mL of HBSS-buffer or an equal amount of HBSS-buffer was administered from the tail vein. Luciferin was administered from the tail vein every week after administration, and the activity of luciferase expressed by Nalm6 cells was measured using IVIS. In the control-administered group, luminescence derived from Nalm6 cells was confirmed throughout the body 2 weeks after administration, and all mice died by 3 weeks after administration, whereas no luminescence was detected until 6 weeks after administration in the iCD19CAR/IL-15αβB cell-administered group (Fig. 22).

### [Example 8]

### 1. Production of iPS cell-derived γδT cells

In the same manner as in [Example 5] 1. and 2., iPS cell-derived γδT cells (iγδT cells) were produced.

### [Experimental Example 8]

### 1. Measurement of concentration of anti-CD3 agonist antibody (UCHT1) suitable for immobilization on culture plate and concentration of RetroNectin (registered trade mark)

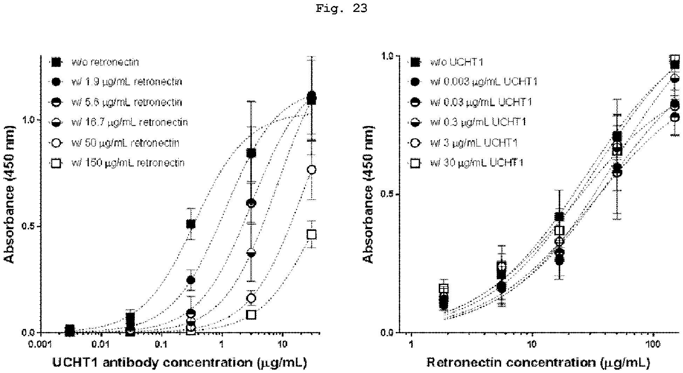

An anti-CD3 agonist antibody (UCHT1) and RetroNectin (registered trade mark) were mixed and immobilized on a culture plate in the same manner as in [Example 1] 5. Thereafter, the amount thereof immobilized on a culture plate was measured using the ELISA method (Fig. 23). Concentration-dependent immobilization of an anti-CD3 agonist antibody free of mixing with RetroNectin (registered trade mark) was confirmed between 3 ng/mL and 3000 ng/mL (0.003 µg/ml to 30 µg/ml). However, the amount of immobilized anti-CD3 agonist antibody decreased as the concentration of RetroNectin (registered trade mark) to be mixed was raised. On the other hand, concentration-dependent immobilization of RetroNectin (registered trade mark) was confirmed between 16.7 µg/mL and 150 µg/mL.

### 2. Verification of concentration range of anti-CD3 agonist antibody and RetroNectin (registered trade mark) necessary for immobilized anti-CD3 agonist antibody and immobilized RetroNectin (registered trade mark) suitable for proliferation of iPS cell-derived iγδT cells

iγδT cells adjusted to 100,000 cells/200 µL in a medium obtained by adding cytokine and the like of Table 1, and anti-CD30 agonist antibody diluted to a final concentration of 0, 3, 30, 300 ng/ml to IMDM medium containing 15% FBS were seeded in a plate with an anti-CD3 agonist antibody (0, 300, 3000, 30000 ng/ml (0, 0.3, 3, 30 µg/ml)) and RetroNectin (registered trade mark) (0, 2,15, 150 µg/mL) immobilized thereon, and cultured for 3 days under 5% CO₂/37°C.

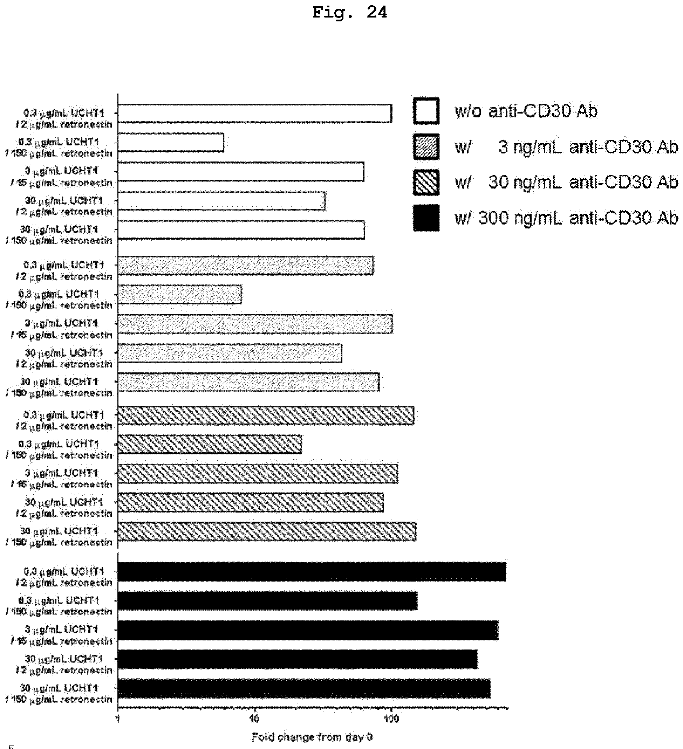

On day 3 of culture, the cells were harvested from the plate, suspended in an appropriate amount of a medium obtained by adding cytokine and the like in Table 2 to IMDM medium containing 15% FBS, seeded in a non-immobilized 96 well plate and cultured at 5% CO₂/37°C. Thereafter, the cells were collected from the plate on any of days 5, 6, 7, 8, 9, 10, and 12 once each timing and 4 to 7 times in total, suspended in an appropriate amount of a medium, seeded in a non-immobilized plate and cultured at 5% CO₂/37°C. On day 13 of culture, the cells were counted using a hemocytometer, and the proliferation rate based on the comparison with the number on day 0 of culture was measured (Fig. 24). Equivalent induction of iγδT cell proliferation was observed in the plates on which a mixture of anti-CD3 agonist antibody and RetroNectin (registered trade mark) was immobilized at 0.3 µg/mL and 2 pg/mL, 3 µg/mL and 15 µg/mL, 30 µg/mL and 150 pg/mL, respectively. Also, anti-CD30 agonist antibody addition concentration-dependent cell proliferation was confirmed.

### [Industrial Applicability]

T cells can be efficiently expansion cultured repeatedly by culturing CD3-positive cells in the presence of a CD3/TCR complex agonist, fibronectin or a variant thereof, and a CD30 agonist. When the CD3-positive cells to be cultured are CD3-positive CD8-positive cell or CD3-positive CD8-positive CD30-positive cells, the produced or expansion cultured cell easily shift to effector T cells and exhibit cytotoxic activity. Therefore, it can be applied to T cell therapy.

This application is based on patent application No. 2018-151580 filed in Japan (filing date: August 10, 2018), No. 2019-042666 (filing date: March 8, 2019) and No. 2019-117878 (filing date: June 25, 2019), the contents of which are incorporated in full herein.

### Preferred Embodiments

1. A method for producing a CD3-positive cell, comprising step (I) of culturing the CD3-positive cell in the presence of a CD3/TCR complex agonist, fibronectin or a variant thereof, and a CD30 agonist.
2. The method according to item 1, further comprising step (II) of culturing the CD3-positive cell cultured in step (I) in the absence of a CD3/TCR complex agonist and fibronectin or a variant thereof, and in the presence of a CD30 agonist.
3. The method according to item 1 or 2, wherein the CD3-positive cell is derived from a pluripotent stem cell.
4. The method according to item 3, wherein the pluripotent stem cell is an iPS cell.
5. The method according to any one of items 1 to 4, wherein the CD3-positive cell is a chimeric antigen receptor-expressing CD3-positive cell.
6. The method according to any one of items 1 to 5, wherein the CD3-positive cell is a CD3-positive CD8-positive cell.
7. The method according to item 6, wherein the CD3-positive CD8-positive cell is a CD3-positive CD8-positive CD4-negative cell.
8. The method according to any one of items 1 to 7, wherein the CD3-positive cell is a γTCR-positive and/or δTCR-positive cell.
9. The method according to any one of items 1 to 8, wherein the CD3/TCR complex agonist is a CD3 agonist and/or a TCR agonist.
10. The method according to item 9, wherein the CD3 agonist is an anti-CD3 agonist antibody or a binding fragment thereof.
11. The method according to item 10, wherein the anti-CD3 agonist antibody or a binding fragment thereof is an anti-CD3 agonist antibody or a binding fragment thereof produced from UCHT1 clone.
12. The method according to item 9, wherein the TCR agonist is at least one selected from the group consisting of an anti-TCR antibody or a binding fragment thereof, an HLA/peptide complex or a multimer thereof, and an HLA/superantigen complex or a multimer thereof.
13. The method according to item 10 or 11, wherein the anti-CD3 agonist antibody or a binding fragment thereof is immobilized on a culture container.
14. The method according to item 13, wherein the anti-CD3 agonist antibody or a binding fragment thereof is immobilized by contacting 1 ng/ml - 50000 ng/ml of the anti-CD3 agonist antibody or a binding fragment thereof with the culture container.
15. The method according to any one of items 1 to 14, wherein the fibronectin or a variant thereof is RetroNectin (registered trade mark).
16. The method according to item 15, wherein the RetroNectin (registered trade mark) is immobilized on the culture container.
17. The method according to item 16, wherein the RetroNectin (registered trade mark) is immobilized by contacting 1 - 150 µg/mL of the RetroNectin (registered trade mark) with the culture container.
18. The method according to any one of items 1 to 17, wherein the CD30 agonist is at least one selected from the group consisting of an anti-CD30 agonist antibody or a binding fragment thereof and a CD30 ligand or a binding fragment thereof.
19. The method according to item 18, wherein the anti-CD30 agonist antibody or a binding fragment thereof is contained in the medium.
20. The method according to item 19, wherein a concentration of the anti-CD30 agonist antibody or a binding fragment thereof in the medium of 1 ng/ml - 1000 ng/ml.
21. The method according to any one of items 1 to 20, wherein the medium comprises at least one selected from IL-7, IL-15, IL-18 and IL-21.
22. The method according to item 21, wherein the medium comprises IL-7, IL-15, IL-18 and IL-21.
23. The method according to item 21 or 22, wherein the medium further comprises TL1A and/or IL-12.
24. The method according to any one of items 1 to 23, wherein the CD3-positive cell produced is further CD197-positive.
25. A CD3-positive cell obtained by the method according to any one of items 1 to 24.
26. A method for expansion culture of a CD3-positive cell, comprising a step of culturing the CD3-positive cell in the presence of a CD3/TCR complex agonist, fibronectin or a variant thereof, and a CD30 agonist.
27. A kit for expansion culture of a CD3-positive cell, comprising the following:
   (1) a culture container in which a CD3/TCR complex agonist, and fibronectin or a variant thereof are immobilized, and
   (2) a medium comprising a CD30 agonist.
28. A method for maintaining a CD3-positive cell as a CD3-positive CD197-positive cell, comprising a step of stimulating a CD30 signal in the CD3-positive cell.
29. The method according to item 28, wherein the CD3-positive cell is a CD3-positive CD8-positive cell.
30. The method according to item 29, wherein the CD3-positive CD8-positive cell is a CD3-positive CD8-positive CD4-negative cell.
31. The method according to any one of items 28 to 30, wherein the medium used in the step of stimulation comprises at least one selected from IL-7, IL-15, IL-18 and IL-21.
32. The method according to item 31, wherein the medium used in the step of stimulation comprises IL-7, IL-15, IL-18 and IL-21.
33. The method according to item 31 or 32, wherein the medium used in the step of stimulation further comprises TL1A and/or IL-12.
34. A kit for maintaining a CD3-positive cell as a CD3-positive CD197-positive cell, comprising a CD30 agonist.
35. A chimeric antigen receptor comprising an antigen binding domain, a transmembrane domain and an intracellular signal transduction domain, wherein the intracellular signal transduction domain comprises a CD30 intracellular signal transduction domain or a mutant thereof.
36. A nucleic acid comprising a polynucleotide encoding the chimeric antigen receptor according to item 35.
37. A chimeric antigen receptor expression vector comprising the nucleic acid according to item 36.
38. A chimeric antigen receptor-expressing cell comprising the chimeric antigen receptor expression vector according to item 37.
39. The cell according to item 38, wherein the chimeric antigen receptor expression cell is a CD3-positive cell.
40. A medicament comprising the cell according to item 38 or 39.
41. The medicament according to item 40 for use in the prophylaxis or treatment of a tumor expressing an antigen recognized by the chimeric antigen receptor according to item 35.
42. An agent for killing a cell expressing an antigen recognized by the chimeric antigen receptor according to item 35, wherein the agent comprises the cell according to item 38 or 39.
43. The cell according to item 38 or 39 for use in the prophylaxis or treatment of a tumor expressing an antigen recognized by the chimeric antigen receptor according to item 35.
44. Use of the cell according to item 38 or 39 in producing an agent for the prophylaxis or treatment of a tumor expressing an antigen recognized by the chimeric antigen receptor according to item 35.
45. A method for preventing or treating a tumor expressing an antigen recognized by the chimeric antigen receptor according to item 35, comprising administering the cell according to item 38 or 39.

## Claims

1. A method for maintaining a CD3-positive cell as a CD3-positive CD197-positive cell, comprising a step of stimulating a CD30 signal in the CD3-positive cell.

2. The method according to claim 1, wherein the CD3-positive cell is a CD3-positive CD8-positive cell.

3. The method according to claim 2, wherein the CD3-positive CD8-positive cell is a CD3-positive CD8-positive CD4-negative cell.

4. The method according to any one of claims 1 to 3, wherein the medium used in the stimulation step comprises at least one selected from IL-7, IL-15, IL-18 and IL-21.

5. The method according to claim 4, wherein the medium used in the stimulation step comprises IL-7, IL-15, IL-18 and IL-21.

6. The method according to claim 4 or 5, wherein the medium used in the stimulation step further comprises TL1A and/or IL-12.

7. A kit for maintaining a CD3-positive cell as a CD3-positive CD197-positive cell, comprising a CD30 agonist.

8. A chimeric antigen receptor comprising an antigen binding domain, a transmembrane domain and an intracellular signal transduction domain, wherein the intracellular signal transduction domain comprises a CD30 intracellular signal transduction domain or a mutant thereof.

9. A nucleic acid comprising a polynucleotide encoding the chimeric antigen receptor according to claim 8.

10. A chimeric antigen receptor expression vector comprising the nucleic acid according to claim 9.

11. A chimeric antigen receptor-expressing cell comprising the chimeric antigen receptor expression vector according to claim 10.

12. The cell according to claim 11, wherein the chimeric antigen receptor expression cell is a CD3-positive cell.

13. A medicament comprising the cell according to claim 11 or 12.

14. The medicament according to claim 13 for use in the prophylaxis or treatment of a tumor expressing an antigen recognized by the chimeric antigen receptor according to claim 8.

15. An agent for killing a cell expressing an antigen recognized by the chimeric antigen receptor according to claim 8, which agent comprises the cell according to claim 11 or 12.

16. The cell according to claim 11 or 12 for use in the prophylaxis or treatment of a tumor expressing an antigen recognized by the chimeric antigen receptor according to claim 8.


SEQUENCE LISTING

<110>  KYOTO UNIVERSITY
       TAKEDA PHARMACEUTICAL COMPANY LIMITED

<120>  A method of producing CD3-positive cells

<130>  092932

<150>  JP 2018-151580
<151>  2018-08-10

<150>  JP 2019-042666
<151>  2019-03-08

<150>  JP 2019-117878
<151>  2019-06-25

<160>  24

<170>  PatentIn version 3.5

<210>  1
<211>  9
<212>  PRT
<213>  Homo sapiens

<400>  1

Arg Met Phe Pro Asn Ala Pro Tyr Leu
1               5


<210>  2
<211>  9
<212>  PRT
<213>  Homo sapiens

<400>  2

Ser Leu Gly Glu Gln Gln Tyr Ser Val
1               5


<210>  3
<211>  9
<212>  PRT
<213>  Homo sapiens

<400>  3

Cys Met Thr Trp Asn Gln Met Asn Leu
1               5


<210>  4
<211>  9
<212>  PRT
<213>  Homo sapiens

<400>  4

Cys Tyr Thr Trp Asn Gln Met Asn Leu
1               5

<210>  5
<211>  544
<212>  PRT
<213>  Artificial Sequence

<220>
<223>  CD19-CAR

<400>  5

Met Asp Trp Thr Trp Arg Ile Leu Phe Leu Val Ala Ala Ala Thr Gly
1               5                   10                  15

Ala His Ser Asp Ile Gln Met Thr Gln Thr Thr Ser Ser Leu Ser Ala
            20                  25                  30

Ser Leu Gly Asp Arg Val Thr Ile Ser Cys Arg Ala Ser Gln Asp Ile
        35                  40                  45

Ser Lys Tyr Leu Asn Trp Tyr Gln Gln Lys Pro Asp Gly Thr Val Lys
    50                  55                  60

Leu Leu Ile Tyr His Thr Ser Arg Leu His Ser Gly Val Pro Ser Arg
65                  70                  75                  80

Phe Ser Gly Ser Gly Ser Gly Thr Asp Tyr Ser Leu Thr Ile Ser Asn
                85                  90                  95

Leu Glu Gln Glu Asp Ile Ala Thr Tyr Phe Cys Gln Gln Gly Asn Thr
            100                 105                 110

Leu Pro Tyr Thr Phe Gly Gly Gly Thr Lys Leu Glu Ile Thr Gly Gly
        115                 120                 125

Gly Gly Ser Gly Gly Gly Gly Ser Gly Gly Gly Gly Ser Glu Val Lys
    130                 135                 140

Leu Gln Glu Ser Gly Pro Gly Leu Val Ala Pro Ser Gln Ser Leu Ser
145                 150                 155                 160

Val Thr Cys Thr Val Ser Gly Val Ser Leu Pro Asp Tyr Gly Val Ser
                165                 170                 175

Trp Ile Arg Gln Pro Pro Arg Lys Gly Leu Glu Trp Leu Gly Val Ile
            180                 185                 190

Trp Gly Ser Glu Thr Thr Tyr Tyr Asn Ser Ala Leu Lys Ser Arg Leu
        195                 200                 205

Thr Ile Ile Lys Asp Asn Ser Lys Ser Gln Val Phe Leu Lys Met Asn
    210                 215                 220

Ser Leu Gln Thr Asp Asp Thr Ala Ile Tyr Tyr Cys Ala Lys His Tyr
225                 230                 235                 240

Tyr Tyr Gly Gly Ser Tyr Ala Met Asp Tyr Trp Gly Gln Gly Thr Ser
                245                 250                 255

Val Thr Val Ser Ser Phe Val Pro Val Phe Leu Pro Ala Lys Pro Thr
            260                 265                 270

Thr Thr Pro Ala Pro Arg Pro Pro Thr Pro Ala Pro Thr Ile Ala Ser
        275                 280                 285

Gln Pro Leu Ser Leu Arg Pro Glu Ala Cys Arg Pro Ala Ala Gly Gly
    290                 295                 300

Ala Val His Thr Arg Gly Leu Asp Phe Ala Cys Asp Ile Tyr Ile Trp
305                 310                 315                 320

Ala Pro Leu Ala Gly Thr Cys Gly Val Leu Leu Leu Ser Leu Val Ile
                325                 330                 335

Thr Leu Tyr Cys Asn His Arg Asn Arg Ser Lys Arg Ser Arg Leu Leu
            340                 345                 350

His Ser Asp Tyr Met Asn Met Thr Pro Arg Arg Pro Gly Pro Thr Arg
        355                 360                 365

Lys His Tyr Gln Pro Tyr Ala Pro Pro Arg Asp Phe Ala Ala Tyr Arg
    370                 375                 380

Ser Arg Phe Ser Val Val Lys Arg Gly Arg Lys Lys Leu Leu Tyr Ile
385                 390                 395                 400

Phe Lys Gln Pro Phe Met Arg Pro Val Gln Thr Thr Gln Glu Glu Asp
                405                 410                 415

Gly Cys Ser Cys Arg Phe Pro Glu Glu Glu Glu Gly Gly Cys Glu Leu
        420                 425                 430

Arg Val Lys Phe Ser Arg Ser Ala Asp Ala Pro Ala Tyr Gln Gln Gly
    435                 440                 445

Gln Asn Gln Leu Tyr Asn Glu Leu Asn Leu Gly Arg Arg Glu Glu Tyr
    450                 455                 460

Asp Val Leu Asp Lys Arg Arg Gly Arg Asp Pro Glu Met Gly Gly Lys
465                 470                 475                 480

Pro Arg Arg Lys Asn Pro Gln Glu Gly Leu Tyr Asn Glu Leu Gln Lys
                485                 490                 495

Asp Lys Met Ala Glu Ala Tyr Ser Glu Ile Gly Met Lys Gly Glu Arg
        500                 505                 510

Arg Arg Gly Lys Gly His Asp Gly Leu Tyr Gln Gly Leu Ser Thr Ala
    515                 520                 525

Thr Lys Asp Thr Tyr Asp Ala Leu His Met Gln Ala Leu Pro Pro Arg
    530                 535                 540

<210>  6
<211>  87
<212>  PRT
<213>  Homo sapiens

<400>  6

His Thr Asn Asn Lys Ile Glu Lys Ile Tyr Ile Met Lys Ala Asp Thr
1               5                   10                  15

Val Ile Val Gly Thr Val Lys Ala Glu Leu Pro Glu Gly Arg Gly Leu
            20                  25                  30

Ala Gly Pro Ala Glu Pro Glu Leu Glu Glu Glu Leu Glu Ala Asp His
        35                  40                  45

Thr Pro His Tyr Pro Glu Gln Glu Thr Glu Pro Pro Leu Gly Ser Cys
    50                  55                  60

Ser Asp Val Met Leu Ser Val Glu Glu Glu Gly Lys Glu Asp Pro Leu
65                  70                  75                  80

Pro Thr Ala Ala Ser Gly Lys

                85

<210>  7
<211>  965
<212>  PRT
<213>  Artificial Sequence

<220>
<223>  CD19-CAR(CD8-CD28-CD30-CD3z)

<400>  7

Met Asp Trp Thr Trp Arg Ile Leu Phe Leu Val Ala Ala Ala Thr Gly
1               5                   10                  15

Ala His Ser Asp Ile Gln Met Thr Gln Thr Thr Ser Ser Leu Ser Ala
            20                  25                  30

Ser Leu Gly Asp Arg Val Thr Ile Ser Cys Arg Ala Ser Gln Asp Ile
        35                  40                  45

Ser Lys Tyr Leu Asn Trp Tyr Gln Gln Lys Pro Asp Gly Thr Val Lys
    50                  55                  60

Leu Leu Ile Tyr His Thr Ser Arg Leu His Ser Gly Val Pro Ser Arg
65                  70                  75                  80

Phe Ser Gly Ser Gly Ser Gly Thr Asp Tyr Ser Leu Thr Ile Ser Asn
                85                  90                  95

Leu Glu Gln Glu Asp Ile Ala Thr Tyr Phe Cys Gln Gln Gly Asn Thr
            100                 105                 110

Leu Pro Tyr Thr Phe Gly Gly Gly Thr Lys Leu Glu Ile Thr Gly Gly
        115                 120                 125

Gly Gly Ser Gly Gly Gly Gly Ser Gly Gly Gly Gly Ser Glu Val Lys
    130                 135                 140

Leu Gln Glu Ser Gly Pro Gly Leu Val Ala Pro Ser Gln Ser Leu Ser
145                 150                 155                 160

Val Thr Cys Thr Val Ser Gly Val Ser Leu Pro Asp Tyr Gly Val Ser
                165                 170                 175

Trp Ile Arg Gln Pro Pro Arg Lys Gly Leu Glu Trp Leu Gly Val Ile
            180                 185                 190

Trp Gly Ser Glu Thr Thr Tyr Tyr Asn Ser Ala Leu Lys Ser Arg Leu
        195                 200                 205

Thr Ile Ile Lys Asp Asn Ser Lys Ser Gln Val Phe Leu Lys Met Asn
    210                 215                 220

Ser Leu Gln Thr Asp Asp Thr Ala Ile Tyr Tyr Cys Ala Lys His Tyr
225                 230                 235                 240

Tyr Tyr Gly Gly Ser Tyr Ala Met Asp Tyr Trp Gly Gln Gly Thr Ser
                245                 250                 255

Val Thr Val Ser Ser Phe Val Pro Val Phe Leu Pro Ala Lys Pro Thr
            260                 265                 270

Thr Thr Pro Ala Pro Arg Pro Pro Thr Pro Ala Pro Thr Ile Ala Ser
        275                 280                 285

Gln Pro Leu Ser Leu Arg Pro Glu Ala Cys Arg Pro Ala Ala Gly Gly
    290                 295                 300

Ala Val His Thr Arg Gly Leu Asp Phe Ala Cys Asp Ile Tyr Ile Trp
305                 310                 315                 320

Ala Pro Leu Ala Gly Thr Cys Gly Val Leu Leu Leu Ser Leu Val Ile
                325                 330                 335

Thr Leu Tyr Cys Asn His Arg Asn Arg Ser Lys Arg Ser Arg Leu Leu
            340                 345                 350

His Ser Asp Tyr Met Asn Met Thr Pro Arg Arg Pro Gly Pro Thr Arg
        355                 360                 365

Lys His Tyr Gln Pro Tyr Ala Pro Pro Arg Asp Phe Ala Ala Tyr Arg
    370                 375                 380

Ser His Thr Asn Asn Lys Ile Glu Lys Ile Tyr Ile Met Lys Ala Asp
385                 390                 395                 400

Thr Val Ile Val Gly Thr Val Lys Ala Glu Leu Pro Glu Gly Arg Gly
                405                 410                 415

Leu Ala Gly Pro Ala Glu Pro Glu Leu Glu Glu Glu Leu Glu Ala Asp
            420                 425                 430

His Thr Pro His Tyr Pro Glu Gln Glu Thr Glu Pro Pro Leu Gly Ser
        435                 440                 445

Cys Ser Asp Val Met Leu Ser Val Glu Glu Glu Gly Lys Glu Asp Pro
    450                 455                 460

Leu Pro Thr Ala Ala Ser Gly Lys Arg Val Lys Phe Ser Arg Ser Ala
465                 470                 475                 480

Asp Ala Pro Ala Tyr Gln Gln Gly Gln Asn Gln Leu Tyr Asn Glu Leu
                485                 490                 495

Asn Leu Gly Arg Arg Glu Glu Tyr Asp Val Leu Asp Lys Arg Arg Gly
            500                 505                 510

Arg Asp Pro Glu Met Gly Gly Lys Pro Arg Arg Lys Asn Pro Gln Glu
        515                 520                 525

Gly Leu Tyr Asn Glu Leu Gln Lys Asp Lys Met Ala Glu Ala Tyr Ser
    530                 535                 540

Glu Ile Gly Met Lys Gly Glu Arg Arg Arg Gly Lys Gly His Asp Gly
545                 550                 555                 560

Leu Tyr Gln Gly Leu Ser Thr Ala Thr Lys Asp Thr Tyr Asp Ala Leu
                565                 570                 575

His Met Gln Ala Leu Pro Pro Arg Leu Glu Gly Gly Gly Glu Gly Arg
            580                 585                 590

Gly Ser Leu Leu Thr Cys Gly Asp Val Glu Glu Asn Pro Gly Pro Arg
        595                 600                 605

Met Leu Leu Leu Val Thr Ser Leu Leu Leu Cys Glu Leu Pro His Pro
    610                 615                 620

Ala Phe Leu Leu Ile Pro Arg Lys Val Cys Asn Gly Ile Gly Ile Gly
625                 630                 635                 640

Glu Phe Lys Asp Ser Leu Ser Ile Asn Ala Thr Asn Ile Lys His Phe
                645                 650                 655

Lys Asn Cys Thr Ser Ile Ser Gly Asp Leu His Ile Leu Pro Val Ala
            660                 665                 670

Phe Arg Gly Asp Ser Phe Thr His Thr Pro Pro Leu Asp Pro Gln Glu
        675                 680                 685

Leu Asp Ile Leu Lys Thr Val Lys Glu Ile Thr Gly Phe Leu Leu Ile
    690                 695                 700

Gln Ala Trp Pro Glu Asn Arg Thr Asp Leu His Ala Phe Glu Asn Leu
705                 710                 715                 720

Glu Ile Ile Arg Gly Arg Thr Lys Gln His Gly Gln Phe Ser Leu Ala
                725                 730                 735

Val Val Ser Leu Asn Ile Thr Ser Leu Gly Leu Arg Ser Leu Lys Glu
            740                 745                 750

Ile Ser Asp Gly Asp Val Ile Ile Ser Gly Asn Lys Asn Leu Cys Tyr
        755                 760                 765

Ala Asn Thr Ile Asn Trp Lys Lys Leu Phe Gly Thr Ser Gly Gln Lys
    770                 775                 780

Thr Lys Ile Ile Ser Asn Arg Gly Glu Asn Ser Cys Lys Ala Thr Gly
785                 790                 795                 800

Gln Val Cys His Ala Leu Cys Ser Pro Glu Gly Cys Trp Gly Pro Glu
                805                 810                 815

Pro Arg Asp Cys Val Ser Cys Arg Asn Val Ser Arg Gly Arg Glu Cys
            820                 825                 830

Val Asp Lys Cys Asn Leu Leu Glu Gly Glu Pro Arg Glu Phe Val Glu
        835                 840                 845

Asn Ser Glu Cys Ile Gln Cys His Pro Glu Cys Leu Pro Gln Ala Met
    850                 855                 860

Asn Ile Thr Cys Thr Gly Arg Gly Pro Asp Asn Cys Ile Gln Cys Ala
865                 870                 875                 880

His Tyr Ile Asp Gly Pro His Cys Val Lys Thr Cys Pro Ala Gly Val
                885                 890                 895

Met Gly Glu Asn Asn Thr Leu Val Trp Lys Tyr Ala Asp Ala Gly His
            900                 905                 910

Val Cys His Leu Cys His Pro Asn Cys Thr Tyr Gly Cys Thr Gly Pro
        915                 920                 925

Gly Leu Glu Gly Cys Pro Thr Asn Gly Pro Lys Ile Pro Ser Ile Ala
    930                 935                 940

Thr Gly Met Val Gly Ala Leu Leu Leu Leu Leu Val Val Ala Leu Gly
945                 950                 955                 960

Ile Gly Leu Phe Met
                965

<210>  8
<211>  400
<212>  PRT
<213>  Homo sapiens

<400>  8

Met Tyr Arg Met Gln Leu Leu Ser Cys Ile Ala Leu Ser Leu Ala Leu
1               5                   10                  15

Val Thr Asn Ser Ala Thr Ser Asn Trp Val Asn Val Ile Ser Asp Leu
            20                  25                  30

Lys Lys Ile Glu Asp Leu Ile Gln Ser Met His Ile Asp Ala Thr Leu
        35                  40                  45

Tyr Thr Glu Ser Asp Val His Pro Ser Cys Lys Val Thr Ala Met Lys
    50                  55                  60

Cys Phe Leu Leu Glu Leu Gln Val Ile Ser Leu Glu Ser Gly Asp Ala
65                  70                  75                  80

Ser Ile His Asp Thr Val Glu Asn Leu Ile Ile Leu Ala Asn Asn Ser
                85                  90                  95

Leu Ser Ser Asn Gly Asn Val Thr Glu Ser Gly Cys Lys Glu Cys Glu
            100                 105                 110

Glu Leu Glu Glu Lys Asn Ile Lys Glu Phe Leu Gln Ser Phe Val His
        115                 120                 125

Ile Val Gln Met Phe Ile Asn Thr Ser Ser Gly Gly Gly Ser Gly Gly
    130                 135                 140

Gly Gly Ser Gly Gly Gly Gly Ser Gly Gly Gly Gly Ser Gly Gly Gly
145                 150                 155                 160

Ser Leu Gln Ile Thr Cys Pro Pro Pro Met Ser Val Glu His Ala Asp
                165                 170                 175

Ile Trp Val Lys Ser Tyr Ser Leu Tyr Ser Arg Glu Arg Tyr Ile Cys
            180                 185                 190

Asn Ser Gly Phe Lys Arg Lys Ala Gly Thr Ser Ser Leu Thr Glu Cys
        195                 200                 205

Val Leu Asn Lys Ala Thr Asn Val Ala His Trp Thr Thr Pro Ser Leu
    210                 215                 220

Lys Cys Ile Arg Asp Pro Ala Leu Val His Gln Arg Pro Ala Pro Pro
225                 230                 235                 240

Ser Thr Val Thr Thr Ala Gly Val Thr Pro Gln Pro Glu Ser Leu Ser
                245                 250                 255

Pro Ser Gly Lys Glu Pro Ala Ala Ser Ser Pro Ser Ser Asn Asn Thr
            260                 265                 270

Ala Ala Thr Thr Ala Ala Ile Val Pro Gly Ser Gln Leu Met Pro Ser
        275                 280                 285

Lys Ser Pro Ser Thr Gly Thr Thr Glu Ile Ser Ser His Glu Ser Ser
    290                 295                 300

His Gly Thr Pro Ser Gln Thr Thr Ala Lys Asn Trp Glu Leu Thr Ala
305                 310                 315                 320

Ser Ala Ser His Gln Pro Pro Gly Val Tyr Pro Gln Gly His Ser Asp
                325                 330                 335

Thr Thr Val Ala Ile Ser Thr Ser Thr Val Leu Leu Cys Gly Leu Ser
            340                 345                 350

Ala Val Ser Leu Leu Ala Cys Tyr Leu Lys Ser Arg Gln Thr Pro Pro
        355                 360                 365

Leu Ala Ser Val Glu Met Glu Ala Met Glu Ala Leu Pro Val Thr Trp
    370                 375                 380

Gly Thr Ser Ser Arg Asp Glu Asp Leu Glu Asn Cys Ser His His Leu
385                 390                 395                 400

<210>  9
<211>  5
<212>  PRT
<213>  Mus musculus

<400>  9

Arg Tyr Thr Met His
1               5

<210>  10
<211>  17
<212>  PRT
<213>  Mus musculus

<400>  10

Tyr Ile Asn Pro Ser Arg Gly Tyr Thr Asn Tyr Asn Gln Lys Phe Lys
1               5                   10                  15

Asp

<210>  11
<211>  10
<212>  PRT
<213>  Mus musculus

<400>  11

Tyr Tyr Asp Asp His Tyr Cys Leu Asp Tyr
1               5                   10

<210>  12
<211>  10
<212>  PRT
<213>  Mus musculus

<400>  12

Ser Ala Ser Ser Ser Val Ser Tyr Met Asn
1               5                   10

<210>  13
<211>  7
<212>  PRT
<213>  Mus musculus

<400>  13

Asp Thr Ser Lys Leu Ala Ser

1               5

<210>  14
<211>  9
<212>  PRT
<213>  Mus musculus

<400>  14

Gln Gln Trp Ser Ser Asn Pro Phe Thr
1               5

<210>  15
<211>  10
<212>  PRT
<213>  Mus musculus

<400>  15

Gly Tyr Ser Phe Thr Gly Tyr Thr Met Asn
1               5                   10

<210>  16
<211>  10
<212>  PRT
<213>  Mus musculus

<400>  16

Leu Ile Asn Pro Tyr Lys Gly Val Ser Thr
1               5                   10

<210>  17
<211>  13
<212>  PRT
<213>  Mus musculus

<400>  17

Ser Gly Tyr Tyr Gly Asp Ser Asp Trp Tyr Phe Asp Val
1               5                   10

<210>  18
<211>  11
<212>  PRT
<213>  Mus musculus

<400>  18

Arg Ala Ser Gln Asp Ile Arg Asn Tyr Leu Asn
1               5                   10

<210>  19
<211>  7

<212>  PRT
<213>  Mus musculus

<400>  19

Tyr Thr Ser Arg Leu His Ser
1               5


<210>  20
<211>  9
<212>  PRT
<213>  Mus musculus

<400>  20

Gln Gln Gly Asn Thr Leu Pro Trp Thr
1               5


<210>  21
<211>  119
<212>  PRT
<213>  Mus musculus

<400>  21

Gln Val Gln Leu Gln Glu Ser Gly Ala Glu Leu Ala Arg Pro Gly Ala
1               5                   10                  15

Ser Val Lys Met Ser Cys Lys Ala Ser Gly Tyr Thr Phe Thr Arg Tyr
            20                  25                  30

Thr Met His Trp Val Lys Gln Arg Pro Gly Gln Gly Leu Glu Trp Ile
        35                  40                  45

Gly Tyr Ile Asn Pro Ser Arg Gly Tyr Thr Asn Tyr Asn Gln Lys Phe
    50                  55                  60

Lys Asp Lys Ala Thr Leu Thr Thr Asp Lys Ser Ser Ser Thr Ala Tyr
65                  70                  75                  80

Met Gln Leu Ser Ser Leu Thr Ser Glu Asp Ser Ala Val Tyr Tyr Cys
                85                  90                  95

Ala Arg Tyr Tyr Asp Asp His Tyr Cys Leu Asp Tyr Trp Gly Gln Gly
            100                 105                 110

Thr Thr Leu Thr Val Ser Ser
        115


<210>  22

<211>  107
<212>  PRT
<213>  Mus musculus

<400>  22

Gln Ile Val Leu Thr Gln Ser Pro Ala Ile Met Ser Ala Ser Pro Gly
1               5                   10                  15

Glu Lys Val Thr Met Thr Cys Ser Ala Ser Ser Ser Val Ser Tyr Met
            20                  25                  30

Asn Trp Tyr Gln Gln Lys Ser Gly Thr Ser Pro Lys Arg Trp Ile Tyr
        35                  40                  45

Asp Thr Ser Lys Leu Ala Ser Gly Val Pro Ala His Phe Arg Gly Ser
    50                  55                  60

Gly Ser Gly Thr Ser Tyr Ser Leu Thr Ile Ser Gly Met Glu Ala Glu
65                  70                  75                  80

Asp Ala Ala Thr Tyr Tyr Cys Gln Gln Trp Ser Ser Asn Pro Phe Thr
                85                  90                  95

Phe Gly Ser Gly Thr Lys Leu Glu Ile Asn Arg
            100                 105

<210>  23
<211>  122
<212>  PRT
<213>  Mus musculus

<400>  23

Glu Val Gln Leu Gln Gln Ser Gly Pro Glu Leu Val Lys Pro Gly Ala
1               5                   10                  15

Ser Met Lys Ile Ser Cys Lys Ala Ser Gly Tyr Ser Phe Thr Gly Tyr
            20                  25                  30

Thr Met Asn Trp Val Lys Gln Ser His Gly Lys Asn Leu Glu Trp Met
        35                  40                  45

Gly Leu Ile Asn Pro Tyr Lys Gly Val Ser Thr Tyr Asn Gln Lys Phe
    50                  55                  60

Lys Asp Lys Ala Thr Leu Thr Val Asp Lys Ser Ser Ser Thr Ala Tyr
65                  70                  75                  80

Met Glu Leu Leu Ser Leu Thr Ser Glu Asp Ser Ala Val Tyr Tyr Cys
                85                  90                  95

Ala Arg Ser Gly Tyr Tyr Gly Asp Ser Asp Trp Tyr Phe Asp Val Trp
            100                 105                 110

Gly Ala Gly Thr Thr Val Thr Val Ser Ser
        115                 120

<210> 24
<211> 109
<212> PRT
<213> Mus musculus

<400> 24

Asp Ile Gln Met Thr Gln Thr Thr Ser Ser Leu Ser Ala Ser Leu Gly
1               5                   10                  15

Asp Arg Val Thr Ile Ser Cys Arg Ala Ser Gln Asp Ile Arg Asn Tyr
            20                  25                  30

Leu Asn Trp Tyr Gln Gln Lys Pro Asp Gly Thr Val Lys Leu Leu Ile
        35                  40                  45

Tyr Tyr Thr Ser Arg Leu His Ser Gly Val Pro Ser Lys Phe Ser Gly
    50                  55                  60

Ser Gly Ser Gly Thr Asp Tyr Ser Leu Thr Ile Ser Asn Leu Glu Gln
65                  70                  75                  80

Glu Asp Ile Ala Thr Tyr Phe Cys Gln Gln Gly Asn Thr Leu Pro Trp
                85                  90                  95

Thr Phe Ala Gly Gly Thr Lys Leu Glu Ile Lys Arg Ala
            100                 105